# EUROPÄISCHE PATENTANMELDUNG

(11) **EP 3 789 505 A1**
(43) Veröffentlichungstag der Anmeldung: **10.03.2021**
(21) Anmeldenummer: 19195688.7
(22) Anmeldetag: 05.09.2019
(51) Int. Cl.: C12Q 1/6886

(54) **VERFAHREN UND MITTEL ZUR DIAGNOSE VON LUNGENKREBS**

(71) Anmelder: Forschungszentrum Borstel, 23845 Borstel (DE); Christian-Albrechts-Universität zu Kiel, 24118 Kiel (DE); LungenClinic Grosshansdorf, 22927 Grosshansdorf (DE)
(72) Erfinder: GOLDMANN, Torsten, 23858 Reinfeld (DE); MARWITZ, Sebastian, 23858 Reinfeld (DE); AMMERPOHL, Ole,, 88471 Laupheim, DE (DE); SCHEUFELE, Swetlana, 23714 Malente (DE); RECK, Martin,, 22926 Ahrensburg (DE)
(74) Vertreter: Moré, Solveig Helga

(57) **Zusammenfassung**

Die vorliegende Erfindung betrifft die Diagnose von Lungentumoren. Sie stellt Verfahren zur Verfügung, die besonders zur Diagnose von Lungentumoren auf Basis einer Flüssigbiopsie (*Liquid Biopsy*) geeignet sind, bei denen zellfreie DNA (zfDNA) eingesetzt wird. Dabei werden sowohl besonders geeignete Analyseverfahren als auch besonders geeignete Sätze an Methylierungsmarkern beschrieben. Gegenstand der Erfindung sind auch Mittel, geeignet zur Diagnose von Lungenkrebs durch Untersuchung der Methylierung eines Satzes von Methylierungsmarkern, bevorzugt in zfDNA aus einer Flüssigbiopsie-Probe eines Patienten, wobei das Mittel Oligonukleotide umfasst, welche mit DNA hybridisieren können, welche die Methylierungsmarker umfasst, sowie die Verwendung dieser Verfahren und Mittel zur Diagnose von Lungentumoren.

## Beschreibung

Die vorliegende Erfindung betrifft die Diagnose von Lungentumoren. Sie stellt Verfahren zur Verfügung, die besonders zur Diagnose von Lungentumoren auf Basis von Flüssigbiopsien (Liquid Biopsies) geeignet sind, bei denen zellfreie DNA (zfDNA) eingesetzt wird. Dabei werden sowohl besonders geeignete Analyseverfahren als auch besonders geeignete Sätze an Methylierungsmarkern beschrieben. Gegenstand der Erfindung sind auch Mittel, geeignet zur Diagnose von Lungenkrebs durch Untersuchung der Methylierung eines Satzes von Methylierungsmarkern, bevorzugt in zfDNA aus einer Flüssigbiopsie-Probe eines Patienten, wobei das Mittel Oligonukleotide umfasst, welche mit DNA hybridisieren können, welche die Methylierungsmarker umfasst, sowie die Verwendung dieser Verfahren und Mittel zur Diagnose von Lungentumoren.

Lungenkrebs ist weltweit die zweithäufigste Krebsart bei Männern und Frauen. In Deutschland werden jährlich ca. 52.500 Neuerkrankungen registriert. Das mittlere Erkrankungsalter liegt für Männer bei 70 und für Frauen bei 69 Jahren. Dabei wird zwischen dem kleinzelligen (engl. *small cell lung cancer*; SCLC) und nicht-kleinzelligen (engl. *non-small cell lung cancer*; NSCLC) Lungenkarzinom unterschieden. NSCLC ist deutlich häufiger und tritt bei 85% der betroffenen Patienten auf. Des Weiteren wird NSCLC in mehrere Subentitäten unterschieden, davon sind die häufigsten Adeno- und Plattenepithelkarzinome.

Dass die Symptome der Erkrankung meistens sehr spät auftreten, spiegelt sich in einer schlechten Prognose wieder. Die 5-Jahres-Überlebensrate liegt bei 15%. Meistens klagen die betroffenen Patienten über einen plötzlich auftretenden und immer schlimmer werdenden Husten, Müdigkeit, Abgeschlagenheit oder plötzliche Gewichtsabnahme.

Das Lungenkarzinom weist, genauso wie die meisten anderen Tumore, eine hohe genomische Heterogenität auf. So können z.B. Mutationen innerhalb von *KRAS, EGFR, BRAF, MEK1, MET, HER2, ALK, ROS1, RET, FGFR1, DDR2, PTEN, LKB1, RB1, CDKN2A* oder *TP53* Genen die Entstehung eines primären Lungenkarzinoms induzieren. Zusätzlich akkumulieren im Laufe der Tumor-Evolution die sogenannten Passenger-Mutationen, die zu verschiedenen Subklonen führen können. Diese Tatsache macht die Entwicklung eines zuverlässigen, nur auf molekulargenetischen Mutationsanalysen basierten Früherkennungstests sehr schwierig, was an vielen Beispielen in der Literatur sichtbar wird.

So haben z.B. Uchida *et al.* versucht ein Lungenkarzinom-Screening basierend auf typischen Mutationen des EGFR-Gens durchzuführen. Die durchschnittliche Sensitivität des Tests betrug dabei 54,4% und fiel bei frühen Stadien IA-IIIA auf 22,2% (Uchida et al. [2015] Clin. Chem. 61: 1191-1196). Couraud *et al.* entwickelten einen NGS-basierten Test, bei dem die bekanntesten Mutationen innerhalb der *EGFR, BRAF, KRAS, HER2* und *PIK3CA* Gene im Plasma analysiert wurden. Die Sensitivität dieses Tests betrug 58%. Auch hier stellte die Erkennung von Tumoren in frühen Stadien ein Problem dar (Couraud et al. [2014] Clin. Cancer Res. 20: 4613-4624). Newmann *et al.* entwickelten 2014 das CAPP-Seq. Hierbei handelte es sich um ein optimiertes NGS-Protokoll mit einer dazugehörigen bioinformatischen Auswertepipeline. Beim CAPP-Seq. wurden die bekanntesten NSCLC-Mutationen im Plasma sequenziert und analysiert, wodurch 100% der Lungenkrebspatienten Stadien II bis IV identifiziert werden konnten. Die Identifikation von Tumoren im Stadium I stellte aber wieder ein Problem dar, und die entsprechende Sensitivität betrug nur 50% (Newman et al. [2014] Nat. Methods 20: 548-554). Diese Beispiele zeigen deutlich die Problematik bei der Entwicklung eines zuverlässigen, nur auf genomischen Analysen basierten Lungenkarzinomfrüherkennungstests.

Zusätzlich zu Mutationen spielen während der Tumor-Evolution auch Epimutationen eine entscheidende Rolle. So werden z.B. Promotoren innerhalb bestimmter Tumor-Suppressorgene hypermethyliert, was wiederum deren transkriptioneller Repression zur Folge hat. Dieses Phänomen ist durch die Überexpression von DNA-Methyltransferasen begleitet. Besonders häufig wurde eine Promotor-Hypermethylierung in der Literatur innerhalb der *P16INK4A, RASSF1A, APC, RARB, CDH1, CDH13, DAPK, FHIT* und *MGMT* Gene beschrieben (Langevin et al. [2015] Transl. Res. 165: 74-90).

Die genomweite Hypomethylierung des NSCLC ist mit der genomischen Instabilität assoziiert. Eine gezielte Hypomethylierung der Gene konnte bisher nur bei *MAGEA3*/*6, TKTL1, BORIS, DDR1, YWHAZ sowie TMSB10* identifiziert werden (u.a. Newman et al. [2014] Nat. Methods 20: 548-554).

Des Weiteren weisen maligne Lungentumore häufig eine veränderte Histon-Acetylierung an den Positionen H4K5, H4K8, H4K12 und H4K16 auf. Auch der globale Anteil an H4K20me3 ist in NSCLC geringer als in gesundem Lungengewebe (Newman et al. [2014] Nat. Methods 20: 548-554). Zusätzlich kann es zu aberranten ncRNA-Expression kommen, wie z.B. *MIR196A, MIR200B, MALAT1* sowie *HOTAIR.*

Laut nationalen und internationalen Empfehlungen wird der betroffene Patient im Moment bei einer Verdachtsdiagnose zunächst einer umfassenden körperlichen Untersuchung unterzogen. Nachfolgend wird der Brustkorb durch bildgebende Verfahren wie z.B. Röntgen oder Computertomographie (CT) untersucht. Falls dabei ein Tumor detektiert wird, wird eine Bronchoskopie empfohlen, bei der die Lunge endoskopisch gründlich analysiert sowie eine Biopsie des Tumors entnommen wird. Die Biopsie wird weiteren histologischen und molekulargenetischen Analysen unterzogen.

Während der histologischen Untersuchung wird festgestellt, ob der Tumor bösartig ist. Falls dies der Fall ist, wird dessen Entität ermittelt. Um die optimale Therapie zu identifizieren, werden zusätzlich molekulargenetische sowie bildgebende Verfahren herangezogen. Vor allem die bildgebenden sowie endoskopischen Verfahren können hierbei aufgrund der Radioaktivität und Invasivität für die betroffenen Patienten belastend sein.

Das Detektionslimit der radiologischen Verfahren liegt bei einer Tumor-Größe von 7 bis 10 mm, was Zellhaufen bestehend aus bereits rund einer Milliarde Tumor-Zellen entspricht. Eine alternative, weniger invasive Methode beruht auf Flüssigbiopsien (*Liquid Biopsies*), mittels derer Tumore viel früher, ab einer Größe von ca. 50 Millionen Zellen, detektiert werden können.

Bei *Liquid Biopsies* werden dem Patienten einige Milliliter Blut entnommen. Aus dem Blutplasma oder Blutserum kann anschließend zirkulierende zellfreie DNA (zfDNA) isoliert werden. Im menschlichen Körper entsteht die zfDNA im Laufe apoptotischer sowie nekrotischer Prozesse. Dabei wird zelluläre, genomische DNA (gDNA) durch DNAsen in ca. 167 bp lange Fragmente gespalten und im Blutkreislauf freigesetzt.

Bei Patienten, die an malignen Erkrankungen leiden, ist in der Gesamtmenge an zfDNA zusätzlich Tumor-DNA enthalten. Je nach Entität bzw. Stadium der Erkrankung kann die zfDNA-Menge stark variieren. Sie enthält jedoch diagnostisch, therapeutisch und prognostisch relevante Informationen.

Zusätzlich zu genetischen Mutationen eines Tumors können auch Epimutationen analysiert werden. Besonders interessant ist in diesem Zusammenhang die DNA-Methylierung. Das DNA-Methylierungsmuster ist gewebespezifisch und ändert sich bereits in frühen Phasen der Tumor-Evolution. Des Weiteren machte eine Studie des *GNAS1 Locus* deutlich, dass die zfDNA-Methylierung im Blut stabil bleibt. Sie wird weder modifiziert noch verfälscht und eignet sich somit als Biomarker in der klinischen Diagnostik (Puszyk et al. [2009] Clin. Chim. Acta 400: 107-110).

Mehrere Studien haben das diagnostische Potential der DNA-Methylierung bereits deutlich gemacht. So zeigte eine *SOX17*-Studie beim Magenkarzinom, dass das Gesamtüberleben der Patientenkohorte mit der nachgewiesenen Menge an methylierter *SOX17*-zfDNA korrelierte (Balgkouranidou et al. [2013] Clin. Chem. Lab. Med. 51: 1505-1510). Eine Studie mit Patientinnen, die an Mammakarzinom litten, zeigte eine signifikante Hypermethylierung des *CST6*-Gens (Chimonidou et al. [2013] Clin. Biochem. 46: 235-240). Liggett et al. ist es gelungen, anhand des DNA-Methylierungsmusters das Pankreaskarzinom von seiner Vorstufe, der chronischen Pankreatitis, zu unterscheiden (Liggett et al. [2010] Cancer 116: 1674-1680).

Auch beim nicht-kleinzelligen Lungenkarzinom wurden von mehreren Arbeitsgruppen Veränderungen des DNA-Methylierungsmusters beschrieben. So konnten z.B. Balgkouranidou et al. eine signifikante Hypermethylierung des BRMS1-Gens bei Patienten mit Bronchialkarzinom nachweisen (Balgkouranidou et al. [2014] Brit. J. Cancer 110: 2054-2062). 2016 detektierten Marwitz et al. DNA-Hypomethylierung innerhalb der *CTLA4-* sowie *PDCD1*-Gene. Auf Transkriptom-Ebene waren diese Gene überexprimiert. Da es sich hier um wichtige *Checkpoint-*Regulatoren handelt, hat diese Arbeit starke therapeutische Relevanz (Marwitz et al. [2017] Clin. Epigenet. 9: 51).

Das diagnostische Potential der DNA-Methylierung wird auch am Beispiel des "Epi proLung" Assays ("Epigenomics AG", Deutschland) deutlich. Dabei wird das zfDNA-Methylierungsmuster der *SHOX2 und PTGER4* Gene analysiert. Bei einer Spezifität von 90% beträgt die Sensitivität 67% (Weiss et al. [2017] J. Thorac. Oncol. 12: 77-84). Für ein zuverlässiges Lungenkrebs-Screening reicht die Empfindlichkeit des "Epi proLung" Tests daher nicht aus. Bisher gibt es keine weiteren auf *Liquid Biopsies* basierende Verfahren, die eine zuverlässige, präventive Lungenkrebs-Früherkennung ermöglichen.

Dem gegenüber stellten sich die Erfinder die Aufgabe, ein zuverlässigeres Verfahren zur Diagnose von Lungenkrebs zur Verfügung zu stellen. Diese Aufgabe wird durch die Erfindung, insbesondere durch den Gegenstand der Ansprüche, gelöst.

Ein Gegenstand der Erfindung ist ein Verfahren zur Diagnose von Lungenkrebs, bei dem man die Methylierung eines Satzes von Methylierungsmarkern in einer Probe eines Patienten bestimmt, wobei man bevorzugt zfDNA aus einer Flüssigbiopsie untersucht. Alternativ kann die Probe auch eine Gewebeprobe sein, z.B. eine solide Gewebeprobe aus einem Tumor oder aus einem Gewebe, in dem möglicherweise ein Tumor vorliegt. Insbesondere kann die Gewebeprobe aus einer Biopsie von Lungengewebe stammen. Auch Pleuraflüssigkeit kann untersucht werden.

Die Erfindung stellt ein Verfahren zur Diagnose von Lungenkrebs zur Verfügung, bei dem die Methylierung eines Satzes von Methylierungsmarkern, bevorzugt in zfDNA aus einer Flüssigbiopsie-Probe eines Patienten, bestimmt wird, wobei optional ein Alignment gegen ein Referenzgenom mit dem Segemehl-Algorithmus durchführt wird.

Die Erfindung stellt ferner ein Verfahren zur Diagnose von Lungenkrebs zur Verfügung, bei dem die Methylierung eines Satzes von Methylierungsmarkern, bevorzugt in zfDNA aus einer Flüssigbiopsie-Probe eines Patienten, bestimmt wird, wobei optional die Methylierung von Methylierungsmarkern in den Genen *SERPINB5*, *DOCK10*, *PCDHB2*, *HIF3A*, *FGD5*, *RCAN2*, *HOXD12*, *OCA2, SLC22A20*, *FADL-1*, *NRXN1*, *ACOXL*, *FAM53A*, *UBE3D* und *AUTS2* bestimmt wird. Zur nichtinvasiven Diagnostik von Lungentumoren (Lungenkarzinomen) wird erfindungsgemäß bevorzugt die zirkulierende zellfreie DNA (zfDNA) aus Flüssigbiopsien, z.B. aus Plasma, Blut, oder Serum , bevorzugt aus Plasma, genutzt. Falls ein Patient an einer malignen Tumorerkrankung leidet, ist in der Gesamtmenge der zirkulierenden DNA auch die Tumor-DNA enthalten, welche alle therapeutisch und prognostisch relevanten Informationen über die genetischen und epigenetischen Charakteristika des Tumors enthält. Die Erfindung stellt sowohl bevorzugte Verfahren zur Diagnose von Lungenkrebs auf dieser Basis also auch bevorzugte Sätze von Methylierungsmarkern bereit.

Im Rahmen der Erfindung wurde gezeigt, dass die Methylierungssignaturen in festen Tumoren, z.B. in Proben aus Operationen oder Biopsien sich z.T. von den Signaturen aus zfDNA aus Flüssigbiopsien unterschieden. Dies kann erklären, warum die bereits erwähnte "Epi proLung"-Studie, bei der das zfDNA-Methylierungsprofil innerhalb der *SHOX2* und *PTGER4* Gene analysiert wurde, bei einer Spezifität von 90% nur eine Sensitivität von 67% aufwies (Weiss et al. [2017] J. Thorac. Oncol. 12: 77-84). Die verwendeten *SHOX2* und *PTGER4* Biomarker stammen aus Analysen primärer Tumorgewebe (Murn et al. [2008] J. Exp. Med. 205: 3091-3103; und Schneider et al. [2011] BMC Cancer 11: 102). Die vorliegende Erfindung zeigt jedoch deutlich (siehe Abschnitt 2.1.3), dass die DNA-Methylierungsmuster zwischen der zfDNA aus dem Plasma und der gDNA aus einem primären Tumor nur bedingt korrelieren. In der Tat enthält die Gesamtmenge der zfDNA nicht nur aus der Lunge bzw. einem Tumor stammende DNA, sondern auch DNA aus weiteren Geweben und Organen.

Das bedeutet, dass die im primären Tumorgewebe stark aberrant methylierten DNA-Regionen im Plasma nicht unbedingt eine differentielle Methylierung aufweisen. Daher reicht es für die Entwicklung eines nicht-invasiven, zfDNA-basierten Früherkennungstests nicht aus, bekannte Biomarker aus den primären Tumoren zu nutzen. Vielmehr ist es notwendig, neue zfDNAspezifische, starke und eindeutige Methylierungssignaturen im Plasma der betroffenen Patienten zu identifizieren. Dies wurde mit der vorliegenden Erfindung erreicht. Erfindungsgemäß vorteilhaft ist es, dass die identifizierten Marker sowohl mit Gewebeproben z.B. soliden Gewebeproben aus Tumorgewebe als auch mit Flüssigbiopsien gute Ergebnisse liefern und somit zur Diagnose von Lungenkrebs aus verschiedenen Arten von Proben geeignet sind.

Um einen erfindungsgemäßen Satz an Methylierungsmarkern, der besonders aussagekräftige differentiell methylierte Regionen umfasst, zu identifizieren, wurden im Rahmen der Erfindung mehrere Schritte durchgeführt, die im Detail im Beispielteil beschrieben sind. Zuerst wurden DNA-Methylierungssignaturen in 40 malignen Lungentumoren sowie deren korrespondierenden Kontrollen untersucht. Dann erfolgte eine Analyse von DNA-Methylierungssignaturen im Blutplasma von neun Patienten. Davon litten fünf Patienten an einem Adeno- und vier an Plattenepithelkarzinom der Lunge. Die übrigen Patienten waren dagegen frei von malignen Erkrankungen und bildeten die Kontrollkohorte. Schließlich wurden zusätzliche Datensätze mehrerer zur Verfügung gestellter Studien ausgewertet, was das Identifizieren weiterer tumorspezifischer und prognostischer CpG *Loci* ermöglichte. Der auf dieser Basis synthetisierte Satz an Methylierungsmarkern, auch als *Plasma Panel* (siehe Tabelle 1) bezeichnet, wurde anschließend im Rahmen einer Pilotstudie validiert. Dieser Satz an Methylierungsmarkern umfasst eine Vielzahl von Regionen, die in zfDNA differenziell methyliert sind und überraschenderweise eine spezifische Aussage über das Vorhandensein eines Tumors, die Tumorentität, das Tumorstadium und/oder die Prognose gestatten.

In einer Ausführungsform betrifft die Erfindung daher ein Verfahren zur Diagnose von Lungenkrebs, bei dem die Methylierung eines Satzes von Methylierungsmarkern, bevorzugt in zfDNA aus einer Flüssigbiopsie-Probe eines Patienten, bestimmt wird, wobei der Satz von Methylierungsmarkern mindestens 60 Regionen umfasst, ausgewählt aus der Gruppe bestehend aus:

| Chromosom | Start | Ende |
|---|---|---|
| chr1 | 6165201 | 6165361 |
| chr1 | 17567892 | 17568189 |
| chr1 | 15426262 | 15426418 |
| chr1 | 15670403 | 15670539 |
| chr2 | 1126410 | 1126557 |
| chr2 | 225642009 | 225642217 |
| chr2 | 236745514 | 236745688 |
| chr2 | 240881986 | 240882138 |
| chr2 | 2179742 | 2179886 |
| chr2 | 30747398 | 30747539 |
| chr2 | 175998270 | 175998415 |
| chr2 | 219647407 | 219647560 |
| chr3 | 56445240 | 56445378 |
| chr3 | 85143433 | 85143600 |
| chr3 | 146123966 | 146124095 |
| chr3 | 68947379 | 68947542 |
| chr3 | 197767819 | 197767978 |
| chr4 | 143487129 | 143487273 |
| chr4 | 26398190 | 26398329 |
| chr4 | 77647893 | 77648027 |
| chr4 | 102497551 | 102497732 |
| chr5 | 39187156 | 39187287 |
| chr5 | 56145736 | 56145896 |
| chr5 | 160171748 | 160171896 |
| chr5 | 16793080 | 16793219 |
| chr5 | 76869108 | 76869253 |
| chr6 | 169050287 | 169050447 |
| chr6 | 76773251 | 76773422 |
| chr6 | 123869831 | 123869971 |
| chr7 | 6268960 | 6269087 |
| chr7 | 38508407 | 38508486 |
| chr7 | 153743779 | 153743947 |
| chr7 | 137230794 | 137230963 |
| chr7 | 151300131 | 151300282 |
| chr8 | 3672236 | 3672387 |
| chr8 | 99510084 | 99510252 |
| chr8 | 101170822 | 101170975 |
| chr8 | 141127042 | 141127183 |
| chr9 | 2050654 | 2050804 |
| chr9 | 9227683 | 9227824 |
| chr9 | 79060522 | 79060633 |
| chr9 | 124334690 | 124334848 |
| chr9 | 126166694 | 126166828 |
| chr10 | 96279972 | 96280055 |
| chr10 | 97033594 | 97033733 |
| chr11 | 134245966 | 134246129 |
| chr12 | 8004422 | 8004573 |
| chr12 | 97140774 | 97140905 |
| chr12 | 111566555 | 111566698 |
| chr12 | 117750775 | 117750937 |
| chr13 | 36828740 | 36828902 |
| chr14 | 93214072 | 93214242 |
| chr15 | 56006471 | 56006552 |
| chr15 | 101547384 | 101547527 |
| chr16 | 4141795 | 4141956 |
| chr18 | 21857621 | 21857750 |
| chr18 | 29528340 | 29528468 |
| chr18 | 46845901 | 46846043 |
| chr19 | 874766 | 874934 |
| chr19 | 6799968 | 6800095 |
| chr20 | 20243607 | 20243747 |
| chr20 | 55079800 | 55079945 |
| chr21 | 30502729 | 30502871 |
| chr21 | 46587906 | 46588052 |

Die vorgenannten Methylierungsmarker sind die in Tabelle 1a genannten Marker, die nur bei zfDNA identifiziert wurden. Bei dieser Analyse wird bevorzugt das Vorhandensein eines Tumors geprüft, wobei der Satz von Methylierungsmarkern optional alle Regionen der Gruppe umfasst.

Dabei kann der Satz von Methylierungsmarkern mindestens 340 Regionen umfassen, ausgewählt aus der Gruppe bestehend aus den in Tabelle 1a aufgelisteten Regionen, wobei der Satz von Methylierungsmarkern bevorzugt alle in Tabelle 1a aufgelisteten Regionen umfasst.

In einer Ausführungsform der oben genannten Verfahren umfasst der Satz von Methylierungsmarkern mindestens 134 Regionen, ausgewählt aus der Gruppe bestehend aus

| Chromosom | Start | Ende |
|---|---|---|
| chr1 | 3289010 | 3289139 |
| chr1 | 17567892 | 17568189 |
| chr1 | 23284417 | 23284507 |
| chr1 | 24277975 | 24278154 |
| chr1 | 47738990 | 47739142 |
| chr1 | 79467955 | 79468081 |
| chr1 | 108975333 | 108975476 |
| chr1 | 196682870 | 196683025 |
| chr1 | 217310510 | 217310654 |
| chr1 | 240656480 | 240656649 |
| chr1 | 240746545 | 240746706 |
| chr1 | 246241918 | 246242056 |
| chr2 | 1129413 | 1129596 |
| chr2 | 1334513 | 1334640 |
| chr2 | 23917010 | 23917136 |
| chr2 | 25124037 | 25124165 |
| chr2 | 46779214 | 46779381 |
| chr2 | 113534514 | 113534653 |
| chr2 | 120417931 | 120418073 |
| chr2 | 131798797 | 131798977 |
| chr2 | 198073787 | 198073950 |
| chr2 | 205889570 | 205889704 |
| chr2 | 207319476 | 207319691 |
| chr3 | 3755582 | 3755730 |
| chr3 | 14959981 | 14960128 |
| chr3 | 25581721 | 25581859 |
| chr3 | 75834579 | 75834736 |
| chr3 | 87031909 | 87032079 |
| chr3 | 122710736 | 122710872 |
| chr3 | 139727561 | 139727706 |
| chr3 | 145864433 | 145864574 |
| chr4 | 1665996 | 1666155 |
| chr4 | 22518120 | 22518271 |
| chr4 | 77306769 | 77306948 |
| chr4 | 82520036 | 82520212 |
| chr4 | 155413871 | 155414011 |
| chr4 | 156601279 | 156601436 |
| chr4 | 162457724 | 162457860 |
| chr4 | 176636441 | 176636580 |
| chr4 | 177654193 | 177654363 |
| chr5 | 14450118 | 14450272 |
| chr5 | 75935318 | 75935450 |
| chr5 | 140475728 | 140475872 |
| chr5 | 146345906 | 146346062 |
| chr5 | 156458027 | 156458167 |
| chr5 | 157169890 | 157170038 |
| chr6 | 20832000 | 20832349 |
| chr6 | 24420281 | 24420413 |
| chr6 | 36331071 | 36331215 |
| chr6 | 54074847 | 54075021 |
| chr6 | 71122323 | 71122483 |
| chr6 | 83604672 | 83604779 |
| chr6 | 90709859 | 90710016 |
| chr6 | 111744738 | 111744881 |
| chr6 | 148806765 | 148806922 |
| chr6 | 155574119 | 155574263 |
| chr6 | 158460178 | 158460323 |
| chr7 | 5549605 | 5549675 |
| chr7 | 40669616 | 40669796 |
| chr7 | 73799798 | 73799908 |
| chr7 | 78030021 | 78030155 |
| chr7 | 81399230 | 81399365 |
| chr7 | 134452355 | 134452524 |
| chr7 | 140335200 | 140335344 |
| chr7 | 146925646 | 146925824 |
| chr7 | 153976496 | 153976643 |
| chr7 | 157941162 | 157941344 |
| chr7 | 157980130 | 157980264 |
| chr7 | 157980485 | 157980624 |
| chr7 | 158314155 | 158314301 |
| chr8 | 6392188 | 6392336 |
| chr8 | 11724061 | 11724159 |
| chr8 | 17237496 | 17237639 |
| chr8 | 21803649 | 21803801 |
| chr8 | 52696850 | 52697008 |
| chr8 | 72183950 | 72184120 |
| chr8 | 81042553 | 81042694 |
| chr8 | 85101824 | 85101952 |
| chr8 | 110703169 | 110703320 |
| chr8 | 121727803 | 121727944 |
| chr8 | 133476418 | 133476558 |
| chr9 | 8813022 | 8813150 |
| chr9 | 90258110 | 90258253 |
| chr9 | 97061691 | 97061835 |
| chr10 | 12533631 | 12533768 |
| chr10 | 32647546 | 32647656 |
| chr10 | 32657588 | 32657719 |
| chr10 | 37511104 | 37511239 |
| chr10 | 62708104 | 62708269 |
| chr10 | 73207931 | 73208064 |
| chr10 | 108812804 | 108812940 |
| chr10 | 115658133 | 115658275 |
| chr10 | 123914649 | 123914808 |
| chr11 | 15025357 | 15025499 |
| chr11 | 19778770 | 19778909 |
| chr11 | 26355535 | 26355711 |
| chr11 | 26600784 | 26600925 |
| chr11 | 26626367 | 26626558 |
| chr11 | 41275397 | 41275536 |
| chr11 | 62158845 | 62158985 |
| chr11 | 70503001 | 70503139 |
| chr11 | 106592142 | 106592304 |
| chr11 | 120644150 | 120644282 |
| chr11 | 122678508 | 122678636 |
| chr11 | 128851150 | 128851286 |
| chr12 | 125571801 | 125571933 |
| chr13 | 48806444 | 48806588 |
| chr13 | 113527733 | 113527876 |
| chr14 | 35030336 | 35030470 |
| chr14 | 104486171 | 104486314 |
| chr15 | 22839905 | 22840043 |
| chr15 | 26964926 | 26965065 |
| chr15 | 29246303 | 29246447 |
| chr15 | 30180680 | 30180842 |
| chr15 | 32404970 | 32405130 |
| chr15 | 64244033 | 64244215 |
| chr15 | 68530927 | 68531091 |
| chr15 | 83579367 | 83579513 |
| chr15 | 88559865 | 88560003 |
| chr16 | 6257325 | 6257474 |
| chr16 | 15665564 | 15665721 |
| chr16 | 24321180 | 24321320 |
| chr16 | 75528556 | 75528698 |
| chr16 | 88013993 | 88014135 |
| chr16 | 89713952 | 89714124 |
| chr17 | 416719 | 416865 |
| chr17 | 19809670 | 19809830 |
| chr17 | 21086965 | 21087112 |
| chr17 | 33364961 | 33365040 |
| chr17 | 64330485 | 64330837 |
| chr17 | 75142732 | 75142885 |
| chr19 | 11890923 | 11891074 |
| chr19 | 49016450 | 49016584 |
| chr19 | 57922060 | 57922195 |
| chr20 | 9706282 | 9706429 |
| chr20 | 33713618 | 33713757 |
| chr21 | 33340955 | 33341038 |
| chr22 | 21206849 | 21206995 |
| chr22 | 30292326 | 30292475 |
| chr22 | 35697444 | 35697606 |

Die genannten Methylierungsmarker sind die in Tabelle 1 b genannten Marker, die nur bei zfDNA identifiziert wurden. Bei dieser Analyse wird bevorzugt die Entität eines Tumors geprüft, wobei insbesondere zwischen Adenokarzinom und Plattenepithelkarzinom unterschieden werden kann. Dabei kann der Satz von Methylierungsmarkern alle Regionen der Gruppe umfassen.

Bei dieser Analyse kann der Satz von Methylierungsmarkern auch mindestens 240 Regionen umfassen, wobei die Gruppe aus den in Tabelle 1b aufgelisteten Regionen besteht. Bevorzugt umfasst der Satz von Methylierungsmarkern alle in Tabelle 1b aufgelisteten Regionen der Gruppe.

Da gezeigt wurde, dass alle in Tabelle 1a und 1b definierten Regionen in den untersuchten Proben differentiell methyliert sind, ist es vorteilhaft alle in Tabelle 1a und 1b definierten Regionen zu analysieren, insbesondere wenn sowohl das Vorhandensein als auch die Entität eines potentiellen Tumors analysiert werden sollen.

Am größten ist die Aussagekraft der Analyse, wenn der Satz von Methylierungsmarkern mindestens 620 Regionen aus einer Gruppe umfasst, welche aus allen in Tabelle 1 aufgelisteten Regionen besteht, insbesondere wenn man ferner die Prognose bestimmt, bevorzugt, wenn der Satz von Methylierungsmarkern alle Regionen der Gruppe umfasst.

Bei der weiteren Analyse der Daten und der Überprüfung anhand von zfDNA von Patienten wurde im Rahmen der Erfindung ein zweiter Satz an Methylierungsmarkern mit verschiedenen Untergruppen identifiziert, mit deren Hilfe unterschiedliche Fragestellungen beantwortet werden können (siehe Tabellen 2-4). Die entsprechenden Methylierungsmarker stellen definierte differentiell methylierte Positionen dar, die in den in Tabelle 1 genannten Regionen liegen. Damit repräsentieren die in Tabellen 2-4 genannten Methylierungsmarker geeignete Untergruppen zur Untersuchung der im *Plasma-Panel* enthaltenen Methylierungsmarker.

Im Rahmen der Erfindung können also entweder differentiell methylierte Regionen, z.B. die in Tabelle 1a, 1b und/oder 1c definierten Regionen, als Methylierungsmarker dienen, oder differentiell methylierte Positionen. Dabei führt die Analyse ganzer Regionen zu zuverlässigeren Ergebnissen, da bei einzelnen Patienten spezifische Positionen nicht unbedingt die gleiche Aussagekraft haben müssen. Dafür ist eine Analyse spezifischer Positionen mit geringerem Aufwand möglich und ist daher günstig, wenn eine kostengünstige Diagnose gestellt werden soll. Die Auswahl richtet sich also nach einer Abwägung zwischen der im jeweiligen Fall nötigen Zuverlässigkeit und dem möglichen Aufwand. Selbstverständlich können auch beide Typen von Methylierungsmarkern gleichzeitig zur Diagnose herangezogen werden.

In diesem Rahmen identifizierte, besonders aussagekräftige Methylierungsmarker liegen z.T. in den Genen *SERPINB5*, *DOCK10*, *PCDHB2*, *HIF3A*, *FGD5*, *RCAN2*, *HOXD12*, *OCA2, SLC22A20, FADL-1, NRXN1*, *ACOXL*, *FAM53A*, *UBE3D* und *AUTS2.* Diese Gene wurden bisher noch nie speziell im Zusammenhang mit Lungenkarzinomen oder bestimmten NSCLC-Entitäten beschrieben.

Die Rolle einiger dieser Gene bei der Tumor-Evolution und Prognose ist bei anderen Krebsarten bekannt. Bei *SERPIN5* handelt es sich z.B. um ein bekanntes Onkogen (Lei et al. [2011] Oncol. Rep. 26: 1115-1120). *HOX*-Gene werden in vielen Krebsarten aberrant exprimiert (Bhatlekar et al. [2014] J. Mol. Med. 92: 811-823). Fehlregulation von *RCAN2* führt zu Proliferation der Tumorzellen (Niitsu et al. [2016] Oncogenesis 5: e253). Veränderte Expression von *DOCK10* hatte bei einigen Studien die Migration von Melanomzellen zur Folge (Gadea et al. [2008] Curr. Biol. 18: 1456-1465). Auch einige *OCA2*-Mutationen sind mit erhöhtem Melanom-Risiko assoziiert (Hawkes et al. [2013] J. Dermatol. Sci. 69: 30-37). Des Weiteren sind *HIF3A* und *FGD5* wichtige Angiogenese-Regulatoren und spielen somit eine entscheidende Rolle während der Tumor-Evolution (Jackson et al. [2010] Expert Opin. Therap. Targets 14: 1047-1057); und Kurogane et al. [2012] Arterioscler. Thromb. Vasc. Biol. 32: 988-996). Die DNA-Methylierung einiger *PCDHB2*-CpG *Loci* ist mit einer schlechten Prognose der Neuroblastom-Patienten assoziiert (Abe et al. [2005] Cancer Res. 65: 828-834). Veränderter Metabolismus ist z.B. ein Kennzeichen maligner Tumore, dabei können der *FADL-1* Fettsäuren-Transporter sowie einige SLC-Transporter eine wichtige Rolle spielen (Lin et al. [2015] Nat. Rev. Drug Discov. 14: 543-560; und Black [1991] J. Bacteriol. 173: 435-442). *UBE3D* kodiert für eine Ubiquitin-Protein-Ligase. Mehrere Studien haben gezeigt, dass einige Ubiquitin-Protein-Ligasen während der Tumor-Evolution eine wichtige Rolle spielen können (u.a. Lisztwan et al. [1999] Genes Dev. 13: 1822-1833). Bei *AUTS2* und *NRXN1* handelt es sich um neuronale Gene. Eine *AUTS2-*Überexpression wurde in Lebermetastasen nachgewiesen (Oksenberg & Ahituv [2013] Trends Genet. 29: 600-608). *NRXN1* könnte für die Nikotinsucht verantwortlich sein (Ching et al. [2010] Am. J. Med. Genet. B. Neuropsychiatr. Genet. 153B: 937-947). Erhöhte Expression von *ACOXL* wurde bereits bei Prostatakarzinomen beschrieben (O'Hurley et al. [2015] PLoS One 10: e0133449). Einige Studien beschreiben *FAM53A* als einen prognostischen und therapeutischen Mammakarzinom-Marker (Fagerholm et al. [2017] Oncotarget 8: 18381-18398). Die vorgenannten Studien gestatten jedoch keinerlei Rückschlüsse darauf, dass eine Methylierung in diesen Genen, geschweige denn in den in Tabelle 2-4 genannten Positionen, mit einer Lungenkrebs-Erkrankung korreliert und entsprechend als diagnostischer Marker für das Vorliegen von Lungentumoren oder die Feststellung der Entität, insbesondere die Diagnose eines Adenokarzinoms oder eines Plattenepithelkarzinoms, herangezogen werden kann.

Damit stellt die Erfindung erstmalig ein Verfahren zur Diagnose von Lungenkrebs zur Verfügung, bei dem die Methylierung eines Satzes von Methylierungsmarkern, bevorzugt in zfDNA aus einer Flüssigbiopsie-Probe eines Patienten, bestimmt wird, wobei die Methylierung von Methylierungsmarkern in den Genen *SERPINB5*, *DOCK10*, *PCDHB2*, *HIF3A*, *FGD5, RCAN2, HOXD12, OCA2, SLC22A20, FADL-1, NRXN1, ACOXL, FAM53A, UBE3D* und *AUTS2* bestimmt wird.

Bevorzugt umfassen diese Methylierungsmarker die in Tabelle 2 genannten Methylierungsmarker, insbesondere, wenn das Vorliegen eines Lungenkarzinoms bestimmt werden soll. Alternativ, insbesondere wenn die Entität eines Lungenkarzinoms bestimmt werden soll, und insbesondere wenn zwischen NSCLC-Typen Adenokarzinom und Plattenepithelkarzinom differenziert werden soll, umfassen die Methylierungsmarker die in Tabelle 3 genannten Methylierungsmarker. Bevorzugt werden sowohl die in Tabelle 2 als auch in Tabelle 3 genannten Methylierungsmarker bestimmt, um beide Fragestellungen zu beantworten. Optional können ferner auch die in Tabelle 4 genannten Methylierungsmarker analysiert werden, was ferner Rückschlüsse auf das Stadium des Tumors gestattet.

Die Erfindung stellt damit ferner ein Verfahren zur Diagnose von Lungenkrebs zur Verfügung, bei dem die Methylierung eines Satzes von Methylierungsmarkern, bevorzugt in zfDNA aus einer Flüssigbiopsie-Probe eines Patienten, bestimmt wird, wobei der Satz von Methylierungsmarkern die 10 folgenden Positionen (siehe auch Tabelle 2) umfasst:

| ID | Chromosom | Position |
|---|---|---|
| 596 | chr11 | 57006229 |
| 1717 | chr15 | 28262724 |
| 2636 | chr18 | 61144199 |
| 2805 | chr19 | 46823441 |
| 4674 | chr2 | 176964685 |
| 4999 | chr2 | 225642035 |
| 5071 | chr3 | 14960020 |
| 5576 | chr4 | 13525705 |
| 6105 | chr5 | 140475760 |
| 6434 | chr6 | 46386723. |

Es wurde gezeigt, dass diese Marker besonders aussagekräftig sind, wenn der kNN-Algorithmus zur Analyse verwendet wird. Mit diesen Markern kann insbesondere das Vorhandensein eines Tumors analysiert werden.

Alternativ oder zusätzlich kann der Satz von Methylierungsmarkern die 10 folgenden Positionen umfassen (siehe auch Tabelle 3):

| ID | Chromosom | Position |
|---|---|---|
| 650 | chr11 | 64993331 |
| 2995 | chr1 | 17568007 |
| 4233 | chr2 | 50574690 |
| 4241 | chr2 | 50574708 |
| 4428 | chr2 | 111874494 |
| 4447 | chr2 | 121276804 |
| 5537 | chr4 | 1666074 |
| 5538 | chr4 | 1666075 |
| 6524 | chr6 | 83604790 |
| 7164 | chr7 | 69971740. |

Es wurde gezeigt, dass diese Marker besonders aussagekräftig sind, wenn der RT-Algorithmus zur Analyse verwendet wird. Mit diesen Markern kann insbesondere die Entität eines Tumors identifiziert werden.

Optional, insbesondere, wenn ferner das Stadium eines Tumors identifiziert werden soll (also z.B. zwischen frühem (I+II) und spätem (III+IV) Stadium eines Lungenkarzinoms differenziert werden soll), kann der Satz von Methylierungsmarkern ferner alle in Tabelle 4 aufgelisteten Positionen umfassen. In diesem Fall kann der SVM-Algorithmus zur Analyse verwendet werden.

Bei Regionen, die sich unter Verwendung von Proben aus frühen Lungenkarzinom-Stadien nicht validieren ließen, könnte es sich z.B. um für Metastasen spezifische Signaturen handeln. Diese Regionen wurden daher für die Berechnung des *Staging*-Parameters genutzt, also für die Berechnung des Stadiums. Bisher kann der in dieser Arbeit beschriebene *Staging*-Parameter die späten Lungenkarzinomstadien mit 80%iger Richtigkeit von frühen Stadien unterscheiden. Generell sollte der *Staging*-Parameter nur als Hinweis genutzt werden. Falls das entwickelte *Panel* ein Lungenkarzinom detektiert, wäre es weiterhin ratsam, therapeutisch relevante Informationen z.B. bezüglich der Größe oder Lage des Tumors durch bildgebende Verfahren, wie z.B. wie MRT, CT oder PET CT zu generieren. Damit ist es auch nicht essentiell, die auf das Stadium bezogenen Methylierungsmarker in jedem Fall mit zu analysieren.

Im Rahmen der Erfindung kann der Lungenkrebs NSCLC oder SCLC sein, bevorzugt NSCLC. Der NSCLC ist bevorzugt ein Adenokarzinom oder Plattenepithelkarzinom. Es wurde gezeigt, dass erfindungsgemäße Marker zwischen diesen Entitäten differenzieren können und somit zur Differentialdiagnose geeignet sind.

Die erfindungsgemäße Diagnose erlaubt eine Aussage über das Vorhandensein eines Tumors, über die Entität eines Tumors (insbesondere die Unterscheidung zwischen Adenokarzinom und Plattenepithelkarzinom), über das Tumor-Stadium und/oder über die Prognose. Am wichtigsten ist die Aussage über Vorhandensein und Entität des Tumors. Weitere Aussagen können optional auch mittels ergänzender Verfahren getroffen werden, wenn das Vorhandensein eines Tumors erfindungsgemäß festgestellt wurde. Optional erlaubt das erfindungsgemäße Verfahren aber auch bereits eine Aussage über das Vorhandensein eines Tumors, über die Entität eines Tumors (insbesondere die Unterscheidung zwischen Adenokarzinom und Plattenepithelkarzinom) und über das Tumorstadium, sowie bevorzugt über die Prognose.

Im Unterschied zu bisher bekannten Verfahren ist das erfindungsgemäße Verfahren auch zur Lungenkrebsfrüherkennung, also auch zur Diagnose in Stadium I oder II, geeignet.Vorteilhafterweise ist diese Diagnose ferner auch auf Basis einer Flüssgbiopsie-Probe, also z.B. einer Blutprobe, möglich, so dass dem Patienten nicht zwingend anderes Gewebe entnommen werden muss.

Erfindungsgemäß wird bevorzugt eine Flüssigbiopsie-Probe eines Patienten analysiert. Bevorzugt ist der Patient ein Mensch. Das Wort Patient wird im Allgemeinen synonym mit Subjekt verwendet. Es kann sich um einen Patienten mit Symptomen handeln, die den Verdacht nahelegen, dass der Patient einen Lungentumor aufweist. Es kann sich aber auch um ein Subjekt ohne Symptome handeln. Das Subjekt bzw. der Patient kann ein Risikopatient für einen Lungentumor sein. Dazu zählen Subjekte, die aufgrund bestimmter Risikofaktoren und/oder ihres Lebensstils (z.B. Rauchen, Verwendung von E-Zigaretten oder anderweitige erhöhte Exposition gegenüber kanzerogenen Agentien, Symptome) ein erhöhtes Risiko für eine Lungenkrebserkrankung besitzen und/oder radiologische Auffälligkeiten aufweisen. Der Patient kann auch ein Patient mit einem bereits behandelten, z.B. einem operierten Lungentumor sein, wobei das Wiederauftreten eines Tumors und/oder eine Metastasierung untersucht werden kann.

Generell kann die zfDNA aus einer Vielzahl von Körperflüssigkeiten extrahiert werden. So wurde z.B. bereits eine erfolgreiche Extraktion aus Blutplasma und -serum, Pleuraerguss oder Urin in der Literatur beschrieben. Erfindungsgemäß kann die Flüssigbiopsie-Probe Blut, Plasma, Serum, Sputum, Bronchialflüssigkeit und Pleuraerguss sein. Bevorzugt ist sie von Blut abgeleitet, z.B. Serum oder Plasma, bevorzugt Plasma. Da Pleuraerguss erst im Laufe der Erkrankung auftritt, ist dieses Material vor allem für die Erkennung späterer Stadien geeignet. Die zfDNA-Extraktion aus dem Plasma oder Serum ist deutlich schneller und kostengünstiger als aus dem Urin, was diese Materialien für ein *Screening* interessanter macht. Schließlich ist die zfDNA-Stabilität relevant, denn zfDNA ist in Plasma stabiler als in Serum.

In einer Ausführungsform stellt die Erfindung Mittel zur Verfügung, welche zur Diagnose von Lungenkrebs mit einem erfindungsgemäßen Verfahren durch Untersuchung der Methylierung eines Satzes von Methylierungsmarkern, bevorzugt in zfDNA aus einer Flüssigbiopsie-Probe eines Patienten, geeignet sind. Die Mittel sind bevorzugt auch zur Diagnose von Lungenkrebs mit einem erfindungsgemäßen Verfahren durch Untersuchung der Methylierung eines Satzes von Methylierungsmarkern in einer anderen Probe eines Patienten, insbesondere einer soliden Gewebeprobe aus einem Tumor oder einem Gewebe, in dem ein Tumor vermutet wird, geeignet.

Dabei umfasst das Mittel Oligonukleotide, welche mit DNA (insbesondere zfDNA oder davon z.B. durch Bisulfitkonvertierung abgeleiteter DNA) hybridisieren können, welche erfindungsgemäße Methylierungsmarker umfasst bzw. daraus besteht. Bevorzugt sind hierbei Methylierungsmarker aus den in den Ansprüchen genannten Untergruppen. Unter "hybridisieren können" ist eine spezifische Hybridisierung zu verstehen, insbesondere unter stringenten Bedingungen, wie sie etwa im experimentellen Teil geschildert sind.

Geeignete Oligonukleotide sind z.B. Oligonukleotide, welche mit den in Tabelle 1a, 1b und/oder 1c, bevorzugt in Tabelle 1a, genannten Regionen hybridisieren können, weil sie komplementär zu diesen Regionen oder einem Fragment daraus sind, welches mindestens 20 Nukleotide, z.B. bei Kopplung an einen festen Träger bevorzugt 60-352, optional 100-190 oder 135-157 Nukleotide umfasst. Die Länge hängt dabei u.a. von der Basenzusammensetzung bzw. Sequenz und der Hybridisierungstemperatur sowie der ausgewählten Technik ab. Da es sich um doppelsträngige DNA handelt, können die Oligonukleotide zu dem Strang in 5'-3' Richtung oder zu dem Strang in 3'-5' Richtung komplementär sein, oder zu beiden. Wichtig ist, dass die ausgewählten Oligonukleotide nicht mit anderen als den in den Tabellen genannten Regionen hybridisieren können, was ebenfalls eine Voraussetzung für eine spezifische Hybridisierung ist. Beispielhafte geeignete Oligonukleotide, die mit den in Tabelle 1a, 1b und 1c genannten Regionen auf Chromosom 1 hybridisieren können, sind in Tabelle 5 aufgeführt.Der Fachmann ist in der Lage, auf Basis der hierin offenbarten Informationen über die Marker auch für andere Marker geeignete Oligonukleotide auszuwählen.

Solche Oligonukleotide können optional weitere Bestandteile umfassen, z.B. spacer oder linker-Regionen.

Die erfindungsgemäßen Oligonukleotide können z.B. an einen festen Träger gekoppelt werden, oder sind Oligonukleotide, die an einen festen Träger gekoppelt sind. Eine solche Kopplung ist z.B. über Adaptoren oder Tags möglich. Eine Option dafür ist die Kopplung an Biotin, welches an Streptavidin oder Avidin binden kann (oder bereits gebunden ist), das an den festen Träger gekoppelt ist.

Der feste Träger kann z.B. ein Genchip, ein Kügelchen oder *Bead*, z.B. ein magnetischer Bead oder eine Säulenmatrix sein. Der Träger erlaubt damit eine einfache Abtrennung der hybridisierten DNA. Im Beispielteil sind an magnetische *Beads* beschrieben, die über eine Streptavidin-Biotin-Bindung an Oligonukleotide gekoppelt sind, welche spezifisch mit den in Tabelle 1 genannten Regionen hybridisieren und als *Capture Probes* eingesetzt werden können. Optional umfassen die erfindungsgemäßen Mittel 638 Oligonukleotide, z.B. Capture Probes, die mit allen in Tabelle 1 genannten Methylierungsmarkern hybridisieren können.

Es kann sich alternativ oder zusätzlich bei den erfindungsgemäßen Oligonukleotiden auch um ein Kit umfassend PCR-Primern zur Amplifikation von Regionen handeln, welche die Methylierungsmarker umfassen oder (insbesondere im Fall von Regionen aus Tabelle 1) daraus bestehen. PCR-Primer weisen bevorzugt eine Länge von ca. 12-40, optional 15-25 Nukleotiden auf, welche mit den genannten Regionen hybridisieren können. Ein solches Kit kann auch Blockierungs-Oligonukleotide oder Detektionssonden umfassen, welche nach Bisulfitkonvertierung spezifisch an vorher methylierte oder unmethylierte DNA binden können. Solche Oligonukleotide können z.B. in PCR-basierten erfindungsgemäßen Verfahren eingesetzt werden.

Eine Analyse per PCR ist vor allem zweckmäßig, wenn nur eine begrenzte Anzahl an Markern analysiert werden soll, also z.B. die Marker in den oben genannten Genen. Bevorzugt werden mit diesem Verfahren die in Tabelle 2 definierten Marker analysiert, alternativ oder zusätzlich auch die in Tabelle 3 definierten Marker, so dass entsprechend geeignete Oligonukleotide ausgewählt werden können.

Optional können ein oder mehrere für Multiplex-PCR geeignete Primer ausgewählt werden. Sonden zur Detektion sind bevorzugt mit geeigneten Farbstoffen markiert.

Die Erfindung stellt auch ein Verfahren zur Verfügung, bei dem die erfindungsgemäßen Mittel für eine Diagnose von Lungenkrebs in einer Probe eines Patienten einsetzt werden, wobei optional zfDNA aus einer Flüssigbiopsie-Probe eines Patienten (auch: Subjekts) untersucht wird. Aufgrund der Auswahl der Marker können aber auch andere Proben, z.B. aus Bronchoskopien, mit den erfindungsgemäßen Mitteln untersucht werden, insbesondere mit solchen, die Marker aus Tabelle 1 a, b und/oder c umfassen, bevorzugt alle Marker aus Tabelle 1a und 1b, optional auch aus Tabelle 1c.

Sollen Sequenzierungsdaten verwendet werden, so stellt die bioinformatische Auswertepipeline ein weiteres Problem dar. Die herkömmlichen gDNA-WGBS-Libraries werden nach dem Prozessieren meist mit dem "Bismarck" Algorithmus *aligned.* Die Ergebnisse des *Alignments* können dann anschließend von zahlreichen Auswertepipelines analysiert werden, wobei genomweite DNA-Methylierungssignaturen extrahiert werden. Das in den Ausführungsbeispielen durchgeführte WGBS-Experiment der zirkulierenden DNA war das erste seiner Art. Dabei stellte sich heraus, dass die zfDNA-*Libraries* eine andere Komplexität sowie Fragmentverteilung als herkömmliche gDNA-*Libraries* besitzen (siehe Abschnitt 1.1.2.5). Dies könnte der Grund dafür sein, dass der am häufigsten verwendete "Bismarck" Algorithmus eine nicht-zufriedenstellende *Mapping* Effizienz von nur 70% lieferte. Aus diesem Grund wurden weitere Algorithmen ausgetestet. Die besten Ergebnisse, mit einer *Mapping* Effizienz von mindestens 98%, lieferte hierbei der "Segemehl" Algorithmus (siehe Abschnitt 1.1.2.5).

Daher wird in der Ausführungsform der Erfindung, die auf Sequenzierung bisulfit-konvertierter zfDNA beruht, besonders der Segemehl-Algorithmus zum Alignment (also zur Anordnung) der Sequenzierungsinformationen der zfDNA gegenüber einem Referenzgenom verwendet. Der Segemehl-Algorithmus findet sich unter https://www.bioinf.uni-leipzig.de/Software/segemehl/. und wird z.B. in Otto et al. genauer beschrieben (Otto et al. [2012] Bioinformatics 28: 1698-1704). Es kann, wie im unten geschilderten Beispiel, Version 0.2.0 verwendet werden, aber auch eine andere Version, wie z.B. 0.3.4.

Gegenstand der Erfindung ist auch ein erfindungsgemäßes Verfahren zur Diagnose eines Lungentumors, welches folgende Schritte umfasst:
a. Extraktion von zfDNA aus einer Flüssigbiopsie-Probe oder genomischer DNA aus einer soliden Gewebeprobe, bevorzugt von zfDNA aus einer Flüssigbiopsie-Probe,
b. Durchführung einer Bisulfitkonvertierung,
c. Herstellung einer Whole Genome Bisulfite Sequencing Library,
d. Anreicherung der die definierten Methlierungsmarker umfassenden DNA-Regionen, wobei diese bevorzugt mit einem erfindungsgemäßen Mittel zur Diagnose in Kontakt gebracht werden,
e. Sequenzierung der angereicherten DNA-Regionen,
f. Alignment der Sequenzierungsdaten gegen ein Referenzgenom unter Verwendung des Segemehl Algorithmus,
g. Berechnung der Methylierungsraten.

Mittel und Verfahren zur Extraktion von genomischer DNA, zur Extraktion von zfDNA aus dem Plasma, Quantifizierung, Qualitätskontrolle (QC) sowie Bisulfitkonvertierung sind dem Fachmann aus dem Stand der Technik bekannt und/oder hierin beschrieben.

Die konvertierte DNA, bevorzugt zfDNA, kann für die Herstellung der Libraries verwendet werden. Die Library Präparation erfolgt in zwei Schritten. Im ersten Schritt wird, z.B. wie im Abschnitt 1.1.2.4 beschrieben, von jeder Probe eine WGBS *Library* hergestellt, welche Informationen über das gesamte Methylom bzw. bevorzugt zfDNA-Methylom des entsprechenden Patienten enthält. Da im weiteren Verlauf jedoch nur die bestimmten, differentiell methylierten Regionen sequenziert und analysiert werden, können diese aus dem gesamten Methylom angereichert werden. Dies kann als zweiter Schritt auf Basis der Whole Genome Bisulfite Sequencing Library erfolgen.

Es können für die Anreicherung verschiedene erfindungsgemäße Sätze von Methylierungsmarkern eingesetzt werden, z.B. die erstmalig im Rahmen der vorliegenden Arbeit in zfDNA identifizierten Marker aus Tab. 1a, alle Marker aus Tabelle 1a, alternativ oder zusätzlich die Marker aus Tabelle 1b und/oder 1c. Es ist aber auch möglich, nur Methylierungsmarker einzusetzen, bei denen im Rahmen der Klassifikation, insbesondere für das Vorhandensein eines Tumors (Tabelle 2) oder die Bestimmung der Entität des Tumors (Tabelle 3), aber optional auch für die Bestimmung des Tumorstadiums (Tabelle 4), eine besondere Bedeutung gefunden wurde.

Zur Anreichung können z.B. *Capture Probes* eingesetzt werden. Diese *Capture Probes* können das gesamte *Plasma-Panel* oder Teile davon abdecken (siehe Abschnitt 1.2.1).

Die angereicherte *Library* kann einer QC unterzogen sowie quantifiziert werden (siehe Abschnitt 1.1.2.2). Sie wird bevorzugt sequenziert, z.B. auf dem "MiSeq" ("Illumina", USA) (siehe Abschnitt 1.2.2). Die Sequenzierdaten können z.B. im "FastQ"-Format gespeichert und anschließend analysiert werden (siehe z.B. Abschnitt 1.2.3). Bevorzugt soll nicht das gesamte Methylom analysiert werden, sondern nur definierte Methylierungsmarker. Bevorzugte Methylierungsmarker sind z.B. die in Tabelle 1 bestimmten 638 Regionen.

Für die Analyse wird, wie erwähnt, insbesondere der Segemehl-Algorithmus zum Alignment gegen ein Referenzgenom eingesetzt. Danach werden die Methylierungsmuster berechnet.

Das Format des "Segemehl"-*Output*-Files ist ein anders als das typische "Bismarck"-Format. Daher kann ggf. eine geeignete, mit "Segemehl" kompatible Analysepipeline eingesetzt werden. Beispielhaft kann in diesem Kontext z.B. das "Bisulfite Analysis Toolkit" genannt werden. Diese modular aufgebaute Software kann auf zahlreichen Rechenclustern verwendet und durch weitere Software sowie eigene Skripte erweitert werden. Für die Identifikation der für Lungenkrebsdiagnose geeigneten, differentiell methylierten Marker kann die Analysepipeline mit eigenen bioinformatischen Skripten ergänzt werden, z.B. den hierin offenbarten.

Alternativ zu dem Diagnoseverfahren mittels Sequenzierung ist es auf Basis der erfindungsgemäßen Ergebnisse auch möglich, eine Analyse über PCR durchzuführen. Dies ist vor allem für kleinere Untergruppen der bestimmten Marker relevant, z.B. wenn zunächst eine Probe eines Patienten nur auf das Vorhandensein eines Tumors und/oder die Bestimmung der Tumorentität untersucht werden soll. In diesem Fall können z.B. geeignete Primer eingesetzt werden, um Regionen der zfDNA zu amplifizieren und die in Tabelle 2 und/oder 3 genannten Positionen nachzuweisen. Dies kann aus aufgereinigter, bisulfit-konvertierter DNA z.B. mittels Realtime-PCR erfolgen. Es können aber auch Multiplex-PCRs oder parallele Ansätze eingesetzt werden.

Als interne Kontrolle kann z.B. beta-Aktin analysiert werden, um zu prüfen, ob die Menge an Gesamt-DNA in der Probe ausreichend ist. Dafür kann zfDNA aus einer Flüssigbiopsie, bevorzugt aus Plasma, z.B. wie in den Ausführungsbeispielen beschrieben, aufgereinigt, bisulfitkonvertiert und wiederum aufgereinigt werden. Es können für die PCR ferner Blocker und Detektionssonden eingesetzt werden, die spezifisch die bisulfit-konvertierten, unmethylierten Sequenzen innerhalb der Regionen erkennen und deren Amplifikation blockieren, so dass die methylierten Sequenzen bevorzugt amplifiziert werden. Methylierungsspezifische Sonden detektieren dann ausschließlich methylierte Sequenzen, die während der PCR amplifiziert wurden.

Vergleichbare Verfahren sind bereits beschrieben, z.B. für das Epi proLung Kit (Epigenomics AG, Berlin) und können für die erfindungsgemäß relevanten Methylierungsmarker z.B. aus Tabelle 2 und 3 adaptiert werden. Selbstverständlich ist es auch möglich, weitere Methylierungsmarker zusätzlich mit diesem Verfahren zu untersuchen, z.B. mehr als 25 differentiell methylierte Positionen oder mehr als 30 differentiell methylierte Positionen, wobei diese bevorzugt die in Tabellen 2 und 3 genannten Methylierungsmarker umfassen und/oder in den in Tabelle 1 genannten Regionen liegen, bevorzugt beides.

Die in der Probe eines Patienten (über Sequenzierungs-basierte Verfahren oder PCR-basierte Verfahren) festgestellten Methylierungsmuster, d.h. die Ergebnisse der Methylierungsmarker-Analyse, können mit den hierin bekannten Mustern für Tumore, optional einer bestimmten Entität und/oder eines bestimmten Stadiums korreliert werden, wie z.B. in den Tabellen angegeben. Dies lässt erfindungsgemäß Aussagen über das Vorhandensein, die Entität, das Stadium und/oder die Prognose eines Lungentumors zu und gestattet so eine zuverlässige Diagnose.

Erfindungsgemäß kann diese Diagnostik eingesetzt werden, um bei Vorhandensein eines Tumors eine Therapie auszuwählen bzw. über die Einleitung einer Therapie zu entscheiden.

In einer Ausführungsform betrifft die Erfindung damit auch ein Verfahren zur Behandlung eines Lungentumors, welches ein erfindungsgemäßes Diagnoseverfahren umfasst, wobei bei Vorliegen eines Tumors dieser Tumor behandelt wird. Vorteilhafterweise kann auch die Entität des Tumors festgestellt werden, wodurch eine z.B. für ein Adenokarzinom oder ein Plattenepithelkarzinom geeignete Therapie ausgewählt werden kann. Eine geeignete Therapie kann z.B. die Verabreichung von geeigneten Medikamenten oder Kombinationen von Medikamenten und/oder eine Bestrahlung umfassen.

Alternativ kann das Diagnoseverfahren eingesetzt werden, um bei Nachweis eines Tumors weitere Diagnoseschritte, wie die Entnahme einer festen Biopsie und oder bildgebende Verfahren durchzuführen.

Gegenstand der Erfindung ist auch eine Verwendung eines erfindungsgemäßen Verfahrens oder eines erfindungsgemäßen Mittels zur Diagnose von Lungenkrebs, wobei die Diagnose eine Aussage über das Vorhandenseins eines Tumors, über die Entität eines Tumors, über das Tumorstadium und/oder über die Prognose erlaubt, bevorzugt über Vorhandensein und Entität des Tumors, optional über alles gleichzeitig.

Zusammenfassend lässt sich sagen, dass es im Rahmen der vorliegenden Erfindung erstmalig gelungen ist, ein NGS-*Panel* zu entwickeln, das auf genomweiten zfDNA-Methylierungssignaturen aus Plasma basiert. Dieses *Plasma-Panel* konnte unter Verwendung von *Liquid Biopsies* einer Patientenkohorte (n=12) erfolgreich validiert werden. Während der Pilot-Studie differenzierte das Plasma *Panel* mit 100%iger Richtigkeit maligne Lungentumore bereits ab Stadium I, identifizierte die häufigsten NSCLC-Subtypen und lieferte weitere Informationen bezüglich der Bestimmung des Stadiums der Lungentumore (*Staging*).

Die Erfindung wird im Folgenden durch Beispiele erläutert, die die Erfindung illustrieren, aber nicht einschränken sollen. Alle in dieser Anmeldung zitierten Referenzen werden durch die Bezugnahme vollumfänglich hierin aufgenommen.

### Legende

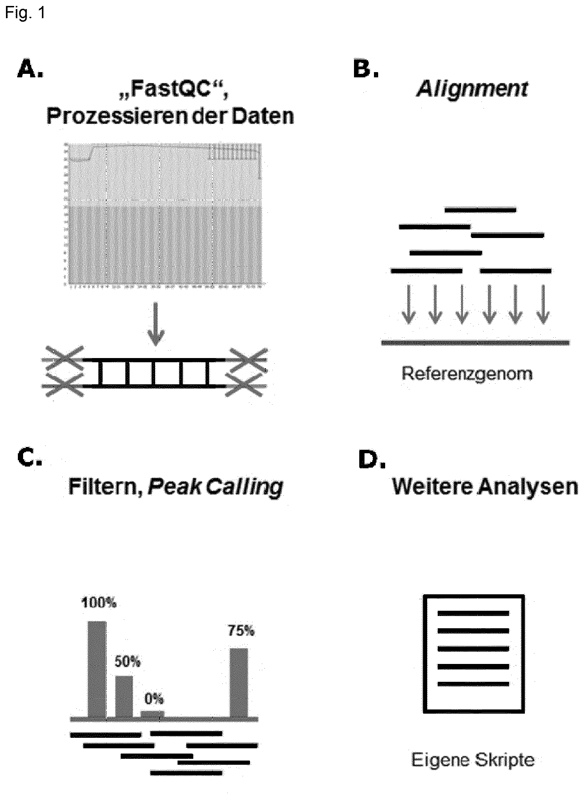

**Fig. 1****:** Die Auswertung der WGBS Sequenzierdaten erfolgte in mehreren Schritten. **A.** Zunächst wurden die Daten einer QC (z.B. mit FastQC) unterzogen und anschließend prozessiert. **B.** Dann wurden die prozessierten Daten gegen ein Referenzgenom (z.B. "HG19") *aligned* und anschließend **C.** zum Berechnen der DNA-Methylierungsraten verwendet. Die Positionen, an denen eine Methylierungsrate ermittelt wurde, wurden dann nach bestimmten Kriterien gefiltert (z.B. *Coverage* und CpG Kontext) und schließlich **D.** weiteren Analysen unter Verwendung eigener Skripte unterzogen.

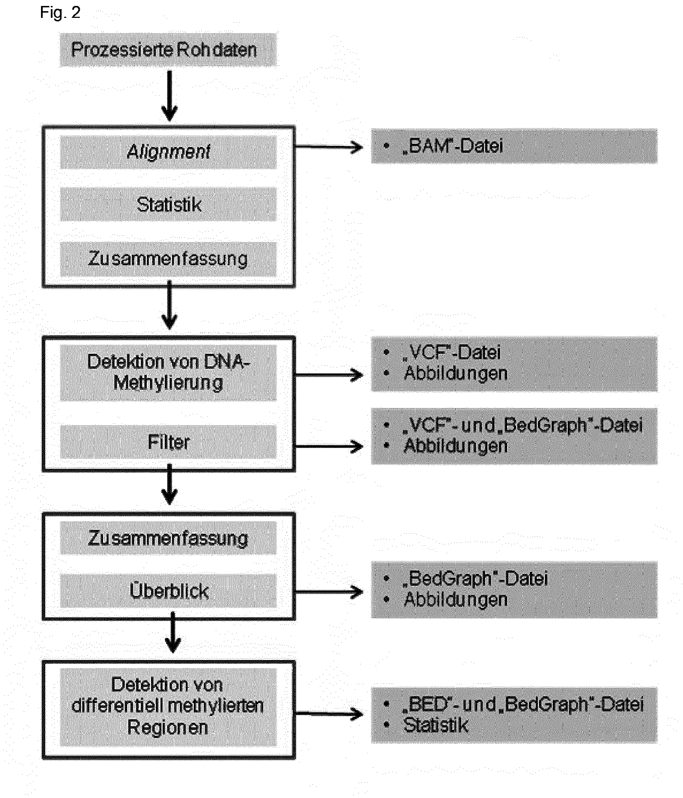

**Fig. 2****:** Prozessierte Sequenzierdaten wurden gegen das "HG19" Referenzgenom aligned, wobei das "Bisulfite Analysis Toolkit" unter Verwendung des Segemehl-Algorithmus eingesetzt wurde. Des Weiteren erfolgten die Detektion von DNA-Methylierungsraten und differentiell methylierter Regionen sowie das Erstellen von Übersichtsgraphiken.

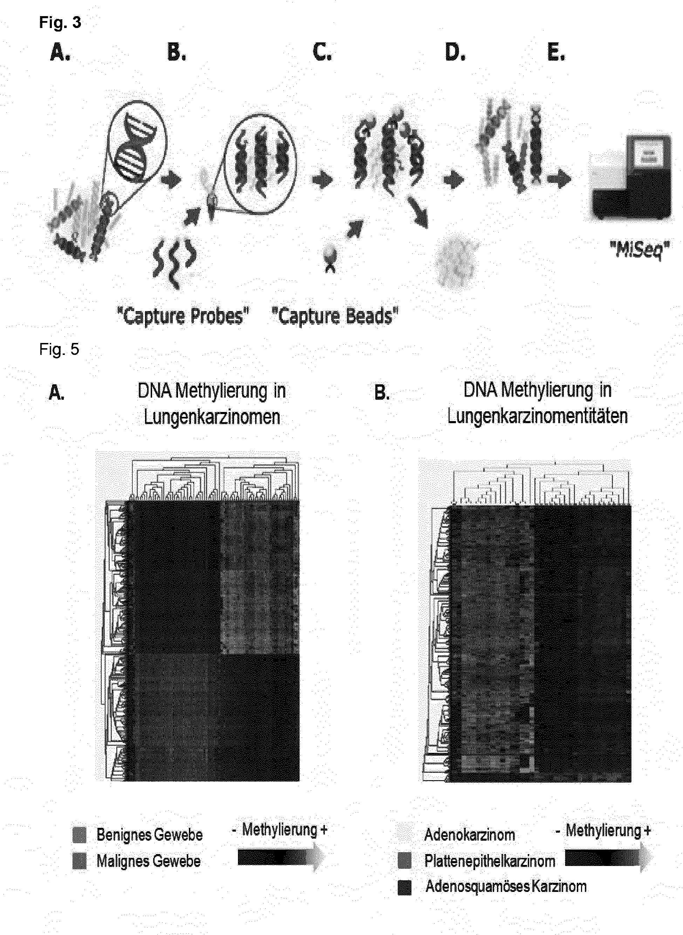

**Fig. 3****:** Die Anreicherung von erfindungsgemäß wichtigen, differentiell methylierten Regionen des Satzes von Methylierungsmarkern war in mehrere Schritte unterteilt. **A.** zunächst wurden, z.B. wie im Abschnitt 1.1.2.4 beschrieben, WGBS *Libraries* hergestellt. Zur Validierung können diese äquimolar gepoolt werden; wenn dies zur Diagnose von Patienten durchgeführt wird, was vom Sequencer und dessen Kapazität sowie dem Probenaufkommen abhängt, dann können Einzelproben durch ein "Barcoding" individuell markiert und gemeinsam squenziert werden, um die Proben dann wieder bioinformatisch zu trennen. **B.** Die 638 differentiell methylierten Regionen wurden dann, hier mit dem "SeqCap Epi Enrichment Kit", an "Capture Probes" hybridisiert, **C.** unter Verwendung von "Capture Beads" angereichert und schließlich **D.** in einer PCR-Reaktion amplifiziert. **E.** Die fertigen NGS *Libraries* wurden dann quantifiziert, einer QC unterzogen und auf dem "MiSeq" sequenziert.

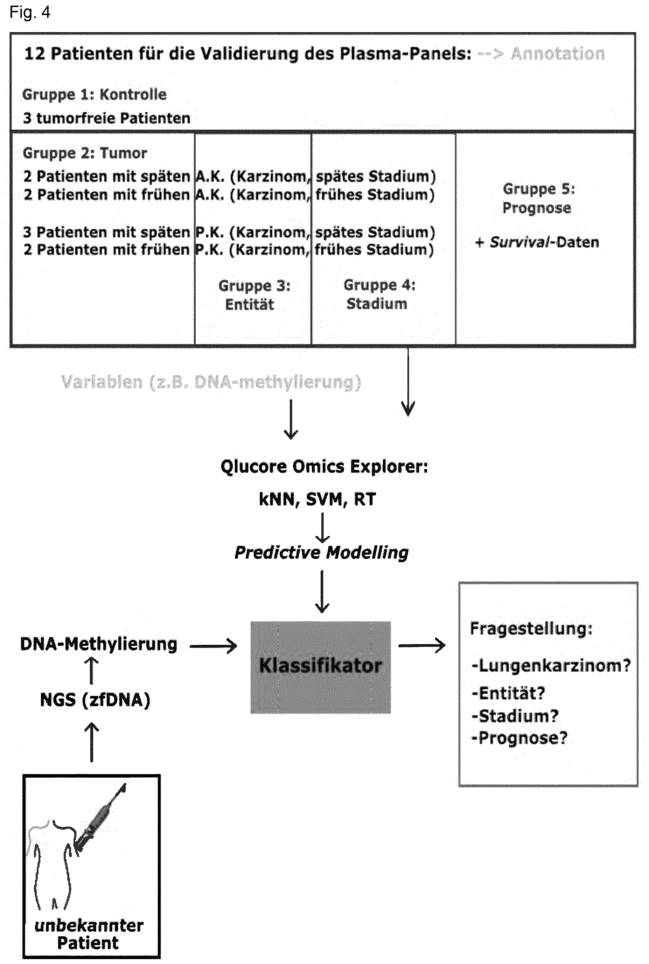

**Fig. 4****:** Das Funktionsprinzip eines Klassifikators. Aus den Daten der Validierungskohorte (12 Patienten) wird zunächst ein Annotationsfile generiert, welcher zusätzlich mit den ermittelten DNA-Methylierungsraten der im *Plasma-Panel* enthaltenen Regionen (siehe Tabelle 1) in die "Qlucore Omics Explorer" Software geladen wird. Die DNA-Methylierungsdaten (Variablen) und der Annotationsfile werden von implementierten Algorithmen ("k-Nearest Neighbors Algorithm" (kNN), "Support Vector Machines" (SVM) und "Random Trees" (RT)) dazu verwendet, ein optimales Model zu erstellen. Dieser Vorgang wird als *Predictive Modelling* bezeichnet. Nachdem der optimale Klassifikator generiert ist, ist dieser in der Lage, das zfDNA-Methylierungsmuster eines unbekannten Patienten zu analysieren und somit eine Diagnose zu stellen (Adenokarzinom (A.K.), Plattenepithelkarzinom (P.K.)).
**Fig. 5****:** Ergebnisse der differentiellen Methylierungsanalyse mit HM 450K. Die hierarchische Clusteranalyse von 40 OP-Präparaten und deren korrespondierenden Kontrollen identifizierte **A.** 898 differentiell methylierte CpG *Loci* in Tumorproben (q< 1 ×10⁻²³, σ/σₘₐₓ> 0,4) (linke Hälfte: ganz links drei Tumorproben, dann benignes Gewebe, rechte Hälfte Tumorgewebe) und **B.** 1.167 differentiell methylierte CpG *Loci* in unterschiedlichen Lungenkarzinomentitäten (FDR < 1 × 10⁻⁴) (heller oberer Rand: Adenokarzinom, grauer oberer Rand: Plattenepithelkarzinom, dunkler oberer Rand: Adenosquamöses Karzinom.Ergebnisse: dunkel: wenig Methylierung, hell: viel Methylierung).

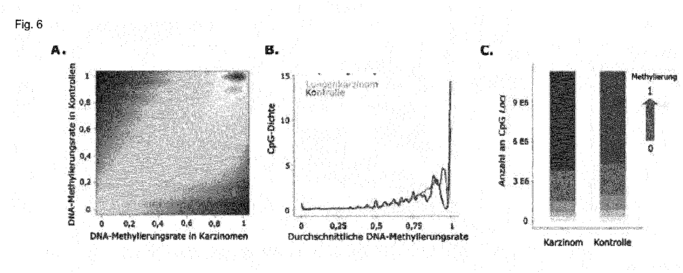

**Fig. 6****:** Die mit den "BAT_calling" und "BAT_filter_vcf" Modulen ermittelten DNA-Methylierungsraten wurden in das "BAT_summarize" Modul des "Bisulfite Analysis Toolkit" geladen. **A.** Das Streudiagramm zeigt deutlich, dass die Lungenkarzinomgruppe anhand des DNA-Methylierungsmusters von der Kontrollgruppe (tumorfreie Patientenkohorte) unterschieden werden kann. **B.** Die mittlere sowie **C.** die gestaffelte Darstellungen der DNA-Methylierungsraten pro Gruppe verdeutlichen die genomweite Hypermethylierung der Lungenkarzinomgruppe im Vergleich zur Kontrollgruppe.

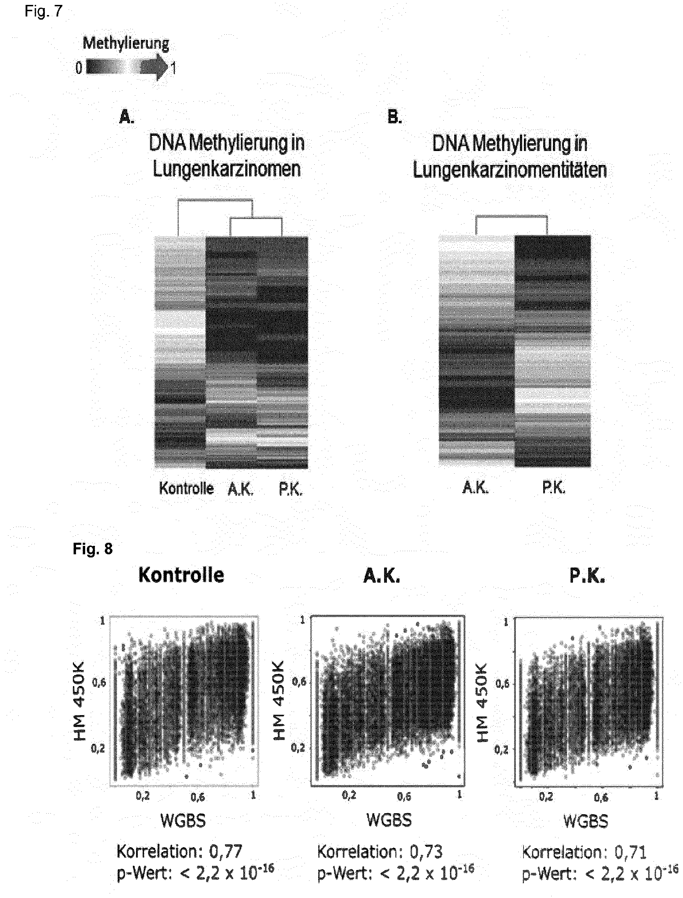

**Fig. 7****:** Die ermittelten zfDNA-Methylierungsmuster wurden normalisiert und einer hierarchischen Clusteranalyse unterzogen. Dabei wurden **A.** 18.000 für das Lungenkarzinom und **B.** 44.000 für die jeweilige Entität spezifische differentiell methylierte CpG *Loci* identifiziert (Adenokarzinom (A.K.), Plattenepithelkarzinom (P.K.)).
**Fig. 8**: "Pearson"-Korrelationsanalyse der mit beiden Methoden (HM 450K und WGBS) detektierten DNA-Methylierungswerte (Adenokarzinom (A.K.), Plattenepithelkarzinom (P.K.)).

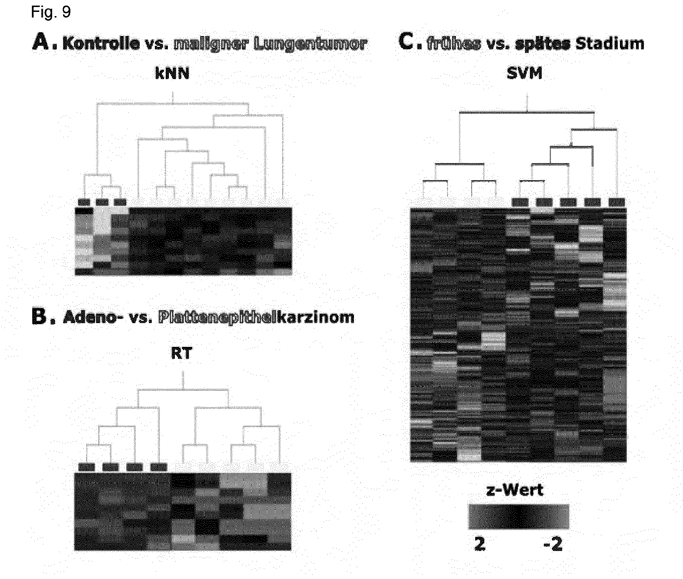

**Fig. 9****:** Die ermittelten zfDNA-Methylierungsraten wurden in die "Qlucore Omics Explorer" Software geladen und unter Verwendung folgender Klassifikationsalgorithmen analysiert: "k-Nearest Neighbors Algorithm" (kNN), "Support Vector Machines" (SVM) und "Random Trees" (RT). Ein hoher z-Wert steht für eine starke Methylierung. **A.** Der kNN Algorithmus konnte unter Analyse von 10 differentiell methylierten Positionen (Markern) die gesunden (Kontrolle) von den an einem malignen Lungenkarzinom erkrankten Patienten unterscheiden. Sowohl die frühen (I, II) als auch die späten (III, IV) Lungenkarzinomstadien wurden mit 100%iger Richtigkeit klassifiziert (helle Balken an Oberseite der Abbildung: maligner Lungentumor, dunkler Balken (3 Spalten links): Kontrolle). Bei 9 der 10 Positionen findet sich im Tumorgewebe eine stärkere Methylierung, bei einer eine schwächere. **B.** Der RT Algorithmus analysierte 10 Positionen, um mit einer 100%igen Richtigkeit die Entität des Tumors zu ermitteln (helle Balken an Oberseite der Abbildung (6 Spalten rechts): Plattenepithelkarzinom, dunkle Balken (4 Spalten links): Adenokarzinom). Bei allen gezeigten Markern findet sich beim Adenokarzinom eine stärkere Metylierung als beim Plattenepithekarzinom. **C.** Die späten Tumorstadien (III, IV) konnten mit einer 80%igen Richtigkeit mit dem SVM Algorithmus identifiziert werden, dabei wurden 523 Positionen analysiert ((helle Balken an Oberseite der Abbildung (4 Spalten links): frühes Stadium (I, II), dunkle Balken an der Oberseite der Abbildung (5 Spalten rechts): spätes Stadium (III, IV)). Dabei sind die ausgewerteten Positionen z.T. in den frühen, z.T. in den späten Stadien stärker methyliert.

### Beispiele

### 1.1 Methoden: "Entwicklung des Plasma-Panels"

Um eine nichtinvasive Lungenkrebsdiagnostik zu ermöglichen, wurde im Rahmen der Erfindung ein geeignetes *Panel,* also ein Satz von Methylierungsmarkern, zur DNA-Methylierungsanalyse im Blutplasma entwickelt. Der Satz von Methylierungsmarkern wird daher auch als *Plasma Panel* bezeichnet. Die Entwicklung des *Plasma Panels* erfolgte in drei voneinander unabhängigen Ansätzen. Im ersten Ansatz wurde geprüft, ob die DNA-Methylierung sich generell als Biomarker für die Lungenkrebsdiagnostik eignet (siehe Abschnitt 1.1.1). Hierfür wurden 40 Lungenkarzinome sowie deren korrespondierenden Kontrollen unter Verwendung des "Illumina Infinium Human Methylation450K BeadChips" (HM 450K) analysiert. Die Methode identifizierte deutliche, tumorspezifische DNA-Methylierungssignaturen. Als nächstes wurden, wie im Abschnitt 1.1.1 beschrieben, die Regionen mit den stärksten DNA-Methylierungsunterschiede ermittelt und in das *Plasma Panel* aufgenommen.

Im zweiten Ansatz wurde untersucht, ob tumorspezifische DNA-Methylierungssignaturen auch im Blutplasma der betroffenen Patienten detektiert werden können (siehe Abschnitt 1.1.2). Dafür wurde zirkulierende, zellfreie DNA aus dem Plasma von Adeno- (n=5) und Plattenepithelkarzinompatienten (n=4) extrahiert und anschließend zu 3 *Pools* vereinigt. Als Kontrolle diente Plasma einer tumorfreien Patientenkohorte (n=19). Die detaillierten Informationen zu den Patienten sind im Abschnitt 1.1.2 zusammengestellt. Durch das *Poolen* wurden individuelle DNA-Methylierungsmuster weitgehend eliminiert, und die allgemeinen tumor- bzw. lungenspezifischen Signaturen dagegen hervorgehoben. Dann wurden die zfDNA-*Pools* einer genomweiten Bisulfitsequenzierung unterzogen (engl. *whole genome bisulfite sequencing* (WGBS), siehe Abschnitt 1.1.2.4). Die Methode detektierte mehrere tausend aberrant methylierte CpG *Loci,* die nicht nur tumor- sondern auch entitätsspezifisch waren. Davon wurden die am besten geeigneten Regionen für die Differenzierung für das Plasma *Panel* ausgewählt (siehe Abschnitt 1.1.2.5.5). Da die Diagnose erfindungsgemäß bevorzugt anhand von Flüssigbiopsien vorgenommen werden soll, sind die hier identifizierten Methylierungsmarker von besonderer Bedeutung.

Im dritten Ansatz wurde das Plasma *Panel* mit 59 tumorspezifischen sowie prognostisch relevanten CpG *Loci* aus weiteren Studien ergänzt (siehe Abschnitt 1.1.3).

### 1.1.1 Nachweis der aberranten DNA-Methylierung in primärem Tumorgewebe

Der HM 450K Datensatz enthielt Informationen über den Methylierungsstatus von 40 Lungenkarzinomen (Adeno- und Plattenepithelkarzinome) und deren korrespondierenden Kontrollen. Der Datensatz wurde mit der "Qlucore Omics Explorer" Software (Version 3.2, "Qlucore", Schweden) ausgewertet und ergab:
1.) 897 CpG *Loci* (T-Test: FDR < 1 × 10⁻²³, σ/σₘₐₓ > 0,4), die zwischen dem Tumor- und gesundem Lungengewebe differenziert methyliert waren.
2.) 1.167 CpG *Loci* (T-Test: FDR < 1 × 10⁻⁴), die zwischen dem Adeno- und Plattenepithelkarzinomgewebe differenzierten.

Um die CpG *Loci* mit den stärksten DNA-Methylierungsunterschieden zu ermitteln, wurden die beiden Listen zunächst nach differentieller Methylierung größer 35% (avg.beta > 0,35) gefiltert und unter Verwendung von "Bedtools" [169] (Version 2.2.6, "The University of Utah", USA) gegen das "HG19" Referenzgenom annotiert. Alle CpG *Loci,* die innerhalb von häufigen SNPs (≥1% der Bevölkerung) lokalisiert waren und nicht proteinkodierend waren, wurden verworfen. Die übrig gebliebenen *Loci* wurden in das finale Plasma *Panel* aufgenommen (Tab. 1).

### 1.1.2 Nachweis der aberranten DNA-Methylierung in Blutplasma

Die zirkulierende zellfreie DNA wird erfindungsgemäß zur nichtinvasiven Diagnostik von soliden Tumoren genutzt. Falls ein Patient an einer malignen Tumorerkrankung leidet, ist in der Gesamtmenge der zirkulierenden DNA auch die Tumor-DNA enthalten, welche alle therapeutisch und prognostisch relevanten Informationen über die genetischen und epigenetischen Charakteristika des Tumors enthält. Daher muss die zfDNA aus dem Blut bzw. Blutplasma isoliert werden. Da zfDNA nur in einer sehr geringen Menge aus dem Blutplasma extrahiert werden kann, wurde hierfür eine Methode gewählt, die sehr spezifisch und effizient die zfDNA anreichert, ohne dabei weitere Bestandteile des Plasmas zu isolieren.

Dafür kann z.B. das "PME free-circulating DNA Extraction Kit" ("Analytik Jena", Deutschland, siehe Abschnitt 1.1.2.1) eingesetzt werden. Es enthält ein Polymer, welches nur sehr spezifisch kurzsträngige dsDNA-Fragmente komplexiert. Der Polymer-zfDNA-Komplex wird anschließend ausgefällt und aufgereinigt. Nach der Aufreinigung kann die Komplexverbindung gelöst werden. Die dabei freigegebene DNA wird in weiteren Schritten, z.B. über Bindung an eine Silica-Säule, vom Polymer gereinigt und aufkonzentriert. Auch andere Methoden, die z.B. auf dem gleichen oder ähnlichen Wirkprinzipien beruhen, können eingesetzt werden. Das dabei entstehende Produkt ist sehr sauber und kann auch für empfindliche NGS-basierte Analysemethoden wie z.B. WGBS verwendet werden.

### 1.1.2.1 Extraktion der zirkulierenden, zellfreien DNA (zfDNA) aus Blutplasma

Blutplasma wurde präpariert und auf Trockeneis verschickt. Hierfür wurde das Vollblut innerhalb von 30 min nach der Entnahme bei 1.500g 10 min lang zentrifugiert. Nach der Zentrifugation wurde der Plasmaüberstand vorsichtig abpipettiert, auf "CryoPure" Gefäße ("Sarstedt AG&Co", Deutschland) verteilt und sofort bei -80°C eingefroren.

Die eingefrorenen Plasmaproben wurden langsam unter lauwarmem Wasser aufgetaut und anschließend bei 4.500g 10 min lang zentrifugiert. Das Pellet wurde verworfen, der klare Überstand in ein 10 mL Röhrchen überführt und mit dem "PME free-circulating DNA Extraction Kit" gemäß den Weisungen des Herstellers prozessiert.

### 1.1.2.2 Quantifizierung und Qualitätskontrolle (QC) der extrahierten zfDNA

Die zfDNA wurde fluorometrisch unter Verwendung des "Qubit dsDNA High Sensitivity Assay Kit" ("Thermo Fisher Scientific", USA) quantifiziert. Hierfür wurde jeweils 1 µL der Probe mit den 198 µL "Qubit dsDNA HS Buffer" sowie 1 µL "Qubit dsDNA HS Reagent" vermischt, 2 min lang inkubiert und anschließend im "Qubit 2.0" Fluorometer ("Thermo Fisher Scientific", USA) vermessen. Bei dem "Qubit dsDNA HS Reagent" handelte es sich um einen Farbstoff, der unter normalen Bedingungen ein sehr schwaches Fluoreszenzsignal erzeugt. Bei Vorhandensein von doppelsträngiger DNA (dsDNA) interkaliert es jedoch in die dsDNA, verändert seine Struktur und erzeugt ein starkes Fluoreszenzsignal. Dabei wird weder einzelsträngige DNA (ssDNA) noch RNA gebunden. Somit korreliert die Signalintensität ausschließlich mit der in der Probe vorhandenen Menge an dsDNA.

Die Qualität der extrahierten zfDNA wurde mit Hilfe des "Agilent 2100 High Sensitivity DNA Kit" ("Agilent", USA) analysiert. Bei der Methode handelte es sich um eine Kapillar-Gelelektrophorese. Zunächst musste das "Gel-Dye Mix" vorbereitet werden. Dabei wurden 300 µL der Gelmatrix mit 15 µL des Farbstoffkonzentrats versetzt, gemischt und auf einen "Spin Filter" gegeben. Die Zentrifugation erfolgte 10 min lang bei 2.240g. Als nächstes wurde der DNA-Chip in der "Priming Station" platziert und äquilibriert. Dafür wurden 9 µL des "Gel-Dye Mix" in das für den Äquilibriervorgang vorgesehene *Well* pipettiert. Der Stempel der "Priming Station" wurde auf einen Milliliter justiert. Nachdem die "Priming Station" fest verschlossen war, wurde der Stempel eine Minute lang heruntergedrückt. Schließlich wurden die restlichen *Wells* des Chips nach Angabe des Herstellers beladen. Der Chip wurde 1 min inkubiert und direkt danach gemessen. Während der Inkubationszeit interkalierte ein im "Gel-Dye Mix" enthaltener fluoreszierender Farbstoff zwischen den Basen der dsDNA. Die dsDNA Fragmente wurden anschließend durch die mikroskopisch kleinen Kapillaren des "Agilent 2100 Bionalyzer" ("Agilent", USA) gezogen und dabei nach Fragmentgröße aufgetrennt und detektiert.

### 1.1.2.3 Bisulfitkonvertierung der zfDNA

Für die genomweite Analyse des DNA-Methylierungsmusters z.B. durch den HM 450K oder die WGBS, wird DNA einer genomweiten PCR-basierten Amplifikation unterzogen. Die DNA-Polymerasen können nicht zwischen Cytosinen und 5-Methylcytosinen unterscheiden, so dass während der Reaktion alle 5-Methylcytosine durch Cytosine ersetzt werden. Die neu synthetisierten Stränge werden nicht erneut methyliert.

Um Cytosine von 5-Methylcytosinen unterscheiden zu können, wird die Probe vor der PCR einer Behandlung mit Natriumbisulfit unterzogen. Dieser Prozess wird als Bisulfitkonvertierung bezeichnet, dabei werden alle unmethylierten Cytosine in Uracile umgewandelt. Die methylierten Cytosine bleiben dagegen unter den gewählten Reaktionsbedingungen unverändert. Die Reaktion der Bisulfitkonvertierung ist in NEB, N.E.B. Bisulfitkonvertierung (verfügbar unter: http://www.neb-online.de/wp-content/uploads/2015/04/NEB epigenetik bisulfit3.jpg) sowie in Clark et al. (Clark et al. [1994] Nucl. Acids Res 22: 2990-2997) dargestellt.

Die Bisulfitkonvertierung der zfDNA kann z.B. mit dem "EZ DNA Methylation-Gold™ Kit" ("Zymo Research", USA) erfolgen. Dafür wurden 10 ng der zuvor extrahierten zfDNA in 20 µL Wasser gelöst, mit 130 µL "CT"-Konvertierungsreagenz versetzt und im *Thermocycler* bei folgendem Programm prozessiert: 10 min 98°C, 2,5 h 64°C, bis zu 20 h bei 4°C. Im nächsten Schritt wurden die bisulfitkonvertierten Proben desulfoniert und aufgereinigt. Hierfür wurden sie mit 600 µL "M-Binding Buffer" versetzt, auf die "Zymo-Spin™ IC" Säulen pipettiert und bei 10.000g 30 s lang zentrifugiert. Dann wurden 100 µL "M-Wash Buffer" auf die Säulen gegeben. Die Säulen wurden 30 s lang bei 10.000g zentrifugiert und für 20 min mit 200 µl "M-Desulphonation Buffer" behandelt. Nach anschließender 30 s langen Zentrifugation bei 10.000g wurden die "Zymo-SpinTM IC" Säulen mit 200 µL "M-Wash Buffer" gewaschen, zur Entfernung verbliebener Flüssigkeiten für 30 s bei 10.000g zentrifugiert und die DNA mit 15 µL "Elution Buffer" bei 10.000g 30 s lang eluiert.

### 1.1.2.4 Whole Genome Bisulfite Sequencing (WGBS)

Um das zfDNA-Methylierungsprofil genomweit analysieren zu können, wurden die zuvor bisulfitkonvertierten Proben einer WGBS unterzogen. WGBS ist eine NGS-basierte Methode (engl. *next generation sequencing*). Heutzutage existieren zahlreiche Technologien, die NGS ermöglichen. Die am meisten verbreitete und auch hier verwendete NGS Technologie bietet die Firma "Illumina" (USA) an. Die zugrunde liegende Sequenzierreaktion ist fluoreszenzbasiert und erfolgt auf einem Glasträger, auch *Flowcell* genannt. Um die DNA-Fragmente an der *Flowcell* zu immobilisieren, werden zunächst spezielle "Illumina" Adapter (kurze Oligonukleotide) ligiert. Nachfolgend wird die Probe einer Denaturierungsreaktion unterzogen. Da sich auf der *Flowcell* nicht nur die Adapterbindestellen, sondern auch *Primer* befinden, kommt es zum "Umknicken" des zu sequenzierenden ssDNA-Fragments. Während der nachfolgenden PCR-Reaktion werden die DNA-Stränge vervielfältigt. Dieser Vorgang wird als *Bridge Amplification* bezeichnet. Dabei entstehen durch die fortschreitende Amplifikation an begrenzten Positionen die sogenannten Sequenziercluster, die nachfolgend dissoziieren. Nach der Clusterbildung erfolgt die eigentliche Sequenzierreaktion, bei der DNA-Basen eingebaut werden, die je nach eingebauter Base Fluoreszenzsignale unterschiedlicher Wellenlängen erzeugen. Nach jedem abgeschlossenem Einbauzyklus werden diese Fluoreszenzsignale detektiert und liefern somit die Informationen über die Basenabfolge innerhalb eines Reads.

Je nach gewünschtem Durchsatz, können unterschiedliche "Illumina" Plattformen genutzt werden. Für die Sequenzierung gezielter Regionen, sogenannten *Panels,* wie dem erfindungsgemäß identifizierten *Panel* oder Satz an Methylierungsmarkern, ist im allgemeinen die schnellere und preisgünstigere "MiSeq" Plattform ausreichend. Die die Sequenzierung kann aber z.B. auch auf den "NextSeq 500" oder "HiSeq" Plattformen erfolgen.

### 1.1.2.4.1 Erstellen der WGBS Libraries (WGBS Bibliotheken)

Während der Bisulfitkonvertierung wird DNA durch die verwendeten Reagenzien sehr stark beansprucht und somit zu einem hohen Anteil degradiert. Deswegen verwenden die herkömmlichen WGBS Protokolle sehr hohe Mengen an DNA, mindestens 500 ng. Da die zellfreie, zirkulierende DNA zum einen von Anfang an schon sehr stark fragmentiert ist und zum anderen nur in einer sehr geringen Menge gewonnen werden kann, ist die Herstellung von WGBS *Libraries* mit herkömmlichen Kits zurzeit schwierig.

Deshalb wurde für folgende Experimente das "Accel-NGS® Methyl-Seq DNA Library Kit" ("Swift Biosciences", USA) etabliert. Das Kit wurde speziell für WGBS der zfDNA entwickelt. Bereits bei zfDNA-Mengen von weniger als 10 ng können damit komplexe WGBS *Libraries* generiert werden. Die zentrale Rolle spielt dabei das Enzym "Adaptase", welches einen 10 nt langen Überhang am 3'-Ende der bisulfitkonvertierten ssDNA anfügt. Dieser Überhang ermöglicht ein besseres Ligieren der Sequenzieradapter und somit eine effizientere *Library* Herstellung. Daher wird erfindungsgemäß bevorzugt ein Verfahren zur Herstellung der WBGS Libraries eingesetzt, welches mittels des Enzyms Adaptase einen 10 nt langen Überhand am 3'-Ende der bisulfitkonvertierten ssDNA einfügt.

Die *Library* Herstellung wurde mit dem "Accel-NGS® Methyl-Seq DNA Library Kit" ("Swift Biosciences", USA) in vier Schritten durchgeführt: Behandlung mit dem Enzym "Adaptase", Extension, Ligation, PCR. Für die Behandlung mit dem Enzym "Adaptase" wurden 10 ng bisulfit-konvertierter zfDNA in 15 µL Wasser aufgenommen und bei 95°C für 2 min denaturiert. Dann wurden zu der Probe 25 µL des "Adaptase Reaction Mix" gegeben, vorsichtig gemischt und im *Thermocycler* prozessiert (Programm 1: 37°C 15 min; 95°C 2 min; 4°C; bei allen Programmen war der Deckel des Thermocyclers vorgeheizt). Als nächstes erfolgte die Extension. Hierfür wurde die Probe mit 44 µL "Extension Reaction Mix" versetzt, vorsichtig gemischt und im *Thermocycler* inkubiert (Programm 2: 98°C 1 min; 62°C 2 min; 65°C 5 min; 4°C).

Das Produkt wurde aufgereinigt. Dafür können z.B. "SPRI Beads" ("Beckman Coulter", USA) eingesetzt werden. Danach erfolgte die Ligation, für die 15 µL des Produkts mit 15 µL "Ligation I Reaction Mix" versetzt und im *Thermocycler* prozessiert wurden (Programm 3: 25°C 1 min; 4°C). Auch bei diesem Schritt wurde das fertige Produkt unter Verwendung von "SPRI Beads" ("Beckman Coulter", USA) aufgereinigt. Schließlich wurde die PCR durchgeführt. Dabei wurden pro Probe 5 µL des jeweiligen Index sowie 25 µL des "Indexing PCR Reaction Mix" hinzugegeben. Die fertige PCR-Reaktion wurde im *Thermocycler* inkubiert (Programm 4: 98°C 30 s; PCR-Zyklen: 98°C 10 s; 60°C 30 s; 68°C 1 min (7-9 Zyklen); 4°C) und über die "SPRI Beads" ("Beckman Coulter", USA) nach den Instruktionen des Herstellers aufgereinigt.

Die fertigen WGBS *Libraries* wurden wie im Abschnitt 1.1.2.2 beschrieben quantifiziert und auf ihre Qualität überprüft.

### "SPRI Beads" Aufreinigung

Die Proben wurden in 1,5 mL Eppendorf Reaktionsgefäße überführt und im vorgeschriebenen Verhältnis mit "SPRI Beads" ("Beckman Coulter", USA) versetzt (Tab. A). Dann wurden die Proben gemischt und 5 min bei Raumtemperatur inkubiert. Da die *Beads* magnetisch waren, konnte zum Pelletieren das Prinzip der magnetischen Separation genutzt werden. Hierfür wurden die Reaktionsgefäße auf einem magnetischen Ständer platziert und dann 2 min lang bei Raumtemperatur inkubiert. Nach der Inkubation wurde der Überstand entfernt, die *Beads* mit je 500 µL 80%igen Ethanol ("Merck Millipore", USA) zwei Mal gewaschen und anschließend an der Luft getrocknet. Sobald das Ethanol verdampft war, wurden die Proben vom magnetischen Ständer entnommen. Die "SPRI Beads" wurden in der vorgeschriebenen Menge "Low EDTA TE" Puffer resuspendiert (Tab. A) und 2 min lang bei Raumtemperatur inkubiert. Schließlich wurden die Proben wieder auf den magnetischen Ständer platziert. Nach ca. 2 min erfolgte eine vollständige Trennung des Überstands und der "SPRI Beads". Der Überstand enthielt das aufgereinigte Produkt, wurde abpipettiert und für den nächsten Schritt verwendet.

**Tab. A: Proben- und Reagenzvolumina für die Aufreinigungsschritte mit den "SPRI Beads".**

| **Schritt** | **Probe** | **"SPRI Beads"** | **"Low EDTA TE" Puffer** |
|---|---|---|---|
| **Extension** | 84µL | 101µL | 15µL |
| **Ligation** | 30µL | 36µL | 20µL |
| **PCR** | 50µL | 40µL | 20µL |

### 1.1.2.4.2 Sequenzierung der WGBS Libraries

Die Sequenzierung der WGBS *Libraries* erfolgte auf der "NextSeq 500" Plattform ("Illumina", USA) im "TATAA-Biocenter" (Göteborg, Schweden). Dabei wurden vier 76 *pair end* (PE) Läufe im Hochdurchsatzmodus durchgeführt.

### 1.1.2.5 Bioinformatische Auswertung der WGBS Ergebnisse

Die WGBS *Libraries* konnten aufgrund der starken Fragmentierung und geringen Mengen an zfDNA nicht mit herkömmlichen Protokollen angefertigt werden. Die mit dem "Accel-NGS® Methyl-Seq DNA Library Kit" ("Swift Biosciences", USA) hergestellten zfDNA *Libraries* wiesen somit eine andere Komplexität und Fragmentverteilung auf, als die herkömmlichen WGBS *Libraries.* Deswegen musste auch eine geeignete bioinformatische Auswertepipeline etabliert werden, um die Daten optimal analysieren zu können.

Generell müssen mehrere Schritte etabliert werden, um WGBS Daten auswerten zu können (Fig. 1). Zunächst wird die Qualität der Rohdaten überprüft. Hierfür wird am häufigsten die "FastQC" Software (Version 0.11.15, "Babraham Bioinformatics", England) verwendet (siehe Abschnitt 1.1.2.5.1). Die Software visualisiert die Qualität der Sequenzierung, Längenverteilung und Zusammensetzung der Reads. Des Weiteren werden Informationen über mögliche Adapterkontaminationen sowie Anzahl an Kmeren und PCR-Duplikaten geliefert. Als Kmere werden Sequenzen mit einer Mindestlänge von zwei Nukleotiden bezeichnet, die sich in den Rohdaten immer wieder wiederholen.

Falls die Qualitätskontrolle zufriedenstellende Ergebnisse liefert, erfolgt das Trimmen der Adaptersequenzen. Bei den zfDNA *Libraries* musste außerdem der von der "Adaptase" erzeugte 10 nt lange Überhang aus den Rohdaten eliminiert werden (siehe Abschnitt 1.1.2.5.2).

Nach dem Trimmen können die Reads gegen ein Referenzgenom der Wahl angeordnet werden, dieser Vorgang wird auch als *Alignment* bezeichnet (siehe Abschnitt 1.1.2.5.3). Für das *Alignment* sind viele Algorithmen verfügbar. Je nach Beschaffenheit der WGBS *Library* muss der passende ausgewählt und optimiert werden. Hierfür kann die *Mapping* Effizienz analysiert werden. Dabei wird berechnet wie viel Prozent an analysierten Reads dem Referenzgenom zugeordnet werden können. Für die herkömmlichen WGBS *Libraries* wird am häufigsten der "Bismarck" Algorithmus verwendet (Krueger & Andrews [2011] Bioinformatics 27: 1571-1572). Bei den hier beschriebenen zfDNA *Libraries* lieferte "Bismarck" (Version 0.15.0, "Babraham Institute", England) jedoch keine zufriedenstellende Ergebnisse (*Mapping* Effizienz von ca. 70%). Deshalb wurden weitere Algorithmen ausgetestet.

Die besten Ergebnisse mit einer *Mapping* Effizienz von mindestens 98% lieferte der "Segemehl" Algorithmus (Version 0.2.0, "Interdisziplinäres Zentrum für Bioinformatik, Universität Leipzig", Deutschland) (Otto et al. [2012] Bioinformatics 28: 1698-1704).

Nach dem *Alignment* werden die Daten nach CpG Kontext sowie der gewünschten *Coverage* (mindestens vierfach) z.B. mit dem "Bisulfite Analysis Toolkit" (Version 0.1, "Interdisziplinäres Zentrum für Bioinformatik, Universität Leipzig", Deutschland) gefiltert und erst dann für das *Peak Calling* verwendet (siehe Abschnitt 1.1.2.5.3). Die *Coverage,* auch Sequenziertiefe genannt, gibt an, wie häufig eine Position beim Sequenzieren abgelesen wurde. Z.B. sagt eine mittlere *Coverage* von 100fach aus, dass jede sequenzierte Base im Durchschnitt 100 Mal abgelesen wurde. Das *Peak Calling* ist der eigentliche Schritt, in dem der Methylierungsstatus des jeweiligen CpG berechnet wird. Dabei werden alle Reads angeschaut, die ein bestimmtes CpG enthalten, das Cytosin zu Uracil Verhältnis wird berechnet und das Ergebnis als eine Zahl zwischen 0 und 1 ausgegeben, wobei 0 einer Methylierung von 0% und 1 einer Methylierung von 100% entspricht. Die herkömmlichen *Libraries* haben eine durchschnittliche *Coverage* von 30 bis 40fach, worauf auch die herkömmlichen Methoden für das *Peak Calling* ausgelegt sind. Die zfDNA *Libraries* hatten aufgrund der geringeren Komplexität eine durchschnittliche *Coverage* von 8 bis 10fach. Dementsprechend musste auch das Filtern und *Peak Calling* z.B. mit dem "Bisulfite Analysis Toolkit" optimiert werden.

Sobald die DNA-Methylierungsraten feststehen, können weitere, spezifische Analysen je nach Fragenstellung in einer Programmiersprache der Wahl erfolgen. Für die hier beschriebenen Analysen wurden "R" (Version 3.2.0, "R Foundation for Statistical Computing", Österreich), "Perl" (Version 5.26.0, "The Perl Foundation", USA) und "Python" (Version 3.3.6, "Python Software Foundation", USA) [203-205] verwendet (siehe Abschnitt 1.1.2.5.3).

Da die hier beschriebenen Analysen eine sehr hohe Rechenkapazität benötigten, erfolgten sie auf einem "NEC-HPC-Linux-Cluster". Zugang zum Vorrechner erfolgte über eine SSH-Verbindung unter Verwendung der "MobaXterm Personal Edition" Software ("Mobatek", Frankreich).

### 1.1.2.5.1 Qualitätskontrolle der Rohdaten

Die Rohdaten wurden im "FastQ"-Format geliefert. Hierbei handelt es sich um ein text-basiertes Format, das zum Speichern der Reads sowie dazugehöriger Qualitätsparameter verwendet wird. Um die Qualität der Sequenzierung zu überprüfen, wurde die "FastQC" Software verwendet.

### 1.1.2.5.2 Datenprozessierung (Trimmen)

Die Rohdaten wurden unter Verwendung der "Cutadapt" Software (Version 1.9.1, "TU Dortmund", Deutschland) (Martin [2012] EMBnet.journal 17) prozessiert. Dabei wurden zwei Schritte durchgeführt.

### 1.) Eliminierung der überpräsentierten Sequenzen

Während der Sequenzierung wurden die ersten 76 Basen jedes DNA-Fragments von beiden Enden abgelesen (76 PE Sequenzierung). Die mit dem "Accel-NGS® Methyl-Seq DNA Library Kit" generierten *Libraries,* enthielten DNA-Fragmente unterschiedlicher Länge. Das bedeutet, wenn ein DNA-Fragment kürzer als 152 bp war, wurden auch die "Illumina Adapter" bzw. die *Flowcell* sequenziert. Das resultierte im Vorhandensein von "NNNNNNNNNNN"-Sequenzen. Da im weiteren Verlauf der Datenanalyse das *Alignment* der dazugehörigen und sonst qualitativ guten Reads aus diesem Grund verhindert werden würde, mussten die überpräsentierten Sequenzen entfernt werden. Der hierfür verwendete Befehl lautete:
***cutadapt** -q 20 -o 5 -minimum-length 30 -a GATCGGAAGAG -A AGATCGGAAGAG -o <Name_Read_1>.clipped.fastq.gz -p <Name_Read_2>.clipped.fastq.gz <Name_Read_1>.fastq.gz <Name_Read_2>.fastq.gz &><Name>.clipping.stats*

### 2.) Beseitigung des von der "Adaptase" erzeugten Überhangs

Während der Herstellung der WGBS *Library* wurde das Enzym "Adaptase" verwendet, welches am 3'-Ende des zweiten Reads einen Überhang mit niedriger Komplexität erzeugte. Dieser Bereich würde genauso wie die überpräsentierte Sequenzen beim späteren *Alignment* stören und musste somit entfernt werden. Der Befehl lautete:
***cutadapt** -minimum-length 25 -u 11* -*o <Name_Read_2>.clipped.trimmed.fastq.gz -p <Name_Read_1* >.*clipped.trimmed.fastq.gz <Name_Read_2>.clipped.fastq.gz <Name_Read_1>.clipped.fastq.gz*

### 1.1.2.5.3 Auswertung der prozessierten Daten

Die anschließende Datenanalyse erfolgte mit dem "Bisulfite Analysis Toolkit" [201]. Die Funktion der modular aufgebauten Software ist in Fig. 2 dargestellt.

Das *Alignment* wurde gegen das "HG19" Referenzgenom durchgeführt. Dabei wurden mehrere Algorithmen getestet, wobei überraschenderweise der"Segemehl"-Algorithmus die besten Ergebnisse lieferte (vgl. Abschnitt 1.1.2.5). Der Algorithmus beruht auf der Suche nach einem optimalen Treffer im Referenzgenom (Hoffmann et al. [2009] PLoS Comput. Biol. 5: e1000502). Die maximal erlaubte Anzahl an Ungenauigkeiten pro *Read* (z.B. Insertionen, Deletionen, Punktmutationen) betrug 10%. Alle Treffer, die diesen Schwellenwert unterschritten, wurden zu dem semi-globalen *Alignment* zugelassen. Letztendlich wurden nur die *Reads* mit einer Genauigkeit von mindestens 90% in einer finalen Datei aufgelistet und für die weiteren Analysen verwendet.

Das dabei bevorzugt eingesetzte "BAM"-Format ist eine komprimierte Version der "SAM"-Datei, ein text-basiertes Format, das zum Speichern von Ergebnissen des *Alignments* vom Algorithmus generiert wird. Die statistische Auswertung der *Mapping* Effizienz erfolgte z.B. mit dem "BAT_mapping_stat" Modul (Kretzmer et al. [2017] F1000Res. 6: 1490).

Schließlich wurden alle *Reads,* die zu einer Probe gehörten, in ein "BAM"-File mit dem "BAT_merging" Modul zusammengeführt. Die überlappenden Sequenzen wurden mit dem "ClipOverlap" (BamUtil Version 1.0.13) Modul eliminiert. Die Befehle lauteten:
*perl **BAT_mapping.pl** -g hg19.fa -i hg19 -p <Name_Read_1>.clipped.trimmed.fastq.gz -q <Name_Read_2>.clipped.trimmed.fastq.gz -t 16 -tmp <Folder> --segemehl segemehl.x -o <Folder>*/*<Name>*
*perl **BAT_mapping_stat.pl** --bam <Name>.bam --fastq <Name>.clipped.trimmed.fastq.gz -b > <Name>.stat*
*perl **BAT_merging.pl** -o <Name>.bam --bam <fiel_1>.bam,<file_2>.bam,* ..., *<file_n>.bam bamUtil_1.0.13*/*bamUtil*/*bin*/*bam **ClipOverlap** --in <Name>.bam --out <Name>.nooverlap.bam*

Im nächsten Schritt wurde die DNA-Methylierung mit Hilfe von "BAT_calling" detektiert. Das Modul erzeugt eine "VCF"-Datei. Hierbei handelt es sich um eine Textdatei, die nur Informationen über die detektierten DNA-Methylierungsraten, *Coverage,* Anzahl an abgedeckten Nukleotiden und den Sequenzkontext enthält. Im weiteren Verlauf der Analysen wurde diese Datei nach CpG Kontext und einer *Coverage* von mindestens achtfach gefiltert. Dabei wurden Abbildungen generiert und weitere "VCF"- sowie "BedGraph"-Dateien erzeugt.

Als nächstes wurde das "BAT_summarize" Modul verwendet, welches die Mittelwerte detektierter DNA-Methylierungsraten zweier Gruppen ermittelte. Die berechneten DNA-Methylierungsraten sowie die genomischen Koordinaten der Cytosine wurden in eine textbasierte "BedGraph"-Datei geschrieben, die im weiteren Verlauf für die Identifikation differentiell methylierter Regionen verwendet wurde.

Die Visualisierung der DNA-Methylierung pro Gruppe erfolgte unter Verwendung des "BAT_overview" Moduls [201]. Die Befehle lauteten:
***BAT_calling.pl** -d hg19.fa -q <Name>.nooverlap.bam --haarz segemehl_0_2_0*/*segemehl*/ *haarz.x* -o *<Folder>*
***BAT_filter_vcf.pl** --vcf <Name>.nooverlap.vcf.gz --out <Name>_CG_cov_final --context CG-MDP_min 8 --MDP_max 50*
***BAT_summarize.pl** --in 1 Adeno_CG_cov.bedgraph,PEKA_CG_cov.bedgraph --in2 Control_CG_cov.bedgraph -I cancer,control --h1 Adeno,PEKA --h2 Control --out pilot --cs hg19.chrom.sizes --bgbw bedGraphToBigWig*
***Rscript BAT_overview.R** -i pilot_cancer_control.txt -o pilot_overview.pdf-p cancer -q control*

### 1.1.2.5.4 Korrelationsanalysen

Im Rahmen dieser Arbeit wurden Daten von zwei Methoden zur genomweiten Untersuchung von DNA-Methylierungsmustern verwendet: WGBS und *Methylation Array* (HM 450K).

Für die Korrelationsanalysen wurde die "Bedtools" Software verwendet. Das "Bedtools Intersect" Modul liest sowohl die WGBS als auch HM 450K Ergebnisse ein, prüft sie auf Überlappung und schreibt die überlappenden CpG *Loci* in eine neue "BED"-Datei. Bei dem "BED"-Format handelt es sich um eine Textdatei. Jede Zeile der Datei enthält genomische Koordinaten eines CpG. Die Spalten sind durch ein Tabulatorzeichen getrennt. Die "BED"-Datei wurde nachfolgend direkt in "R" geladen und der "Pearson" Korrelationsanalyse unterzogen (p-Wert < 0,01). Die Visualisierung der Ergebnisse erfolgte ebenfalls in R.

### 1.1.2.5.5 Auswählen der CpG Loci für das Plasma Panel

Die WGBS Daten wurden wie beschrieben ausgewertet. Die mit dem "BAT_summarize" Modul generierte "BedGraph"-Datei enthielt drei Gruppen (Kontrolle, Adenokarzinom, Plattenepithelkarzinom) mit jeweils 11.289.424 Positionen pro Gruppe. Die "BedGraph"-Datei wurde in zwei Listen unterteilt. Die erste Liste enthielt 29.877 *Loci,* die DNA-Methylierungsunterschiede zwischen der Tumor- und Kontrollgruppen zeigten. Die zweite Liste enthielt 76.374 CpG *Loci,* die unterschiedlich jeweils in Adeno- und Plattenepithelkarzinomgruppen methyliert waren. Als differentiell methyliert wurden die Regionen bezeichnet, welche einen DNA-Methylierungsunterschied von mindestens 15% aufwiesen.

Als nächstes wurden die beiden Listen nach Chromosomen sortiert und mit dem "HG19" Referenzgenom annotiert. Die CpG *Loci,* die auf Chromosomen X, Y und M (mitochondriales Chromosom) sowie innerhalb von häufigen SNPs (≥1% der Bevölkerung) lokalisiert waren und nicht proteinkodierend waren, wurden verworfen.

Die verbliebenen CpG *Loci* mussten eines der drei Kriterien erfüllen, um in das Plasma *Panel* aufgenommen zu werden:
1.) differentiell methyliertes CpG wurde von beiden Methoden detektiert (WGBS und HM 450K),
2.) differentiell methyliertes CpG liegt innerhalb eines Clusters bestehend aus mindestens zwei weiteren differentiell methylierten CpG *Loci,* alle CpG *Loci* des Clusters sind entweder hypo- oder hypermethyliert, der Abstand zwischen den CpG *Loci* beträgt 2 bis 20 Nukleotide,
3.) es handelt sich um ein CpG mit der höchsten differentiellen DNA-Methylierungsrate (>0.8).

Die DNA-Regionen, die eines der drei Kriterien erfüllten, wurden in das Plasma *Panel* aufgenommen (siehe Tab. 1). Alle verwendeten Aufrufe sind unten detailliert beschrieben.

### 1.1.3 Weitere Komponenten des Plasma Panels (in silico Datenanalysen)

### 1.1.3.1 Die prognostische Studie

Zusätzlich zu den diagnostisch bzw. therapeutisch relevanten Informationen (z.B. Stadium und Tumorentität) sollte das *Panel* auch prognostische Informationen beinhalten. Deswegen wurde es um 33 CpG *Loci* erweitert, die im Rahmen einer klinischen Studie erhoben wurden. Der Titel der Studie lautete: "Comprehensive characterization of non-small cell lung cancer (NSCLC) by integrated clinical and molecular analysis".

Der zur Verfügung gestellte HM 450K-Datensatz enthielt Informationen über den DNA-Methylierungsstatus von insgesamt 41 Lungenkarzinomen. Die Patienten wurden je nach Überlebensdauer klassifiziert. Dabei wurden 28 Patienten zu der prognostisch günstigen (Überlebensdauer länger als 15 Monate) und 13 zu der ungünstigen Gruppe (Überlebensdauer kürzer als 13 Monate) gezählt. Die 33 in das *Panel* aufgenommene CpG *Loci* konnten beide Gruppen anhand des DNA-Methylierungsmusters voneinander trennen und enthielten somit für die Prognose relevanten Informationen.

### 1.1.3.2 Die bivalente Chromatin Studie

Zusätzlich zu den WGBS und HM 450K Ergebnissen wurden 26 differentiell methylierte Regionen aus der Studie zu bivalentem Chromatin in Tumoren in das Plasma *Panel* aufgenommen.

Die bivalenten Promotoren tragen sowohl aktivierende als auch reprimierende Histonmodifikationen, die vor allem während der Zelldifferenzierungsprozesse eine wichtige Rolle spielen. In Tumorzellen sind sie häufig fehlerhaft reguliert. Während der Studie wurden WGBS- und HM 450K-Datensätze verschiedener Tumorproben und -zelllinien (n=7000) analysiert.

### 1.2 Methoden: "Validierung des Plasma Panels/Untersuchung von Patientenproben"

Der erfindungsmäßige Satz an Methylierungsmarkern, das *Plasma Panel,* enthielt 630 differentiell methylierte Regionen (Tab. 1). Es wurde von der Firma "Roche" (Schweiz) synthetisiert und auf Trockeneis verschickt. Hierbei handelte es sich um ein nach Kundenwunsch synthetisiertes, nicht kommerziell erhältliches "SeqCap Epi Enrichment Kit" ("Roche", Schweiz). Laut Hersteller war das *Panel* sowohl für die Analyse von Gewebeproben als auch zirkulierender, zellfreier DNA geeignet.

Es wurde im Rahmen einer Pilotstudie validiert. Hierfür wurde vom DZL Blutplasma von 12 Patienten zur Verfügung gestellt. Davon waren drei Patienten zum Zeitpunkt der Untersuchung gesund bzw. tumorfrei (Kontrollgruppe) und neun litten an nicht-kleinzelligem Lungenkarzinomen unterschiedlicher Stadien (Tumorgruppe).

Die Validierung erfolgte in mehreren Schritten. Zunächst wurde das Validierungsmaterial, die zirkulierende, zellfreie DNA, vorbereitet. Die Extraktion aus dem Plasma, Quantifizierung, Qualitätskontrolle (QC) sowie Bisulfitkonvertierung erfolgten wie bereits in Abschnitten 1.1.2.1-1.1.2.3 beschrieben.

Je 10 ng der konvertierten zfDNA wurden dann für die Herstellung der Libraries verwendet. Die Library Präparation erfolgte in zwei Schritten. Im ersten Schritt wurde wie im Abschnitt 1.1.2.4 beschrieben von jeder Probe eine WGBS *Library* hergestellt, die Informationen über das gesamte zfDNA-Methylom des entsprechenden Patienten enthielt. Da jedoch im weiteren Verlauf nur die 638 differentiell methylierten Regionen sequenziert und analysiert werden sollten, wurden sie im zweiten Schritt aus dem gesamten Methylom extrahiert und angereichert. Das geschah unter Verwendung des "SeqCap Epi Enrichment Kit", dessen Bestandteil das von "Roche" synthetisierte Plasma *Panel* war (siehe Abschnitt 1.2.1).

Die fertige *Library* wurde einer QC unterzogen sowie quantifiziert (siehe Abschnitt 1.1.2.2) und anschließend auf dem "MiSeq" ("Illumina", USA) sequenziert (siehe Abschnitt 1.2.2). Die Sequenzierdaten wurden im "FastQ"-Format gespeichert und mussten anschließend analysiert werden (siehe Abschnitt 1.2.3). Hierfür wurde die bioinformatische Pipeline aus dem Abschnitt 1.1.2.5 angepasst, da diesmal nicht das gesamte Methylom, sondern nur die 638 bestimmten Regionen des Plasma *Panels* analysiert werden sollten.

Die Ergebnisse wurden schließlich für die Entwicklung eines Klassifikators verwendet, der nachfolgend die DNA-Methylierungsmuster interpretierte und diagnostisch sowie klinisch relevante Informationen über den gesundheitlichen Zustand eines Patienten lieferte (siehe Abschnitt 1.2.3.3).

Nach dem gleichen Prinzip können auch Proben eines Patienten analysiert werden, bei dem eine Diagnose von Lungentumoren erfolgen soll. Hier werden die Proben jedoch zur Analyse nicht gepoolt.

### 2.2.1 Anreicherung von differentiell methylierten Regionen

Für die Extraktion und Anreicherung von 630 differentiell methylierten Regionen aus dem gesamten zfDNA-Methylom wurde das "SeqCap Epi Enrichment Kit" verwendet. Einer der Bestandteile des Kits war das designte Plasma *Panel* (siehe Tab. 1). Die dort enthaltenen Oligonukleotide, auch "Capture Probes" genannt, hybridisierten an die differentiell methylierte Regionen und konnten im weiteren Verlauf angereichert und amplifiziert werden (Fig. 3).

### Hybridisierungsreaktion

Die 12 hergestellten WGBS *Libraries* wurden innerhalb der verschiedenen Gruppen äquimolar *gepoolt* und zunächst für eine Hybridisierungsreaktion vorbereitet. Bei diagnostischen Proben werden entweder Einzelproben hybridisiert oder Pools von Proben, die jeweils mit einem "Barcode" versehen sind, zum Einsatz kommen. Hierfür wurden 1 µg des WGBS *Library*-Pools mit 10 µL "Bisulfite Capture Enhancer", 1 µL "SeqCap HE Universal Oligo" und 1 µL "SeqCap HE Index Oligo" in ein 1,5 mL Reaktionsgefäß mit einem kleinen Loch im Deckel pipettiert. Die Probe wurde in einem Vakuumkonzentrator solange eingedampft, bis ein klares weißliches Pellet zu sehen war. Die "SeqCap HE Universal" und "SeqCap HE Index" Oligonukleotide wurden in einem Überschuss hinzugegeben (1 µL entsprach 1.000 pmol) und dienten dazu, die freiliegenden WGBS Universal- und Indexadapter zu binden. Somit sollte verhindert werden, dass die WGBS Adapter die nachfolgende Hybridisierungsreaktion stören.

Für die eigentliche Hybridisierungsreaktion wurden 7,5 µL zweifacher "Hybridisation Buffer" sowie 3 µL "Hybridisation Component A" direkt auf das Pellet gegeben, 10 s gemischt, kurz zentrifugiert und 10 min bei 95°C inkubiert. Dann wurde die Probe in ein 0,2 µL Reaktionsgefäß überführt, mit 4,5 µL "Capture Probes" versetzt, gut gemischt und bei 47°C 72 h lang in einem *Thermocycler* inkubiert. Der Deckel des *Thermocyclers* war auf 57°C vorgeheizt. Die "Capture Probes" wurden speziell für dieses Projekt synthetisiert. Sie enthielten 638 unterschiedliche Oligonukleotide, die komplementär zu den untersuchten differentiell methylierten Regionen (siehe Tab. 1) waren und im Laufe der Hybridisierungsreaktion diese gezielt banden.

### Anreicherung und Waschen der hybridisierten "Capture Probes"

Im nächsten Schritt wurden die gebundenen "Capture Probes" angereichert und mehrfach gewaschen. Hierfür wurden mehrere Waschpuffer sowie die "Capture Beads" nach Angaben des Herstellers vorbereitet.

Die hybridisierte Probe wurde mit 100 µL "Capture Beads" versetzt, kurz gemischt und 45 min bei 47°C im *Thermocycler* inkubiert. Der Deckel des *Thermocyclers* war auf 57°C vorgeheizt. Um das Absetzen der *Beads* zu verhindern, wurden die Proben alle 15 min kurz aus dem *Thermocycler* entnommen und gemischt. Bei den hier verwendeten "Capture Beads" handelte es sich um Streptavidin-*Beads*, die mit den biotinylierten "Capture Probes" interagierten.

Nach der Inkubation wurden die Proben aus dem *Thermocycler* entnommen und die "Capture Beads" mehreren Waschschritten unterzogen. Das Trennen der *Beads* vom Puffer erfolgte jedes Mal bei Raumtemperatur unter Verwendung des "DynaMag™-PCR" Magnets ("Thermo Fisher Scientific", USA).

Im ersten Teil des Waschprotokolls wurden nur Puffer verwendet, die zuvor auf 47°C vorgewärmt wurden. Dabei wurde die Probe mit 100 µL einfachem "Wash Buffer I" versetzt, kurz gemischt und mit Hilfe eines Magneten pelletiert. Der Überstand wurde verworfen, die *Beads* in 200 µL einfachem "Stringent Wash Buffer" gelöst, 5 min lang im Thermocycler bei 47°C inkubiert und wieder mit Hilfe eines Magneten pelletiert. Der Überstand wurde wieder verworfen und die *Beads* zwei weitere Male mit 200 µL einfachem "Stringent Wash Buffer" gewaschen.

Der zweite Teil des Waschprotokolls erfolgte komplett bei Raumtemperatur, dementsprechend mussten die hierfür verwendeten Puffer auf Raumtemperatur vorgewärmt werden. Zunächst wurden die zuvor bei 47°C gewaschene "Capture Beads" in 200 µl einfachen "Wash Buffer I" gelöst, 2 min lang gemischt und mit Hilfe eines Magneten pelletiert. Der Überstand wurde verworfen, die *Beads* mit 200 mL einfachem "Wash Buffer II" versetzt, 1 min lang gemischt und wieder mit Hilfe eines Magneten pelletiert. Auch hier wurde der Überstand verworfen, die *Beads* in 200 mL "Wasch Buffer III" gelöst, kurz gemischt und schließlich auf dem Magneten vom Überstand getrennt.

Für die darauffolgende Elution wurden 50 µL dH₂O direkt auf die *Beads* gegeben, 2 min lang bei Raumtemperatur inkubiert und mit Hilfe eines Magneten pelletiert. Der Überstand wurde aus dem Reaktionsgefäß vorsichtig abpipettiert und für alle weiteren Schritte verwendet.

### Amplifikation der angereicherten differentiell methylierten Regionen

Nach dem Waschen erfolgte die Amplifikation der angereicherten, differentiell methylierten Regionen. Hierfür wurden z.B. zu den 20 µL des Eluats 25 µL zweifacher "KAPA HiFi HotStart Ready Mix" ("Roche", Schweiz) sowie 5 µL "Post LM PCR Oligonukleotide" ("Roche", Schweiz) gegeben, gut gemischt und unter Verwendung von folgendem PCR-Programm im *Thermocycler* mit vorgeheiztem Deckel amplifiziert:
Schritt 1: 45 s 98°C
Schritt 2: 15 s 98°C
Schritt 3: 30 s 60°C
Schritt 4: 30 s 72°C
Schritt 5: Wiederholung der Schritte 1-4 für 15 weitere Male
Schritt 6: 60 s 72°C
Schritt 7: Pause bei 4°C

### Aufreinigung der angereicherten und amplifizierten differentiell methylierten Regionen

Die amplifizierten Regionen wurden nachfolgend aufgereinigt, z.B. unter Verwendung der "AmpureXP" *Beads* ("Beckman Coulter", USA). Hierfür wurden die *Beads* zunächst auf Raumtemperatur vorgewärmt. Die Probe wurde in ein 1,5 mL Reaktionsgefäß überführt. Zu 50 µL Probe wurden 50 µL dH₂O und 180 µL "AmpureXP" *Beads* gegeben. Die Probe wurde kurz gemischt, 15 min lang bei Raumtemperatur inkubiert, kurz zentrifugiert und auf den "DynaMag™-2" Magneten ("Thermo Fisher Scientific", USA) gestellt. Der Überstand wurde verworfen und die *Beads* zwei Mal mit jeweils 200 µL frisch zubereitetem 80%igem Ethanol gewaschen. Dann wurden die *Beads* 15 min lang bei Raumtemperatur getrocknet. Um die *Libraries* zu eluieren, wurden 52 µL dH₂O auf die trockenen *Beads* pipettiert. Die *Beads* wurden gut gemischt, 2 min lang bei Raumtemperatur inkubiert und wieder auf "DynaMag™-2" gestellt. Der Überstand wurde vorsichtig abpipettiert und für die Quantifizierung, QC (siehe Abschnitt 1.1.2.2) sowie Sequenzierung auf dem "MiSeq" verwendet.

### 1.2.2 Sequenzierung des Plasma Panels

Die Sequenzierung der NGS *Library* aus angereicherten, differentiell methylierten Regionen erfolgte auf dem "MiSeq".

Hierfür wurde die hergestellte *Library* zunächst auf 4 nM verdünnt und denaturiert. Dann wurden 5 µl der 4 nM Library in ein 1,5 mL Reaktionsgefäß überführt, mit 5 µL 0,2 N NaOH versetzt, kurz gemischt, 1 min lang bei 280g zentrifugiert und 5 min bei Raumtemperatur inkubiert. Die denaturierte *Library* wurde dann mit 990 µL "Buffer HT1" ("Illumina", USA) versetzt und wieder gut gemischt. Das ergab eine 20 pM *Library,* die anschließend mit "Buffer HT1" auf 4 pM verdünnt und mit 10% "PhiX" ("Illumina", USA) versetzt wurde.

Schließlich wurde eine "MiSeq 150 V3" Kassette ("Illumina", USA) mit der fertigen Probe beladen und in einem 76 PE Lauf sequenziert.

### 1.2.3 Bioinformatische Auswertung der Sequenzierdaten

### 1.2.3.1 Qualitätskontrolle und Prozessieren der Rohdaten

Die Daten wurden wie in Abschnitten 1.1.2.5.1 und 1.1.2.5.2 beschrieben einer "FastQC" Analyse unterzogen und anschließend prozessiert.

### 1.2.3.2 Auswertung der prozessierten Daten

Die prozessierten Daten wurden wie in Abschnitt 1.1.2.5.3 beschrieben gegen das "HG19" Referenzgenom mit dem "Segemehl" Algorithmus *aligned.* Die PCR-Duplikate wurden unter Verwendung von "Samtools" (Version 1.3.1, "Wellcome Trust Sanger Institute", England, "Broad Institute of MIT and Harvard", USA) entfernt. Der Befehl lautete:
***samtools rmdup** -S <Name>.bam <Name>_wo_dup.bam*

Die DNA-Methylierungsraten innerhalb der sequenzierten Regionen wurden mit dem "BAT_calling" Modul berechnet und mit dem "BAT_filter_vcf" Modul nach dem CpG Kontext und einer *Coverage* von mindestens achtfach gefiltert (siehe Abschnitt 1.1.2.5.3). Schließlich wurden die Daten gegen die Regionen des Plasma *Panels* annotiert. Die Aufrufe lauteten:

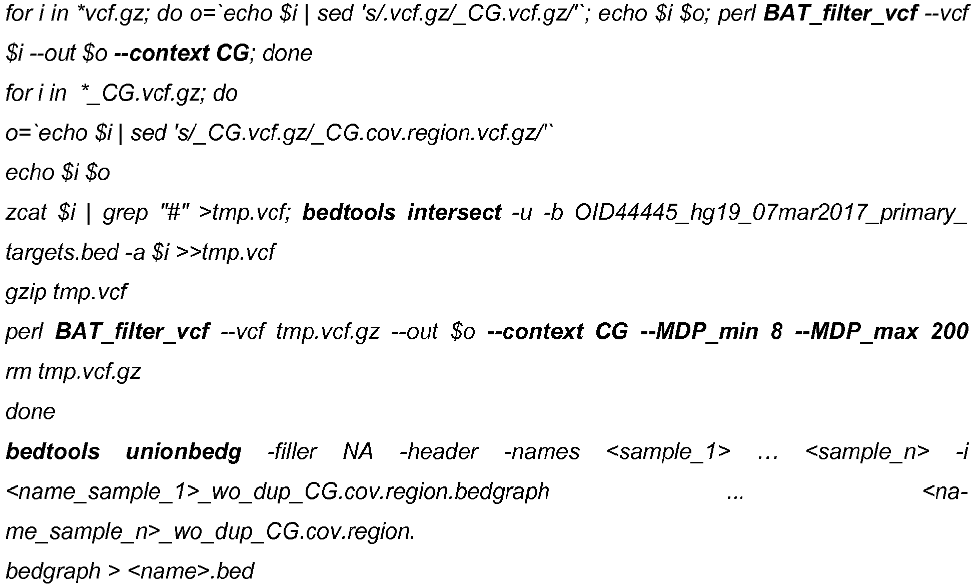

### 1.2.3.3 Erstellen eines Klassifikators

Mit Hilfe des *Plasma Panels* sollte das DNA-Methylierungsmuster eines Patienten analysiert werden. Daraus sollte geschlossen werden, ob ein Patient an einem malignen Lungentumor erkrankt ist. Falls ja, sollten aus dem DNA-Methylierungsprofil Informationen über die Entität des Tumors und die Prognose des betroffenen Patienten abgeleitet werden. Dies kann anhand der Korrelation zwischen den bei dem Patienten vorliegenden Methylierungsmuster und den erfindungsgemäß wichtigen Methylierungsmarkern erfolgen.

Hierfür kann ein Klassifikator erstellt werden, der in der Lage ist, die Ergebnisse der in den Abschnitten 1.2.3.1 und 1.2.3.2 beschriebenen Pipeline, schnell und zuverlässig zu interpretieren. Ein Klassifikator, auch *Predictive Modelling* genannt, ist ein Beispiel für das supervidierte Lernen. Das Ziel eines Klassifikators ist es, nach dem Erhalt von Variablen (z.B. DNA-Methylierungsmustern) und einer Annotation, zunächst ein Modell zu erstellen, das später in der Lage ist, die Variablen von unabhängigen Proben richtig zu klassifizieren (Fig. 4).

Die Software "Qlucore Omics Explorer" z.B. bietet mehrere Möglichkeiten an, unter Verwendung von DNA-Methylierungsdaten einen für die jeweilige Fragestellung optimalen Klassifikator zu erstellen. Dabei kann aus drei Algorithmen gewählt werden: "k-Nearest Neighbors Algorithm" (kNN), "Support Vector Machines" (SVM) und "Random Trees" (RT). Bei kNN wird eine Klassenzuordnung anhand der Berücksichtigung von k nächsten Nachbarn vorgenommen. SVM beschreibt jedes Objekt durch einen Vektor in einem Vektorraum. Innerhalb des Vektorraums wird eine Hyperebene so gesetzt, dass sie als Trennfläche zwischen den Gruppen agiert und sie in zwei Klassen unterteilt. RT besteht aus mehreren unkorrelierten Entscheidungsbäumen, die während des Lernprozesses generiert wurden. Jeder Baum fällt eine Entscheidung, die Klasse mit den meisten Stimmen entscheidet letztendlich über die endgültige Klassifikation.

Generell ist es schwierig für eine neue Fragestellung im Voraus die Aussage treffen zu können, welcher Algorithmus die optimalen Ergebnisse liefern wird. Daher wurden alle drei verfügbaren Algorithmen getestet, um den jeweils besten für die jeweilige Kategorie zu finden.

### 2. Ergebnisse

### 2.1 Ergebnisse: "Entwicklung des Plasma Panels"

### 2.1.1 Detektion der tumor- und entitätsspezifischen DNA-Methylierung in primärem Tumorgewebe

40 OP-Präparate und korrespondierende Kontrollen wurden unter Verwendung des "Illumina Infinium HumanMethylation450K BeadChips" auf ihre genomweite DNA-Methylierung untersucht.

Im Vergleich zum gesunden Lungengewebe wurden im malignen Tumorgewebe 898 aberrant methylierte CpG *Loci* identifiziert (q< 1×10⁻²³, σ/σₘₐₓ> 0,4; Abb. 5A). Adeno- und Plattenepithelkarzinome stellen die zwei häufigsten Entitäten des nicht-kleinzelligen Lungenkarzinoms dar. Eine Analyse ergab 1.167 zwischen den Tumorentitäten differentiell methylierte CpG *Loci* (FDR < 1 × 10⁻⁴; Abb. 5B).

Nachfolgend wurden die CpG *Loci* ausgewählt, die eine zuverlässige Klassifikation von Lungentumoren aufgrund von Malignität und Entität ermöglichten. Hierfür wurden die im Abschnitt 1.1.1 beschriebenen bioinformatische Analysen durchgeführt, welche 287 CpG *Loci* ergaben. Diese *Loci* wurden in einen erfindungsgemäß bevorzugten Satz an Methylierungsmarkern, das Plama *Panel,* aufgenommen (Tab. 1).

### 2.1.2 Detektion der tumor- und entitätsspezifischen DNA-Methylierung in Blutplasma

Wie in Abschnitt 1.1.2.2 beschrieben, wurde jede einzelne zellfreie, zirkulierende DNA Probe nach der Extraktion quantifiziert und einer strikten Qualitätskontrolle unterzogen. Die Gesamtmenge der extrahierten DNA betrug pro Probe 10 bis 30 ng, davon wurden 1 ng mit dem "Agilent 2100 Bioanalyzer" analysiert. Dabei zeigten die Proben einen klaren *Peak* bei ca. 167 bp. Die *Peaks* bei 35 und 10.380 bp entsprachen den unteren bzw. oberen Markern (nicht gezeigt).

Nach der Bisulfitkonvertierung wurden die zfDNA Proben zur Herstellung von WGBS *Libraries* verwendet. Die fertigen *Libraries* wurden wiederum quantifiziert und nachfolgend einer Qualitätskontrolle unter Verwendung des "Agilent 2100 Bioanalyzers" unterzogen. Alle Proben zeigten einen klaren *Peak bei* ca. 300 bp und entsprachen somit den Sequenzieranforderungen.

Die hergestellten WGBS *Libraries* wurden auf Trockeneis zu "TATAA Biocenter" geschickt, dort *gepoolt* und je nach Probe mit einer mittleren *Coverage* von acht- bis zehnfach auf einer "Next-Seq 500" Plattform sequenziert. Die Rohdaten wurden im "FastQ"-Format geliefert.

Die Qualität der Rohdaten wurde unter Verwendung der "FastQC" Software überprüft. Da die Proben 76 PE sequenziert wurden, lag die *Read* Länge wie erwartet bei 76 bp. Innerhalb eines Reads betrug der Gehalt an Adaptern sowie nicht identifizierbaren Signalen 0%. Die Genauigkeit der Sequenzierung wurde in "Phred"-Werten angegeben. Jeder "Phred"-Wert beschreibt, wie genau das Ablesen von Nukleotiden im Verlauf der Sequenzierung erfolgte. Die Rohdaten wiesen einen "Phred"-Score von über 30 auf, was einer Genauigkeit von mehr als 99,9% entsprach. Des Weiteren konnte nur eine sehr geringe Menge an Kmeren detektiert werden. Als Kmere werden Sequenzen mit einer Mindestlänge von zwei Nukleotiden bezeichnet, die sich in den Rohdaten immer wieder wiederholen. Die Anzahl an PCR-Duplikaten lag bei nahezu 0%. Die Menge an PCR-Duplikaten wird ermittelt, indem die Prozentanzahl deduplizierter Sequenzen berechnet und mit der Anzahl aller Sequenzen verglichen wird. Eine geringe Menge an Kmeren sowie PCR-Duplikaten weist auf eine gute *Library-* und Sequenzierqualität hin.

Des Weiteren wurde eine für WGBS typische Basenzusammensetzung analysiert. Im Laufe der Bisulfitkonvertierung wurden die meisten unmethylierten Cytosine durch Thymine ersetzt. Deshalb lag der Thymingehalt der Rohdaten bei ca. 50% und der Cytosingehalt bei nahezu 0%. Die Adenin- und Guaninzusammensetzung wurde während der Bisulfitkonvertierung nicht beeinflusst und lag bei jeweils 25%.

Nachfolgend wurden die WGBS Rohdaten unter Verwendung der "Cutadapt" Software prozessiert (siehe Abschnitt 1.1.2.5.2). Durch das Prozessieren wurden sowohl überpräsentierte Sequenzen, als auch der 10 nt lange Überhang am Anfang von *Read 2* entfernt.

Die prozessierten Sequenzierdaten wurden dann in das "Bisulfite Analysis Toolkit" geladen und mit dem dort implementierten "Segemehl" Algorithmus gegen das "HG19" Referenzgenom *aligned.* Die Effizienz des *Alignments* wird als *Mapping* Effizienz angegeben. Dabei wird ermittelt, wie viel Prozent an *Reads* dem Referenzgenom zugeordnet werden können. In diesem Fall betrug die *Mapping* Effizienz des "Segemehl" Algorithmus 98% bis 99% und war somit für alle weiteren Analysen geeignet.

Die *Alignments* der Kontroll-, Adenokarzinom- und Plattenepithelkarzinomgruppen wurden als nächstes in das "BAT_calling" Modul geladen. Das Modul ermittelte DNA-Methylierungsraten jeweiliger Cytosine. Die Cytosine, die innerhalb einer CpG-Region lagen und eine *Coverage* von mindestens achtfach aufwiesen, wurden dann unter Verwendung des "BAT_filtering" Moduls identifiziert und für alle weiteren Analysen verwendet.

Mehr als 4 Millionen CpG *Loci* pro Gruppe erfüllten die Kriterien und wurden im weiteren Verlauf mit dem "BAT_overview" Modul analysiert. Die Ergebnisse zeigten deutlich, dass sowohl Lungenkarzinom- als auch Kontrollgruppe anhand der DNA-Methylierungsmuster voneinander unterschieden werden können (Fig. 6A). Des Weiteren wird eine genomweite Hypermethylierung der Lungenkarzinomgruppen im Vergleich zur Kontrollgruppe sichtbar (Fig. 6A).

Um die für die jeweilige Gruppe spezifische, differentiell methylierte Regionen zu detektieren, erfolgte das Filtern nach einem DNA-Methylierungsunterschied von mindestens 15%. Die Anzahl an differentiell methylierten CpG *Loci* im Plasma von Lungenkarzinompatienten betrug dabei 18.000 (Fig. 7A). Des Weiteren wurden 44.000 CpG *Loci* identifiziert, die je nach Entität in Adeno- und Plattenepithelkarzinompatienten differentiell methyliert waren (Fig. 7B). Diese *Loci* wurden wie im Abschnitt 1.1.2.5.5 beschrieben weiteren Analysen unterzogen und für das Erstellen des Plasma *Panels* verwendet. Der fertige Satz an Methylierungsmarkern, d.h. das fertige Plasma *Panel,* enthielt 630 differenziell methylierte Regionen (Tab. 1). Mit diesen differentiell methylierten Regionen hybridisierende Oligonukleotide wurden als *"Capture Probes"* synthetisiert und stellen damit Mittel zur Diagnose von Lungentumoren dar.

### 2.1.3 Korrelationsanalysen der verwendeten Methoden zur genomweiten Detektion von DNA-Methylierungsmustern

Um die detektierten DNA-Methylierungsmuster in den OP-Präparaten mit denen im Blutplasma der Lungenkarzinompatienten zu vergleichen, wurde unter Verwendung von "R" und "Bedtools" eine "Pearson" Korrelationsanalyse durchgeführt (siehe Abschnitt 1.1.2.5.4), welche je nach Probe eine Übereinstimmung von 71 bis 77% ergab (p-Wert < 2,2 × 10⁻¹⁶, Fig. 8).

Dies zeigt, dass Ergebnisse auf Basis von OP-Präparaten oder festen Biopsien nicht ohne weiteres auf Flüssigbiopsien übertragen werden können, so dass die vorliegend erfolgte Validierung mit Flüssigbiopsien entscheidend für die Aussagekraft des Diagnoseverfahrens ist.

### 2.2 Ergebnisse zur "Validierung des Plasma Panels"

### 2.2.1 Erstellen der NGS Libraries

Zunächst wurden die extrahierten zfDNA-Proben wie im Abschnitt 1.1.2.2 beschrieben quantifiziert und einer Qualitätskontrolle unterzogen. Hierfür wurden jeweils 1 ng der Proben mit dem "Agilent 2100 Bioanalyzer" untersucht. Alle verwendeten zfDNA-Proben zeigten einen klaren *Peak* bei ca. 167 bp. Nachfolgend wurden die Proben bisulfitkonvertiert und zur Herstellung von NGS *Libraries* verwendet. Wie im Abschnitt 1.2.1 beschrieben, erfolgte die Herstellung der *Libraries* in zwei Schritten.

Im ersten Schritt wurden WGBS *Libraries* hergestellt, welche die Informationen über das gesamte zfDNA-Methylom umfassten. Alle 12 hergestellten WGBS *Libraries* zeigten einen klaren, großen *Peak bei* ca. 300 bp. Bei den größeren 300 bis 1.000 bp Peaks handelte es sich um die sogenannte *Daisy Chains,* d.h. aneinander hybridisierte ssDNA-Fragmente. Nach Angaben des Herstellers beeinflussen sie weder die darauffolgende Hybridisierungsreaktion noch die eigentliche Sequenzierung und müssen somit nicht eliminiert werden.

Im zweiten Schritt wurden die hergestellten WGBS *Libraries* quantifiziert, äquimolar *gepoolt* und mit dem "SeqCap Epi Enrichment Kit" prozessiert. Das hier verwendete Kit enthielt die sogenannten "Capture Probes", die speziell hierfür synthetisiert wurden. Die "Capture Probes" hybridisieren gezielt an die 638 Regionen des Plasma *Panels* (siehe Tab. 1). Nach der Hybridisierung wurden die "Capture Probes" samt den gebundenen differentiell methylierten Regionen angereichert, gewaschen und amplifiziert. Die amplifizierte *Library* wurde dann quantifiziert und einer Qualitätskontrolle unterzogen (z.B. "Agilent 2100 High Sensitivity DNA Kit"). Die fertige *Library* wies einen hohen Peak bei ca. 300 bp auf und entsprach somit den Sequenzieranforderungen des "MiSeq".

### 2.2.2 Sequenzierung und Datenanalyse

Zunächst wurde die Sequenzierung auf dem "MiSeq" optimiert. Die Sequenzierung erfolgte in einem 76 PE Modus. Es wurden also von beiden Enden die ersten 76 bp der sequenzierten DNA-Fragmente abgelesen. Um die optimale Clusterdichte zu erzielen, wurde die *Library* auf 4 pM verdünnt. Die hier beschriebene *Libraries* waren unbalanciert. Als unbalanciert werden *Libraries* bezeichnet, deren AT- bzw. GC-Konzentration weniger als 40% oder mehr als 60% beträgt. Solche Libraries weisen aufgrund ihrer Zusammensetzung meist eine nicht zufriedenstellende Sequenzierqualität auf. Um diese zu verbessern, kann die Library mit "PhiX Control V3" versetzt werden. Die Konzentration an "PhiX" muss je nach *Library* individuell angepasst werden. Die optimale Konzentration an "PhiX Control V3" betrug im vorliegenden Fall 10%. Nach der Sequenzierung wurden die Daten im "FastQ"-Format gespeichert. Die Qualität der Rohdaten wurde unter Verwendung der "FastQC" Software überprüft.

Aufgrund der 76 PE Sequenzierung, lag die *Read* Länge bei 76 bp. Der Gehalt an Adaptern sowie nicht identifizierbaren Signalen innerhalb eines Reads betrug 0%. Die Rohdaten wiesen einen "Phred"-Score von über 30, was einer Sequenziergenauigkeit von mehr als 99,9% entsprach. Die Basenzusammensetzung (Thymin-Gehalt bei ca. 50%, Cytosin-Gehalt bei nahezu 0%, Adenin- und Guanin-Gehalt bei 25%) wies auf eine erfolgreiche Bisulfitkonvertierung hin. Bei den ersten 10 nt des zweiten Reads handelte es sich um einen durch das Enzym "Adaptase" erzeugten Überhang. Das Abweichen des experimentell ermittelten vom theoretisch berechneten GC-Gehalt lag auch an der Bisulfitkonvertierung.

Die Anzahl an PCR-Duplikaten lag bei ca. 15%. Die Anzahl an deduplizierten Sequenzen wich stark von der Gesamtmenge ab. Das ist jedoch für ein *Panel* nicht ungewöhnlich. Im Gegensatz zu einer genomweiten Sequenzierung, wird bei einem *Panel* nur ein kleiner Bereich des Genoms sequenziert. Das führt zu einer sehr geringen Komplexität der *Library* und dementsprechend zur Entstehung von PCR-Duplikaten. Die Anzahl an Kmeren ist sehr gering und für die weitere Auswertung nicht störend.

Zusammenfassend lässt sich sagen, dass die *Panel* Sequenzierdaten eine sehr gute Qualität aufwiesen. Um die Daten zu prozessieren, wurden zwei Schritte durchgeführt. Zunächst wurde unter Verwendung der "Cutadapt" Software der 10 nt lange Überhang am Anfang von *Read 2* sowie Adapter entfernt. Dann wurden die PCR-Duplikate mit der "Samtools"-Software vollständig eliminiert.

Die prozessierten Sequenzierdaten wurden dann in das "Bisulfite Analysis Toolkit" geladen. Das *Alignment* erfolgte mit "Segemehl" gegen das "HG19" Referenzgenom. Die *Mapping* Effizienz betrug mindestens 90%. D.h., mindestens 90% der Rohdaten konnten dem Referenzgenom zugeordnet werden. Die mittlere *Coverage,* also die Sequenziertiefe, betrug je nach Probe 10 bis 30fach.

Im nächsten Schritt sollte die DNA-Methylierung detektiert werden. Hierfür wurden die 12 *Alignments* in das "BAT_calling" Modul geladen. Die ermittelten Positionen wurden dann zunächst unter Verwendung der "Bedtools" gegen das "HG19" Referenzgenom annotiert. Dann wurden die methylierten Positionen mit dem "BAT_filtering" Modul nach einer *Coverage* von mindestens achtfach gefiltert. Des Weiteren wurden mit dem Modul für das Erstellen eines Klassifikators nur solche Positionen ausgewählt, die sich zum einen in einer CpG Region befanden und zum anderen im Plasma *Panel* (Tab. 1) aufgelistet waren.

### 2.2.3 Erstellen eines Klassifikators

Die ermittelten zfDNA-Methylierungsraten wurden zum Erstellen eines Klassifikators verwendet. Wie in Abschnitt 1.2.3.3 beschrieben, wurde hierfür die "Qlucore Omics Explorer" Software verwendet, die folgende Klassifikationsalgorithmen enthielt: "k-Nearest Neighbors Algorithm" (kNN), "Support Vector Machines" (SVM) und "Random Trees" (RT).

Das *Plasma Panel* wurde so entworfen, dass es optimal in der Lage sein sollte, die Informationen bezüglich der Malignität, der Entität und des Stadiums eines Tumors und zu liefern. Diese Fragestellungen konnten durch die Wahl eines geeigneten Klassifikators zuverlässig beantwortet werden. Ferner sollten sich auch Informationen zur Prognose entnehmen lassen.

Um einen Klassifikator zu bewerten, wurden zwei Parameter betrachtet: die Richtigkeit (engl. *accuracy*) und die Komplexität. Die Richtigkeit eines Klassifikators wurde in Werten zwischen 0 und 1 angegeben, wobei 0 einer Richtigkeit von 0% und 1 einer Richtigkeit von 100% entsprach. Die Komplexität gab an, wie viele differentiell methylierte Positionen oder Marker analysiert werden mussten, damit der Klassifikator diese Richtigkeit erzielte. Je weniger Marker ausgewertet werden mussten, desto geeigneter war der Klassifikator für die Klinik. Denn mit der Anzahl an zu analysierenden Positionen steigen die Fehlerrate, Zeit und Kosten der Methode.

Die erste Fragestellung lautete, ob ein Patient generell an einem malignen Lungentumor litt. Hierfür lieferten sowohl der kNN als auch der RT Algorithmus eine Richtigkeit von 100%. Der RT Algorithmus benötigte für die Klassifikation 237 differentiell methylierte, im *Panel* enthaltene Positionen. Der kNN dagegen nur 10 Positionen, was ihn damit für diese Fragestellung als optimal qualifizierte (Fig. 9A). Bei 9 der 10 Positionen findet sich im Tumorgewebe eine stärkere Methylierung, bei einer eine schwächere.

Die Fragestellung bezüglich der Entität konnten alle drei Algorithmen mit einer Richtigkeit von 100% beantworten. Für die Berechnungen benötigte kNN 22 Positionen, SVM 22 Positionen und RT 10 Positionen. Somit war der RT Algorithmus für diese Fragestellung am besten geeignet (Fig. 9B), aber auch die anderen Algorithmen können verwendet werden. Bei allen ausgewerteten Markern findet sich beim Adenokarzinom eine stärkere Methylierung als beim Plattenepithekarzinom.

Für die letzte Fragestellung des Tumorstadiums war es am schwierigsten, einen geeigneten Klassifikator zu wählen. Der SVM Algorithmus schaffte es, unter Verwendung von 523 Positionen die späten Tumorstadien mit einer 80%igen Richtigkeit zu differenzieren (Fig. 9C). Dabei sind die ausgewerteten Positionen z.T. in den frühen, z.T. in den späten Stadien stärker methyliert.

Alle Positionen und Klassifikationsparameter sind im Anhang detailliert beschrieben (siehe Tab. 2-4). Die beschriebenen Ergebnisse machen es somit möglich, eine Diagnose von Lungenkrebs aus einer Flüssigbiopsie eines Patienten mit Hilfe von Sequenzierung von aufgereinigter, bisulfit-konvertierter und über an die Methylierungsmarker hybridisierende Oligonukleotide angereicherte DNA durchzuführen. Dabei wenden die Sequenzierungsdaten bevorzugt mit dem Segemehl-Algorithmus gegen ein Referenzgenom aligned und dann anhand der Korrelation der Methylierung, optional anhand der Klassifikation wie oben beschrieben, ausgewertet.

### 3.1 Weitere Informationen zu Entwicklung und Validierung des Plasma Panels

### Auswählen der CpG Loci für das Plasma Panel

### a. Filtern nach Chromosom

Die Chromosome M, X und Y wurden verworfen, die Befehle lauteten:
***grep -v** "chrM" <Name>.bedgraph* | ***grep -v** "chrX"* | ***grep -v** "chrY"* > *<Name>. ohneMXY.bedgraph*
***cut -f1** <Name>ohneMXY.bedgraph* | *sort* | *uniq*

### b. Annotation mit dem "HG19" Referenzgenom

***less gencode.v19.only.genes.bed*** | *perl -ane 'if($F[5] eq "+")($F[1]*=*$F[1]-1500}eise($F[2]* =*$F[2]*+*1500}; print "$F[0]\t$F[1]\t$F[2]\t$F[3]\t$F[4]\t$F[5]\n"'* > *gencode.v19.only.genes. TSS_1500nt.bed*
***bedtools intersect** -wa -wb -a <Name>ohneMXY.bedgraph -b gencode.v19.only.genes.TSS-1500nt.bed*

### c. Auswählen der CpG Loci, die von WGBS und HM 450K detektiert wurden

***bedtools intersect -wa -wb** -a* <*WGBS_data*>*.bedgraph-b* <*450K_BeadChip_data*>*.bed* | *perl -ane 'if(($F[3]>0* && *$F[7]>0)* || *($F[3]<0* && *$F[7]<0)){print $_}'* > *overlap_ WGBS_450K_BeadChip.bed*
***bedtools intersect -wa -wb** -a overlap_WGBS_450K_BeadChip.bed -b gen-code.v19.only.genes. TSS_1500nt.bed* | *cut -f1-4,8,9,13* > *overlap_WGBS_450K_BeadChip_gencode.v19.bed*

### d. Auswählen der differentiell methylierten CpG Cluster

Hierbei wurden CpG *Loci* ausgewählt, die innerhalb eines Clusters bestehend aus mindestens zwei weiteren differentiell methylierten CpG *Loci* lagen. Alle CpG *Loci* des Clusters waren entweder hypo- oder hypermethyliert. Der Abstand zwischen den CpG *Loci* betrug zwei bis 20 nt.

***less** <Name>ohneMXY.bedgraph* | *sort -k10,10* | ***bedtools groupby** -g 7,8,9,10,11,12 -c 1,2,3,1 -o collapse,collapse,collapse,count* | ***perl** -ane 'if($F[-1]>*=*3){print $*_}' | *perl -ane '*@*chr*=*split(*/*,*/*,$F[6]);* @*start*=*split(*/*,*/*,$F[7]);* @*end*=*split(*/*,*/*,$F[8]); for($i*=*0; $i*<*$F[-1]; $i*++*){print "$chr[$i]\t$start[$i]\t$end[$i]\ t$F[0]\t$F[1]\t$F[2]\t$F[3]\t$F[4]\t$F[5]\n"}'* > < *Name*>*ohneMXY _mind3CpG-annotation.bedgraph*
***perl** CpG_cluster_Swetlana --min 2 --max 20 --in <Name>ohneMXY_mind3CpG_annotation.bedgraph* | ***grep** protein* > *<Name>ohneMXY_mind3CpG_3diffCpG.bedgraph*
***less** <Name>ohneMXY_mind3CpG_3diffCpG.bedgraph* | ***bedtools groupby** -g 7,11* -*c 3,1,2,3 -o collapse,distinct,min,max* | ***perl** -ane 'print "$F[3]\t$F[4]\t$F[5]\t$F[2];$F[0]\n"'* > *<Name>ohneMXY_mind3CpG_3diffCpG_sortiert.bedgraph*
***bedtools intersect -wa -wb** -a <Name>ohneMXY_mind3CpG_3diffCpG _sortiert.bedgraph -b <Diff_fiel>.bedgraph* | ***bedtools groupby** -g 1,2,3,4 -c 8 -o mean* | ***perl** -ane '$a*=*abs($F[4]); chomp $*_; *print "$_\t$a\n"'* | ***sort** -k6,6n* | ***tail** -150* > *<Name>ohneMXY_mind3CpG_3diffCpG_sortiert_beste_150_regionen.bedgraph*

### e. Auswählen der Positionen mit der höchsten differentiellen DNA-Methylierung

***bedtools intersect** -v -a <Name>ohneMXY.bedgraph -b <Name*>*ohneMXY_mind3CpG_3diffCpG_sortiert_beste_150_regionen.bedgraph* | ***bedtools intersect -wa -wb** -a stdin -b gencode.v19.only.genes. TSS_1500nt_ohnechrM.bed* | ***grep protein*** | *perl -ane '$a*=*abs($F[5]); chomp $_; print "$_\t$a\n"'* | ***sort*** -*V -k13,13n* | *cut -f1,2,3,10,13* | *tail-100* > <*Name*>*ohneMXY_die_besten_einzel cpg.bedgraph*

**Tab. 1: Satz von Methylierungsmarkern (Plasma Panel, 630 differentiell methylierte Regionen).**

| In der Spalte "Tumor" ist angegeben, ob in Tumorgewebe eine verstärkte (hypermethyliert) oder verminderte (hypomethyliert) Methylierung identifiziert wurde. **A.** 350 Regionen, die einen malignen Tumor der Lunge detektieren. **B.** 247 Regionen, welche die häufigsten Lungenkarzinomentitäten (Adeno- und Plattenepithelkarzinom) voneinander unterscheiden. **C.** 33 prognostisch relevante CpG *Loci.* Methode: zfDNA (WBGS): zfDNA oder OP-Präparate (HM 450 K): OP, Die bivalente Chromatin Studie: bChrSt. | | | | |
|---|---|---|---|---|
| **A. Lungenkarzinom- oder Lungengewebe?** | | | | **Tumor** |
| Chromosom | Start | Ende | Methode | |
| chr1 | 57955028 | 57955174 | zfDNA, OP | hypomethyliert |
| chr1 | 193191311 | 193191476 | zfDNA, OP | hypermethyliert |
| chr10 | 85985699 | 85985859 | zfDNA, OP | hypomethyliert |
| chr10 | 110084584 | 110084739 | zfDNA, OP | hypomethyliert |
| chr10 | 130860130 | 130860266 | zfDNA, OP | hypomethyliert |
| chr11 | 57798784 | 57798925 | zfDNA, OP | hypomethyliert |
| chr11 | 57948628 | 57948769 | zfDNA, OP | hypomethyliert |
| chr11 | 58034333 | 58034464 | zfDNA, OP | hypomethyliert |
| chr11 | 59634150 | 59634282 | zfDNA, OP | hypomethyliert |
| chr11 | 59824464 | 59824610 | zfDNA, OP | hypomethyliert |
| chr11 | 131547241 | 131547390 | zfDNA, OP | hypomethyliert |
| chr12 | 7818556 | 7818707 | zfDNA, OP | hypomethyliert |
| chr12 | 111016497 | 111016626 | zfDNA, OP | hypomethyliert |
| chr12 | 128899218 | 128899363 | zfDNA, OP | hypomethyliert |
| chr14 | 58064847 | 58064987 | zfDNA, OP | hypomethyliert |
| chr14 | 88621354 | 88621491 | zfDNA, OP | hypomethyliert |
| chr15 | 63349114 | 63349271 | zfDNA, OP | hypermethyliert |
| chr15 | 87516105 | 87516269 | zfDNA, OP | hypomethyliert |
| chr16 | 20055126 | 20055299 | zfDNA, OP | hypomethyliert |
| chr16 | 34255462 | 34255596 | zfDNA, OP | hypomethyliert |
| chr17 | 46799562 | 46799708 | zfDNA, OP | hypermethyliert |
| chr2 | 2019860 | 2020000 | zfDNA, OP | hypomethyliert |
| chr2 | 66671403 | 66671543 | zfDNA, OP | hypermethyliert |
| chr2 | 118569132 | 118569281 | zfDNA, OP | hypomethyliert |
| chr2 | 155089787 | 155089940 | zfDNA, OP | hypomethyliert |
| chr20 | 29960903 | 29961067 | zfDNA, OP | hypomethyliert |
| chr21 | 31987899 | 31988061 | zfDNA, OP | hypomethyliert |
| chr3 | 159175958 | 159176133 | zfDNA, OP | hypomethyliert |
| chr4 | 77703312 | 77703460 | zfDNA, OP | hypomethyliert |
| chr5 | 5033914 | 5034062 | zfDNA, OP | hypomethyliert |
| chr5 | 5568513 | 5568662 | zfDNA, OP | hypomethyliert |
| chr5 | 141130550 | 141130698 | zfDNA, OP | hypomethyliert |
| chr6 | 5132810 | 5132954 | zfDNA, OP | hypermethyliert |
| chr6 | 20877268 | 20877408 | zfDNA, OP | hypomethyliert |
| chr6 | 27648240 | 27648385 | zfDNA, OP | hypermethyliert |
| chr6 | 55956239 | 55956395 | zfDNA, OP | hypomethyliert |
| chr7 | 149112327 | 149112464 | zfDNA, OP | hypermethyliert |
| chr8 | 54798658 | 54798811 | zfDNA, OP | hypomethyliert |
| chr1 | 2198804 | 2198961 | OP | hypermethyliert |
| chr1 | 6515521 | 6515702 | OP | hypermethyliert |
| chr1 | 6520115 | 6520257 | OP | hypermethyliert |
| chr1 | 19764609 | 19764757 | OP | hypermethyliert |
| chr1 | 34642324 | 34642455 | OP | hypermethyliert |
| chr1 | 47694840 | 47694995 | OP | hypermethyliert |
| chr1 | 50883315 | 50883461 | OP | hypermethyliert |
| chr1 | 50886707 | 50886857 | OP | hypermethyliert |
| chr1 | 50886870 | 50887021 | OP | hypermethyliert |
| chr1 | 79472375 | 79472516 | OP | hypermethyliert |
| chr1 | 110610821 | 110610964 | OP | hypermethyliert |
| chr1 | 110611386 | 110611542 | OP | hypermethyliert |
| chr1 | 110611971 | 110612108 | OP | hypermethyliert |
| chr1 | 119522559 | 119522707 | OP | hypermethyliert |
| chr1 | 150595130 | 150595282 | OP | hypermethyliert |
| chr1 | 153896523 | 153896648 | OP | hypomethyliert |
| chr1 | 155162673 | 155162808 | OP | hypomethyliert |
| chr1 | 158324396 | 158324540 | OP | hypomethyliert |
| chr1 | 158549201 | 158549351 | OP | hypomethyliert |
| chr1 | 158575697 | 158575854 | OP | hypomethyliert |
| chr1 | 158736216 | 158736378 | OP | hypomethyliert |
| chr1 | 159284004 | 159284160 | OP | hypomethyliert |
| chr1 | 159284209 | 159284363 | OP | hypomethyliert |
| chr1 | 159682419 | 159682564 | OP | hypomethyliert |
| chr1 | 160782978 | 160783141 | OP | hypomethyliert |
| chr1 | 161008634 | 161008907 | OP | hypomethyliert |
| chr1 | 166039366 | 166039510 | OP | hypomethyliert |
| chr1 | 175050401 | 175050549 | OP | hypomethyliert |
| chr1 | 182025968 | 182026117 | OP | hypermethyliert |
| chr1 | 223948836 | 223948969 | OP | hypermethyliert |
| chr1 | 248903024 | 248903175 | OP | hypomethyliert |
| chr10 | 15688934 | 15689073 | OP | hypomethyliert |
| chr10 | 34405682 | 34405834 | OP | hypermethyliert |
| chr10 | 44285786 | 44285947 | OP | hypomethyliert |
| chr10 | 98129672 | 98129823 | OP | hypomethyliert |
| chr10 | 98129826 | 98129981 | OP | hypomethyliert |
| chr10 | 102894966 | 102895098 | OP | hypermethyliert |
| chr10 | 104000754 | 104000901 | OP | hypermethyliert |
| chr10 | 118892505 | 118892640 | OP | hypermethyliert |
| chr10 | 118893055 | 118893205 | OP | hypermethyliert |
| chr10 | 121075240 | 121075380 | OP | hypomethyliert |
| chr10 | 134598276 | 134598414 | OP | hypermethyliert |
| chr11 | 627096 | 627254 | OP | hypermethyliert |
| chr11 | 31826508 | 31826642 | OP | hypermethyliert |
| chr11 | 40136733 | 40136880 | OP | hypomethyliert |
| chr11 | 40312591 | 40312717 | OP | hypomethyliert |
| chr11 | 57005866 | 57005971 | OP | hypomethyliert |
| chr11 | 57006196 | 57006350 | OP | hypomethyliert |
| chr11 | 59270333 | 59270463 | OP | hypomethyliert |
| chr11 | 68166958 | 68167099 | OP | hypermethyliert |
| chr11 | 69061832 | 69061978 | OP | hypomethyliert |
| chr11 | 75831643 | 75831777 | OP | hypermethyliert |
| chr11 | 86085859 | 86085993 | OP | hypermethyliert |
| chr11 | 123885618 | 123885776 | OP | hypomethyliert |
| chr11 | 133005846 | 133005990 | OP | hypomethyliert |
| chr12 | 5918113 | 5918249 | OP | hypomethyliert |
| chr12 | 21590167 | 21590318 | OP | hypomethyliert |
| chr12 | 50665695 | 50665835 | OP | hypermethyliert |
| chr12 | 54423481 | 54423625 | OP | hypermethyliert |
| chr12 | 54448654 | 54448816 | OP | hypermethyliert |
| chr12 | 54448836 | 54448981 | OP | hypermethyliert |
| chr12 | 56329564 | 56329709 | OP | hypermethyliert |
| chr12 | 62584958 | 62585102 | OP | hypermethyliert |
| chr12 | 75601386 | 75601538 | OP | hypermethyliert |
| chr12 | 114847503 | 114847664 | OP | hypermethyliert |
| chr12 | 126142819 | 126142966 | OP | hypomethyliert |
| chr12 | 129595318 | 129595466 | OP | hypomethyliert |
| chr13 | 41593317 | 41593485 | OP | hypomethyliert |
| chr13 | 42188553 | 42188701 | OP | hypermethyliert |
| chr13 | 58207783 | 58207923 | OP | hypermethyliert |
| chr14 | 21623728 | 21623873 | OP | hypomethyliert |
| chr14 | 37128511 | 37128658 | OP | hypermethyliert |
| chr14 | 55907221 | 55907370 | OP | hypermethyliert |
| chr14 | 57274684 | 57274828 | OP | hypermethyliert |
| chr14 | 57275089 | 57275229 | OP | hypermethyliert |
| chr14 | 57275889 | 57276137 | OP | hypermethyliert |
| chr14 | 57276179 | 57276336 | OP | hypermethyliert |
| chr14 | 57276449 | 57276590 | OP | hypermethyliert |
| chr14 | 57277149 | 57277295 | OP | hypermethyliert |
| chr14 | 57278109 | 57278251 | OP | hypermethyliert |
| chr14 | 57284449 | 57284596 | OP | hypermethyliert |
| chr14 | 60977778 | 60977928 | OP | hypermethyliert |
| chr14 | 60978086 | 60978221 | OP | hypermethyliert |
| chr14 | 77769608 | 77769754 | OP | hypomethyliert |
| chr15 | 42749674 | 42749956 | OP | hypermethyliert |
| chr15 | 45409243 | 45409393 | OP | hypermethyliert |
| chr15 | 72520560 | 72520691 | OP | hypomethyliert |
| chr15 | 86233150 | 86233290 | OP | hypermethyliert |
| chr15 | 89920745 | 89920964 | OP | hypermethyliert |
| chr15 | 89922266 | 89922403 | OP | hypermethyliert |
| chr16 | 23850031 | 23850175 | OP | hypomethyliert |
| chr16 | 29086204 | 29086356 | OP | hypermethyliert |
| chr16 | 31580915 | 31581053 | OP | hypermethyliert |
| chr16 | 48592619 | 48592755 | OP | hypermethyliert |
| chr16 | 59789141 | 59789301 | OP | hypomethyliert |
| chr16 | 59790110 | 59790246 | OP | hypomethyliert |
| chr16 | 66613021 | 66613174 | OP | hypermethyliert |
| chr16 | 66613201 | 66613354 | OP | hypermethyliert |
| chr16 | 76342543 | 76342697 | OP | hypomethyliert |
| chr17 | 750165 | 750314 | OP | hypermethyliert |
| chr17 | 31689711 | 31689863 | OP | hypomethyliert |
| chr17 | 32613223 | 32613361 | OP | hypomethyliert |
| chr17 | 35299524 | 35299661 | OP | hypermethyliert |
| chr17 | 55951984 | 55952129 | OP | hypermethyliert |
| chr17 | 59532229 | 59532369 | OP | hypermethyliert |
| chr17 | 67536233 | 67536383 | OP | hypomethyliert |
| chr17 | 72619477 | 72619639 | OP | hypomethyliert |
| chr18 | 20714264 | 20714392 | OP | hypomethyliert |
| chr18 | 21596836 | 21596981 | OP | hypomethyliert |
| chr18 | 61143869 | 61144219 | OP | hypomethyliert |
| chr18 | 61144261 | 61144399 | OP | hypomethyliert |
| chr19 | 9609321 | 9609462 | OP | hypermethyliert |
| chr19 | 18761488 | 18761632 | OP | hypermethyliert |
| chr19 | 19625186 | 19625348 | OP | hypermethyliert |
| chr19 | 42600201 | 42600339 | OP | hypermethyliert |
| chr19 | 48285227 | 48285396 | OP | hypermethyliert |
| chr19 | 53038895 | 53039056 | OP | hypermethyliert |
| chr2 | 2336353 | 2336494 | OP | hypomethyliert |
| chr2 | 3642551 | 3642688 | OP | hypermethyliert |
| chr2 | 43496147 | 43496286 | OP | hypermethyliert |
| chr2 | 45171739 | 45171891 | OP | hypermethyliert |
| chr2 | 45232352 | 45232491 | OP | hypermethyliert |
| chr2 | 63280990 | 63281212 | OP | hypermethyliert |
| chr2 | 63281305 | 63281462 | OP | hypermethyliert |
| chr2 | 63282625 | 63282788 | OP | hypermethyliert |
| chr2 | 63282935 | 63283081 | OP | hypermethyliert |
| chr2 | 63283888 | 63284202 | OP | hypermethyliert |
| chr2 | 73021274 | 73021424 | OP | hypermethyliert |
| chr2 | 100516804 | 100516939 | OP | hypomethyliert |
| chr2 | 105069122 | 105069275 | OP | hypomethyliert |
| chr2 | 105086941 | 105087083 | OP | hypomethyliert |
| chr2 | 124920570 | 124920719 | OP | hypomethyliert |
| chr2 | 127453687 | 127453859 | OP | hypomethyliert |
| chr2 | 162280362 | 162280605 | OP | hypermethyliert |
| chr2 | 176964058 | 176964200 | OP | hypermethyliert |
| chr2 | 176964383 | 176964599 | OP | hypermethyliert |
| chr2 | 176964651 | 176964790 | OP | hypermethyliert |
| chr2 | 176980760 | 176980908 | OP | hypermethyliert |
| chr2 | 176980985 | 176981133 | OP | hypermethyliert |
| chr2 | 176982263 | 176982420 | OP | hypermethyliert |
| chr2 | 176986185 | 176986321 | OP | hypermethyliert |
| chr2 | 176988868 | 176989016 | OP | hypermethyliert |
| chr2 | 176989280 | 176989410 | OP | hypermethyliert |
| chr2 | 177014478 | 177014625 | OP | hypermethyliert |
| chr2 | 177014869 | 177015014 | OP | hypermethyliert |
| chr2 | 177027372 | 177027510 | OP | hypermethyliert |
| chr2 | 177029509 | 177029683 | OP | hypermethyliert |
| chr2 | 192113945 | 192114079 | OP | hypermethyliert |
| chr2 | 200326645 | 200326782 | OP | hypermethyliert |
| chr2 | 208989171 | 208989315 | OP | hypermethyliert |
| chr2 | 223161815 | 223161963 | OP | hypermethyliert |
| chr2 | 223162956 | 223163101 | OP | hypermethyliert |
| chr2 | 223163250 | 223163396 | OP | hypermethyliert |
| chr20 | 5282874 | 5283037 | OP | hypomethyliert |
| chr20 | 29979773 | 29979904 | OP | hypomethyliert |
| chr20 | 60119429 | 60119586 | OP | hypomethyliert |
| chr21 | 38076799 | 38076947 | OP | hypermethyliert |
| chr21 | 38076967 | 38077102 | OP | hypermethyliert |
| chr21 | 38077182 | 38077314 | OP | hypermethyliert |
| chr21 | 38082537 | 38082677 | OP | hypermethyliert |
| chr3 | 128202420 | 128202557 | OP | hypermethyliert |
| chr3 | 147106484 | 147106639 | OP | hypermethyliert |
| chr3 | 147108444 | 147108594 | OP | hypermethyliert |
| chr3 | 147108764 | 147108915 | OP | hypermethyliert |
| chr3 | 147109715 | 147109852 | OP | hypermethyliert |
| chr3 | 147113649 | 147113806 | OP | hypermethyliert |
| chr3 | 147113839 | 147113992 | OP | hypermethyliert |
| chr3 | 147127584 | 147127733 | OP | hypermethyliert |
| chr3 | 147128049 | 147128198 | OP | hypermethyliert |
| chr3 | 147131253 | 147131426 | OP | hypermethyliert |
| chr3 | 160167891 | 160168052 | OP | hypermethyliert |
| chr3 | 178907589 | 178907716 | OP | hypermethyliert |
| chr3 | 181421475 | 181421609 | OP | hypermethyliert |
| chr3 | 181421626 | 181421778 | OP | hypermethyliert |
| chr4 | 16639102 | 16639249 | OP | hypomethyliert |
| chr4 | 16773604 | 16773747 | OP | hypomethyliert |
| chr4 | 16795659 | 16795823 | OP | hypomethyliert |
| chr4 | 16862004 | 16862148 | OP | hypomethyliert |
| chr4 | 38871251 | 38871401 | OP | hypermethyliert |
| chr4 | 40336180 | 40336325 | OP | hypomethyliert |
| chr4 | 81189629 | 81189764 | OP | hypermethyliert |
| chr4 | 84469489 | 84469634 | OP | hypomethyliert |
| chr4 | 111550587 | 111550727 | OP | hypermethyliert |
| chr4 | 111550752 | 111550898 | OP | hypermethyliert |
| chr4 | 151504646 | 151504792 | OP | hypermethyliert |
| chr5 | 1879607 | 1879772 | OP | hypermethyliert |
| chr5 | 5146264 | 5146403 | OP | hypomethyliert |
| chr5 | 9782073 | 9782214 | OP | hypomethyliert |
| chr5 | 33737859 | 33738007 | OP | hypomethyliert |
| chr5 | 140174811 | 140174969 | OP | hypermethyliert |
| chr5 | 140810843 | 140810977 | OP | hypermethyliert |
| chr5 | 140811566 | 140811712 | OP | hypermethyliert |
| chr6 | 28227021 | 28227193 | OP | hypermethyliert |
| chr6 | 30130783 | 30131058 | OP | hypomethyliert |
| chr6 | 33141218 | 33141345 | OP | hypomethyliert |
| chr6 | 34984865 | 34985013 | OP | hypermethyliert |
| chr6 | 36253031 | 36253158 | OP | hypermethyliert |
| chr6 | 50791127 | 50791269 | OP | hypermethyliert |
| chr6 | 50813472 | 50813737 | OP | hypermethyliert |
| chr6 | 100905379 | 100905516 | OP | hypermethyliert |
| chr6 | 100912869 | 100913017 | OP | hypermethyliert |
| chr6 | 101846889 | 101847032 | OP | hypermethyliert |
| chr6 | 138866798 | 138866965 | OP | hypermethyliert |
| chr7 | 811109 | 811265 | OP | hypermethyliert |
| chr7 | 1596186 | 1596331 | OP | hypomethyliert |
| chr7 | 2609786 | 2609933 | OP | hypermethyliert |
| chr7 | 3988693 | 3988828 | OP | hypermethyliert |
| chr7 | 4786820 | 4787032 | OP | hypermethyliert |
| chr7 | 7759144 | 7759281 | OP | hypomethyliert |
| chr7 | 27142023 | 27142169 | OP | hypermethyliert |
| chr7 | 27204708 | 27204859 | OP | hypermethyliert |
| chr7 | 27204903 | 27205058 | OP | hypermethyliert |
| chr7 | 54612258 | 54612404 | OP | hypermethyliert |
| chr7 | 65617286 | 65617424 | OP | hypermethyliert |
| chr7 | 96621248 | 96621396 | OP | hypermethyliert |
| chr7 | 96622543 | 96622774 | OP | hypermethyliert |
| chr7 | 154087897 | 154088047 | OP | hypomethyliert |
| chr7 | 154428954 | 154429110 | OP | hypomethyliert |
| chr8 | 12236159 | 12236321 | OP | hypomethyliert |
| chr8 | 24151806 | 24151954 | OP | hypomethyliert |
| chr8 | 70981967 | 70982102 | OP | hypermethyliert |
| chr8 | 128807993 | 128808124 | OP | hypomethyliert |
| chr8 | 133072190 | 133072337 | OP | hypom ethyl iert |
| chr9 | 37002618 | 37002762 | OP | hypermethyliert |
| chr1 | 6165201 | 6165361 | zfDNA | hypomethyliert |
| chr1 | 17567892 | 17568189 | zfDNA | hypomethyliert |
| chr1 | 15426262 | 15426418 | zfDNA | hypomethyliert |
| chr1 | 15670403 | 15670539 | zfDNA | hypermethyliert |
| chr10 | 96279972 | 96280055 | zfDNA | hypomethyliert |
| chr10 | 97033594 | 97033733 | zfDNA | hypermethyliert |
| chr11 | 134245966 | 134246129 | zfDNA | hypermethyliert |
| chr12 | 8004422 | 8004573 | zfDNA | hypermethyliert |
| chr12 | 97140774 | 97140905 | zfDNA | hypermethyliert |
| chr12 | 111566555 | 111566698 | zfDNA | hypermethyliert |
| chr12 | 117750775 | 117750937 | zfDNA | hypermethyliert |
| chr13 | 36828740 | 36828902 | zfDNA | hypermethyliert |
| chr14 | 93214072 | 93214242 | zfDNA | hypomethyliert |
| chr15 | 56006471 | 56006552 | zfDNA | hypermethyliert |
| chr15 | 101547384 | 101547527 | zfDNA | hypomethyliert |
| chr16 | 4141795 | 4141956 | zfDNA | hypermethyliert |
| chr18 | 21857621 | 21857750 | zfDNA | hypomethyliert |
| chr18 | 29528340 | 29528468 | zfDNA | hypermethyliert |
| chr18 | 46845901 | 46846043 | zfDNA | hypermethyliert |
| chr19 | 874766 | 874934 | zfDNA | hypomethyliert |
| chr19 | 6799968 | 6800095 | zfDNA | hypomethyliert |
| chr2 | 1126410 | 1126557 | zfDNA | differentiell |
| chr2 | 225642009 | 225642217 | zfDNA | differentiell |
| chr2 | 236745514 | 236745688 | zfDNA | hypomethyliert |
| chr2 | 240881986 | 240882138 | zfDNA | differentiell |
| chr2 | 2179742 | 2179886 | zfDNA | hypermethyliert |
| chr2 | 30747398 | 30747539 | zfDNA | hypermethyliert |
| chr2 | 175998270 | 175998415 | zfDNA | hypermethyliert |
| chr2 | 219647407 | 219647560 | zfDNA | hypomethyliert |
| chr20 | 20243607 | 20243747 | zfDNA | hypermethyliert |
| chr20 | 55079800 | 55079945 | zfDNA | hypermethyliert |
| chr21 | 30502729 | 30502871 | zfDNA | hypermethyliert |
| chr21 | 46587906 | 46588052 | zfDNA | hypomethyliert |
| chr3 | 56445240 | 56445378 | zfDNA | hypermethyliert |
| chr3 | 85143433 | 85143600 | zfDNA | hypermethyliert |
| chr3 | 146123966 | 146124095 | zfDNA | hypomethyliert |
| chr3 | 68947379 | 68947542 | zfDNA | hypermethyliert |
| chr3 | 197767819 | 197767978 | zfDNA | hypermethyliert |
| chr4 | 143487129 | 143487273 | zfDNA | hypermethyliert |
| chr4 | 26398190 | 26398329 | zfDNA | hypermethyliert |
| chr4 | 77647893 | 77648027 | zfDNA | hypermethyliert |
| chr4 | 102497551 | 102497732 | zfDNA | hypomethyliert |
| chr5 | 39187156 | 39187287 | zfDNA | hypermethyliert |
| chr5 | 56145736 | 56145896 | zfDNA | hypermethyliert |
| chr5 | 160171748 | 160171896 | zfDNA | hypermethyliert |
| chr5 | 16793080 | 16793219 | zfDNA | hypermethyliert |
| chr5 | 76869108 | 76869253 | zfDNA | hypermethyliert |
| chr6 | 169050287 | 169050447 | zfDNA | hypermethyliert |
| chr6 | 76773251 | 76773422 | zfDNA | hypomethyliert |
| chr6 | 123869831 | 123869971 | zfDNA | hypomethyliert |
| chr7 | 6268960 | 6269087 | zfDNA | hypermethyliert |
| chr7 | 38508407 | 38508486 | zfDNA | hypermethyliert |
| chr7 | 153743779 | 153743947 | zfDNA | hypomethyliert |
| chr7 | 137230794 | 137230963 | zfDNA | hypomethyliert |
| chr7 | 151300131 | 151300282 | zfDNA | hypermethyliert |
| chr8 | 3672236 | 3672387 | zfDNA | hypermethyliert |
| chr8 | 99510084 | 99510252 | zfDNA | hypermethyliert |
| chr8 | 101170822 | 101170975 | zfDNA | hypomethyliert |
| chr8 | 141127042 | 141127183 | zfDNA | hypomethyliert |
| chr9 | 2050654 | 2050804 | zfDNA | hypermethyliert |
| chr9 | 9227683 | 9227824 | zfDNA | hypermethyliert |
| chr9 | 79060522 | 79060633 | zfDNA | hypermethyliert |
| chr9 | 124334690 | 124334848 | zfDNA | hypomethyliert |
| chr9 | 126166694 | 126166828 | zfDNA | hypermethyliert |
| chr1 | 180202441 | 180202578 | bChrSt | hypermethyliert |
| chr10 | 102984159 | 102984316 | bChrSt | hypermethyliert |
| chr10 | 102986926 | 102987078 | bChrSt | hypomethyliert |
| chr10 | 124905661 | 124905811 | bChrSt | hypermethyliert |
| chr11 | 18416284 | 18416422 | bChrSt | hypomethyliert |
| chr11 | 20178032 | 20178171 | bChrSt | hypermethyliert |
| chr11 | 20181732 | 20181875 | bChrSt | hypermethyliert |
| chr11 | 31821190 | 31821332 | bChrSt | hypermethyliert |
| chr11 | 31831813 | 31831955 | bChrSt | hypermethyliert |
| chr12 | 6644024 | 6644165 | bChrSt | hypomethyliert |
| chr13 | 100621076 | 100621217 | bChrSt | hypomethyliert |
| chr13 | 100624236 | 100624376 | bChrSt | hypomethyliert |
| chr14 | 23790611 | 23790772 | bChrSt | hypermethyliert |
| chr17 | 46674335 | 46674487 | bChrSt | hypermethyliert |
| chr17 | 48048913 | 48049070 | bChrSt | hypermethyliert |
| chr2 | 162283732 | 162283879 | bChrSt | hypermethyliert |
| chr2 | 175199619 | 175199764 | bChrSt | hypermethyliert |
| chr2 | 175200596 | 175200742 | bChrSt | hypermethyliert |
| chr2 | 223163736 | 223163879 | bChrSt | hypermethyliert |
| chr4 | 13525615 | 13525755 | bChrSt | hypomethyliert |
| chr4 | 113432474 | 113432622 | bChrSt | hypermethyliert |
| chr6 | 100051116 | 100051256 | bChrSt | hypomethyliert |
| chr6 | 100054673 | 100054827 | bChrSt | hypomethyliert |
| chr6 | 100060971 | 100061117 | bChrSt | hypomethyliert |

| **Tab: 1 B. Entität: Adeno- oder Plattenepithelkarzinom?** | | | | **Entität** |
|---|---|---|---|---|
| Chromosom | Start | Ende | Methode | meth. Entitäten |
| chr1 | 52158087 | 52158220 | zfDNA, OP | SQC<ADC |
| chr1 | 61668739 | 61668922 | zfDNA, OP | SQC<ADC |
| chr1 | 64578151 | 64578293 | zfDNA, OP | SQC<ADC |
| chr1 | 77533495 | 77533671 | zfDNA, OP | SQC<ADC |
| chr1 | 171868017 | 171868187 | zfDNA, OP | SQC<ADC |
| chr1 | 214646125 | 214646279 | zfDNA, OP | SQC<ADC |
| chr11 | 1328403 | 1328548 | zfDNA, OP | SQC<ADC |
| chr11 | 4079459 | 4079623 | zfDNA, OP | SQC<ADC |
| chr11 | 71188639 | 71188789 | zfDNA, OP | SQC<ADC |
| chr11 | 104972062 | 104972193 | zfDNA, OP | SQC<ADC |
| chr11 | 105010212 | 105010354 | zfDNA, OP | SQC<ADC |
| chr12 | 52946925 | 52947067 | zfDNA, OP | SQC>ADC |
| chr12 | 88538122 | 88538272 | zfDNA, OP | SQC<ADC |
| chr12 | 109096126 | 109096269 | zfDNA, OP | SQC<ADC |
| chr14 | 90083196 | 90083338 | zfDNA, OP | SQC<ADC |
| chr16 | 58155114 | 58155256 | zfDNA, OP | SQC<ADC |
| chr17 | 29667240 | 29667387 | zfDNA, OP | SQC<ADC |
| chr2 | 9987364 | 9987518 | zfDNA, OP | SQC<ADC |
| chr2 | 25501964 | 25502121 | zfDNA, OP | SQC<ADC |
| chr2 | 172266609 | 172266746 | zfDNA, OP | SQC<ADC |
| chr2 | 178178843 | 178179018 | zfDNA, OP | SQC<ADC |
| chr2 | 179897218 | 179897356 | zfDNA, OP | SQC<ADC |
| chr20 | 31446106 | 31446254 | zfDNA, OP | SQC<ADC |
| chr3 | 4348279 | 4348416 | zfDNA, OP | SQC<ADC |
| chr3 | 38567580 | 38567725 | zfDNA, OP | SQC<ADC |
| chr3 | 111629808 | 111629952 | zfDNA, OP | SQC<ADC |
| chr3 | 114074222 | 114074369 | zfDNA, OP | SQC<ADC |
| chr3 | 122841556 | 122841705 | zfDNA, OP | SQC<ADC |
| chr3 | 150948199 | 150948350 | zfDNA, OP | SQC<ADC |
| chr3 | 164915101 | 164915268 | zfDNA, OP | SQC<ADC |
| chr5 | 122506718 | 122506853 | zfDNA, OP | SQC<ADC |
| chr6 | 63990944 | 63991095 | zfDNA, OP | SQC<ADC |
| chr6 | 64572767 | 64572911 | zfDNA, OP | SQC<ADC |
| chr7 | 20381014 | 20381160 | zfDNA, OP | SQC<ADC |
| chr7 | 21813010 | 21813162 | zfDNA, OP | SQC<ADC |
| chr7 | 98722395 | 98722537 | zfDNA, OP | SQC>ADC |
| chr7 | 102574027 | 102574188 | zfDNA, OP | SQC<ADC |
| chr7 | 102574397 | 102574549 | zfDNA, OP | SQC<ADC |
| chr7 | 111825737 | 111825894 | zfDNA, OP | SQC<ADC |
| chr7 | 116377388 | 116377530 | zfDNA, OP | SQC>ADC |
| chr7 | 122056569 | 122056711 | zfDNA, OP | SQC<ADC |
| chr8 | 38643499 | 38643633 | zfDNA, OP | SQC<ADC |
| chr8 | 42772303 | 42772478 | zfDNA, OP | SQC>ADC |
| chr8 | 145599209 | 145599355 | zfDNA, OP | SQC<ADC |
| chr1 | 3607047 | 3607181 | OP | SQC<ADC |
| chr1 | 220101648 | 220101795 | OP | SQC<ADC |
| chr1 | 220101867 | 220102015 | OP | SQC<ADC |
| chr1 | 236849398 | 236849548 | OP | SQC<ADC |
| chr1 | 236849891 | 236850048 | OP | SQC<ADC |
| chr10 | 11206799 | 11206938 | OP | SQC<ADC |
| chr11 | 30606998 | 30607133 | OP | SQC<ADC |
| chr11 | 64992997 | 64993132 | OP | SQC>ADC |
| chr11 | 64993266 | 64993396 | OP | SQC>ADC |
| chr11 | 65360248 | 65360394 | OP | SQC<ADC |
| chr11 | 77160268 | 77160416 | OP | SQC<ADC |
| chr11 | 82444721 | 82444866 | OP | SQC<ADC |
| chr12 | 4381723 | 4381963 | OP | SQC<ADC |
| chr12 | 33592568 | 33592710 | OP | SQC<ADC |
| chr13 | 28674372 | 28674520 | OP | SQC<ADC |
| chr15 | 69087740 | 69087878 | OP | SQC<ADC |
| chr15 | 83316148 | 83316297 | OP | SQC<ADC |
| chr16 | 1202369 | 1202544 | OP | SQC<ADC |
| chr16 | 56224714 | 56224858 | OP | SQC<ADC |
| chr16 | 81564475 | 81564626 | OP | SQC<ADC |
| chr16 | 86600252 | 86600386 | OP | SQC<ADC |
| chr17 | 693067 | 693222 | OP | SQC<ADC |
| chr17 | 693313 | 693458 | OP | SQC<ADC |
| chr17 | 66292297 | 66292442 | OP | SQC<ADC |
| chr17 | 74696666 | 74696814 | OP | SQC<ADC |
| chr17 | 75196873 | 75197007 | OP | SQC<ADC |
| chr17 | 80794200 | 80794346 | OP | SQC<ADC |
| chr18 | 2847458 | 2847590 | OP | SQC<ADC |
| chr18 | 24131050 | 24131188 | OP | SQC<ADC |
| chr18 | 24131310 | 24131449 | OP | SQC<ADC |
| chr19 | 10572284 | 10572428 | OP | SQC<ADC |
| chr2 | 30834597 | 30834737 | OP | SQC>ADC |
| chr2 | 50574632 | 50574774 | OP | SQC<ADC |
| chr2 | 54054275 | 54054427 | OP | SQC<ADC |
| chr2 | 63276135 | 63276276 | OP | SQC<ADC |
| chr2 | 236444206 | 236444348 | OP | SQC<ADC |
| chr20 | 20349092 | 20349238 | OP | SQC<ADC |
| chr20 | 47444494 | 47444648 | OP | SQC<ADC |
| chr20 | 47444775 | 47445083 | OP | SQC<ADC |
| chr21 | 26934497 | 26934635 | OP | SQC<ADC |
| chr3 | 141102520 | 141102668 | OP | SQC<ADC |
| chr3 | 172167531 | 172167678 | OP | SQC<ADC |
| chr3 | 172394613 | 172394766 | OP | SQC<ADC |
| chr3 | 186914643 | 186914790 | OP | SQC<ADC |
| chr3 | 196435366 | 196435518 | OP | SQC<ADC |
| chr4 | 57522417 | 57522559 | OP | SQC<ADC |
| chr4 | 57522562 | 57522846 | OP | SQC<ADC |
| chr5 | 912634 | 912893 | OP | SQC<ADC |
| chr5 | 1883876 | 1884018 | OP | SQC<ADC |
| chr5 | 16179056 | 16179202 | OP | SQC<ADC |
| chr5 | 33936177 | 33936331 | OP | SQC<ADC |
| chr5 | 36607322 | 36607477 | OP | SQC<ADC |
| chr5 | 169064355 | 169064518 | OP | SQC<ADC |
| chr7 | 653234 | 653373 | OP | SQC<ADC |
| chr7 | 1491753 | 1492006 | OP | SQC<ADC |
| chr7 | 2158351 | 2158498 | OP | SQC<ADC |
| chr7 | 4228700 | 4228842 | OP | SQC<ADC |
| chr7 | 19156542 | 19156690 | OP | SQC<ADC |
| chr7 | 19157127 | 19157340 | OP | SQC<ADC |
| chr7 | 45197376 | 45197524 | OP | SQC<ADC |
| chr8 | 41754102 | 41754249 | OP | SQC<ADC |
| chr8 | 123874964 | 123875109 | OP | SQC<ADC |
| chr8 | 123875144 | 123875280 | OP | SQC<ADC |
| chr1 | 3289010 | 3289139 | zfDNA | SQC<ADC |
| chr1 | 17567892 | 17568189 | zfDNA | SQC>ADC |
| chr1 | 23284417 | 23284507 | zfDNA | SQC>ADC |
| chr1 | 24277975 | 24278154 | zfDNA | SQC>ADC |
| chr1 | 47738990 | 47739142 | zfDNA | SQC<ADC |
| chr1 | 79467955 | 79468081 | zfDNA | SQC>ADC |
| chr1 | 108975333 | 108975476 | zfDNA | SQC<ADC |
| chr1 | 196682870 | 196683025 | zfDNA | SQC<ADC |
| chr1 | 217310510 | 217310654 | zfDNA | SQC>ADC |
| chr1 | 240656480 | 240656649 | zfDNA | SQC<ADC |
| chr1 | 240746545 | 240746706 | zfDNA | SQC<ADC |
| chr1 | 246241918 | 246242056 | zfDNA | SQC<ADC |
| chr10 | 12533631 | 12533768 | zfDNA | SQC>ADC |
| chr10 | 32647546 | 32647656 | zfDNA | SQC>ADC |
| chr10 | 32657588 | 32657719 | zfDNA | SQC>ADC |
| chr10 | 37511104 | 37511239 | zfDNA | SQC>ADC |
| chr10 | 62708104 | 62708269 | zfDNA | SQC>ADC |
| chr10 | 73207931 | 73208064 | zfDNA | SQC<ADC |
| chr10 | 108812804 | 108812940 | zfDNA | SQC<ADC |
| chr10 | 115658133 | 115658275 | zfDNA | SQC>ADC |
| chr10 | 123914649 | 123914808 | zfDNA | SQC>ADC |
| chr11 | 15025357 | 15025499 | zfDNA | SQC>ADC |
| chr11 | 19778770 | 19778909 | zfDNA | SQC<ADC |
| chr11 | 26355535 | 26355711 | zfDNA | SQC>ADC |
| chr11 | 26600784 | 26600925 | zfDNA | SQC>ADC |
| chr11 | 26626367 | 26626558 | zfDNA | SQC>ADC |
| chr11 | 41275397 | 41275536 | zfDNA | SQC>ADC |
| chr11 | 62158845 | 62158985 | zfDNA | SQC>ADC |
| chr11 | 70503001 | 70503139 | zfDNA | SQC>ADC |
| chr11 | 106592142 | 106592304 | zfDNA | SQC<ADC |
| chr11 | 120644150 | 120644282 | zfDNA | SQC<ADC |
| chr11 | 122678508 | 122678636 | zfDNA | SQC<ADC |
| chr11 | 128851150 | 128851286 | zfDNA | SQC>ADC |
| chr12 | 125571801 | 125571933 | zfDNA | SQC>ADC |
| chr13 | 48806444 | 48806588 | zfDNA | SQC>ADC |
| chr13 | 113527733 | 113527876 | zfDNA | SQC<ADC |
| chr14 | 35030336 | 35030470 | zfDNA | SQC>ADC |
| chr14 | 104486171 | 104486314 | zfDNA | SQC>ADC |
| chr15 | 22839905 | 22840043 | zfDNA | SQC<ADC |
| chr15 | 26964926 | 26965065 | zfDNA | SQC>ADC |
| chr15 | 29246303 | 29246447 | zfDNA | SQC>ADC |
| chr15 | 30180680 | 30180842 | zfDNA | SQC<ADC |
| chr15 | 32404970 | 32405130 | zfDNA | SQC<ADC |
| chr15 | 64244033 | 64244215 | zfDNA | SQC<ADC |
| chr15 | 68530927 | 68531091 | zfDNA | SQC>ADC |
| chr15 | 83579367 | 83579513 | zfDNA | SQC<ADC |
| chr15 | 88559865 | 88560003 | zfDNA | SQC>ADC |
| chr16 | 6257325 | 6257474 | zfDNA | SQC>ADC |
| chr16 | 15665564 | 15665721 | zfDNA | SQC>ADC |
| chr16 | 24321180 | 24321320 | zfDNA | SQC<ADC |
| chr16 | 75528556 | 75528698 | zfDNA | SQC>ADC |
| chr16 | 88013993 | 88014135 | zfDNA | SQC<ADC |
| chr16 | 89713952 | 89714124 | zfDNA | SQC>ADC |
| chr17 | 416719 | 416865 | zfDNA | SQC<ADC |
| chr17 | 19809670 | 19809830 | zfDNA | SQC<ADC |
| chr17 | 21086965 | 21087112 | zfDNA | SQC>ADC |
| chr17 | 33364961 | 33365040 | zfDNA | SQC<ADC |
| chr17 | 64330485 | 64330837 | zfDNA | SQC>ADC |
| chr17 | 75142732 | 75142885 | zfDNA | SQC<ADC |
| chr19 | 11890923 | 11891074 | zfDNA | SQC<ADC |
| chr19 | 49016450 | 49016584 | zfDNA | SQC>ADC |
| chr19 | 57922060 | 57922195 | zfDNA | SQC>ADC |
| chr2 | 1129413 | 1129596 | zfDNA | SQC>ADC |
| chr2 | 1334513 | 1334640 | zfDNA | SQC>ADC |
| chr2 | 23917010 | 23917136 | zfDNA | SQC>ADC |
| chr2 | 25124037 | 25124165 | zfDNA | SQC>ADC |
| chr2 | 46779214 | 46779381 | zfDNA | SQC>ADC |
| chr2 | 113534514 | 113534653 | zfDNA | SQC<ADC |
| chr2 | 120417931 | 120418073 | zfDNA | SQC>ADC |
| chr2 | 131798797 | 131798977 | zfDNA | SQC<ADC |
| chr2 | 198073787 | 198073950 | zfDNA | SQC>ADC |
| chr2 | 205889570 | 205889704 | zfDNA | SQC>ADC |
| chr2 | 207319476 | 207319691 | zfDNA | SQC<ADC |
| chr20 | 9706282 | 9706429 | zfDNA | SQC>ADC |
| chr20 | 33713618 | 33713757 | zfDNA | SQC<ADC |
| chr21 | 33340955 | 33341038 | zfDNA | SQC<ADC |
| chr22 | 21206849 | 21206995 | zfDNA | SQC>ADC |
| chr22 | 30292326 | 30292475 | zfDNA | SQC<ADC |
| chr22 | 35697444 | 35697606 | zfDNA | SQC<ADC |
| chr3 | 3755582 | 3755730 | zfDNA | SQC>ADC |
| chr3 | 14959981 | 14960128 | zfDNA | SQC<ADC |
| chr3 | 25581721 | 25581859 | zfDNA | SQC>ADC |
| chr3 | 75834579 | 75834736 | zfDNA | SQC<ADC |
| chr3 | 87031909 | 87032079 | zfDNA | SQC<ADC |
| chr3 | 122710736 | 122710872 | zfDNA | SQC>ADC |
| chr3 | 139727561 | 139727706 | zfDNA | SQC<ADC |
| chr3 | 145864433 | 145864574 | zfDNA | SQC>ADC |
| chr4 | 1665996 | 1666155 | zfDNA | SQC>ADC |
| chr4 | 22518120 | 22518271 | zfDNA | SQC<ADC |
| chr4 | 77306769 | 77306948 | zfDNA | SQC<ADC |
| chr4 | 82520036 | 82520212 | zfDNA | SQC<ADC |
| chr4 | 155413871 | 155414011 | zfDNA | SQC<ADC |
| chr4 | 156601279 | 156601436 | zfDNA | SQC>ADC |
| chr4 | 162457724 | 162457860 | zfDNA | SQC>ADC |
| chr4 | 176636441 | 176636580 | zfDNA | SQC>ADC |
| chr4 | 177654193 | 177654363 | zfDNA | SQC<ADC |
| chr5 | 14450118 | 14450272 | zfDNA | SQC<ADC |
| chr5 | 75935318 | 75935450 | zfDNA | SQC>ADC |
| chr5 | 140475728 | 140475872 | zfDNA | SQC>ADC |
| chr5 | 146345906 | 146346062 | zfDNA | SQC>ADC |
| chr5 | 156458027 | 156458167 | zfDNA | SQC<ADC |
| chr5 | 157169890 | 157170038 | zfDNA | SQC>ADC |
| chr6 | 20832000 | 20832349 | zfDNA | SQC>ADC |
| chr6 | 24420281 | 24420413 | zfDNA | SQC<ADC |
| chr6 | 36331071 | 36331215 | zfDNA | SQC<ADC |
| chr6 | 54074847 | 54075021 | zfDNA | SQC>ADC |
| chr6 | 71122323 | 71122483 | zfDNA | SQC>ADC |
| chr6 | 83604672 | 83604779 | zfDNA | SQC<ADC |
| chr6 | 90709859 | 90710016 | zfDNA | SQC>ADC |
| chr6 | 111744738 | 111744881 | zfDNA | SQC>ADC |
| chr6 | 148806765 | 148806922 | zfDNA | SQC<ADC |
| chr6 | 155574119 | 155574263 | zfDNA | SQC<ADC |
| chr6 | 158460178 | 158460323 | zfDNA | SQC>ADC |
| chr7 | 5549605 | 5549675 | zfDNA | SQC<ADC |
| chr7 | 40669616 | 40669796 | zfDNA | SQC>ADC |
| chr7 | 73799798 | 73799908 | zfDNA | SQC>ADC |
| chr7 | 78030021 | 78030155 | zfDNA | SQC<ADC |
| chr7 | 81399230 | 81399365 | zfDNA | SQC<ADC |
| chr7 | 134452355 | 134452524 | zfDNA | SQC>ADC |
| chr7 | 140335200 | 140335344 | zfDNA | SQC>ADC |
| chr7 | 146925646 | 146925824 | zfDNA | SQC>ADC |
| chr7 | 153976496 | 153976643 | zfDNA | SQC>ADC |
| chr7 | 157941162 | 157941344 | zfDNA | SQC<ADC |
| chr7 | 157980130 | 157980264 | zfDNA | SQC<ADC |
| chr7 | 157980485 | 157980624 | zfDNA | SQC<ADC |
| chr7 | 158314155 | 158314301 | zfDNA | SQC<ADC |
| chr8 | 6392188 | 6392336 | zfDNA | SQC<ADC |
| chr8 | 11724061 | 11724159 | zfDNA | SQC<ADC |
| chr8 | 17237496 | 17237639 | zfDNA | SQC<ADC |
| chr8 | 21803649 | 21803801 | zfDNA | SQC<ADC |
| chr8 | 52696850 | 52697008 | zfDNA | SQC<ADC |
| chr8 | 72183950 | 72184120 | zfDNA | SQC>ADC |
| chr8 | 81042553 | 81042694 | zfDNA | SQC>ADC |
| chr8 | 85101824 | 85101952 | zfDNA | SQC>ADC |
| chr8 | 110703169 | 110703320 | zfDNA | SQC<ADC |
| chr8 | 121727803 | 121727944 | zfDNA | SQC<ADC |
| chr8 | 133476418 | 133476558 | zfDNA | SQC<ADC |
| chr9 | 8813022 | 8813150 | zfDNA | SQC<ADC |
| chr9 | 90258110 | 90258253 | zfDNA | SQC<ADC |
| chr9 | 97061691 | 97061835 | zfDNA | SQC>ADC |

| **Tab 1 C. Prognose: günstig oder ungünstig?** | | | |
|---|---|---|---|
| Chromosom | Position | Methode | Methylierung |
| 1 | 18063105 | *Paired Biopsies* (HM 450K) | hohe Methylierung = schlechte Prognose |
| 1 | 26699448 | *Paired Biopsies* (HM 450K) | hohe Methylierung = schlechte Prognose |
| 1 | 115677211 | *Paired Biopsies* (HM 450K) | hohe Methylierung = schlechte Prognose |
| 1 | 226187852 | *Paired Biopsies* (HM 450K) | hohe Methylierung = schlechte Prognose |
| 1 | 226187876 | *Paired Biopsies* (HM 450K) | hohe Methylierung = schlechte Prognose |
| 1 | 226188006 | *Paired Biopsies* (HM 450K) | hohe Methylierung = schlechte Prognose |
| 2 | 27362420 | *Paired Biopsies* (HM 450K) | hohe Methylierung = schlechte Prognose |
| 2 | 241314588 | *Paired Biopsies* (HM 450K) | hohe Methylierung = schlechte Prognose |
| 2 | 241344707 | *Paired Biopsies* (HM 450K) | hohe Methylierung = schlechte Prognose |
| 3 | 13914731 | *Paired Biopsies* (HM 450K) | hohe Methylierung = schlechte Prognose |
| 5 | 176167283 | *Paired Biopsies* (HM 450K) | hohe Methylierung = schlechte Prognose |
| 6 | 29528774 | *Paired Biopsies* (HM 450K) | hohe Methylierung = schlechte Prognose |
| 6 | 154869909 | *Paired Biopsies* (HM 450K) | hohe Methylierung = schlechte Prognose |
| 7 | 42195875 | *Paired Biopsies* (HM 450K) | hohe Methylierung = schlechte Prognose |
| 8 | 10452896 | *Paired Biopsies* (HM 450K) | hohe Methylierung = schlechte Prognose |
| 8 | 11614472 | *Paired Biopsies* (HM 450K) | hohe Methylierung = schlechte Prognose |
| 8 | 49382369 | *Paired Biopsies* (HM 450K) | hohe Methylierung = schlechte Prognose |
| 8 | 49466210 | *Paired Biopsies* (HM 450K) | hohe Methylierung = schlechte Prognose |
| 8 | 49494724 | *Paired Biopsies* (HM 450K) | hohe Methylierung = schlechte Prognose |
| 8 | 49496369 | *Paired Biopsies* (HM 450K) | hohe Methylierung = schlechte Prognose |
| 8 | 49496391 | *Paired Biopsies* (HM 450K) | hohe Methylierung = schlechte Prognose |
| 8 | 49533444 | *Paired Biopsies* (HM 450K) | hohe Methylierung = schlechte Prognose |
| 8 | 49547126 | *Paired Biopsies* (HM 450K) | hohe Methylierung = schlechte Prognose |
| 8 | 49823433 | *Paired Biopsies* (HM 450K) | hohe Methylierung = schlechte Prognose |
| 9 | 133792985 | *Paired Biopsies* (HM 450K) | hohe Methylierung = schlechte Prognose |
| 10 | 54223605 | *Paired Biopsies* (HM 450K) | hohe Methylierung = schlechte Prognose |
| 11 | 1673436 | *Paired Biopsies* (HM 450K) | hohe Methylierung = schlechte Prognose |
| 14 | 99700232 | *Paired Biopsies* (HM 450K) | hohe Methylierung = schlechte Prognose |
| 20 | 584773 | *Paired Biopsies* (HM 450K) | hohe Methylierung = schlechte Prognose |
| 20 | 2508981 | *Paired Biopsies* (HM 450K) | hohe Methylierung = schlechte Prognose |
| 20 | 4201164 | *Paired Biopsies* (HM 450K) | hohe Methylierung = schlechte Prognose |
| 20 | 43028501 | *Paired Biopsies* (HM 450K) | hohe Methylierung = schlechte Prognose |
| 20 | 46323481 | *Paired Biopsies* (HM 450K) | hohe Methylierung = schlechte Prognose |

**Tab. 2: Der kNN Algorithmus verwendete zehn Positionen, um die Lungenkarzinompatienten von den gesunden Probanden unterscheiden zu können. In der Spalte "Tumor" ist angegeben, ob in Tumorgewebe eine verstärkte (+) oder verminderte (-) Methylierung identifiziert wurde. A**

| **a) ID** | **Chromosom** | **Position** | **Tumor** |
|---|---|---|---|
| 596 | chr11 | 57006229 | + |
| 1717 | chr15 | 28262724 | + |
| 2636 | chr18 | 61144199 | - |
| 2805 | chr19 | 46823441 | |
| 4674 | chr2 | 176964685 | + |
| 4999 | chr2 | 225642035 | + |
| 5071 | chr3 | 14960020 | + |
| 5576 | chr4 | 13525705 | + |
| 6105 | chr5 | 140475760 | + |
| 6434 | chr6 | 46386723 | + |
| | | | |

| **b) Gruppe** | | **Richtigkeit** | **Anzahl anProben** |
|---|---|---|---|
| Maligner Tumor | | 1 | 9 |
| Kontrolle | | 1 | 3 |
| Mittelwert | | 1 | 12 |
| Weitere Parameter | | | |
| K | | 5 | |
| Rangordnung | | Vergleich zweier Gruppen | |
| Normalisierung | | Mittelwert = 0, Varianz = 1 | |
| Fehlender Wert | | Mittelwert | |

**Tab. 3: Der RT Algorithmus analysierte zehn Positionen um die Entität eines Tumors zu ermitteln. Alle Positionen waren beim Adenokarzinom im Vergleich zum Plattenepithelkarzinom hypermethyliert.**

| **a) ID** | **Chromosom** | **Position** |
|---|---|---|
| 650 | chr11 | 64993331 |
| 2995 | chr1 | 17568007 |
| 4233 | chr2 | 50574690 |
| 4241 | chr2 | 50574708 |
| 4428 | chr2 | 111874494 |
| 4447 | chr2 | 121276804 |
| 5537 | chr4 | 1666074 |
| 5538 | chr4 | 1666075 |
| 6524 | chr6 | 83604790 |
| 7164 | chr7 | 69971740 |
| | | |

| **b) Gruppe** | **Richtigkeit** | **Anzahl an Proben** |
|---|---|---|
| Adenokarzinom | 1 | 4 |
| Plattenepithelkarzinom | 1 | 5 |
| Mittelwert | 1 | 9 |
| Weitere Parameter | | |
| Max. Tiefe | 25 | |
| Min. Anteil der Stichprobe | 1% | |
| Max. Anzahl an Kategorien | 10 | |
| Max. Anzahl an Bäumen | 250 | |
| Richtigkeit des Waldes | 0.1 | |
| Kriterien für das Terminieren | Max. Anzahl an Bäumen | |
| Rangordnung | Vergleich zweier Gruppen | |
| Normalisierung | Mittelwert = 0, Varianz = 1 | |
| Fehlender Wert | Mittelwert | |

**Tab. 4: Für das Staging (Feststellen des Tumorstadiums) wurden vom SVM Algorithmus 523 Positionen analysiert. Einige Positionen sind im späten Stadium verstärkt methyliert (+), andere Positionen hingegen vermindert methyliert (-).**

| ***a) ID*** | ***Chromosom*** | ***Position*** | ***Spätes (III, IV) Stadium*** |
|---|---|---|---|
| 16 | chr10 | 12533708 | + |
| 17 | chr10 | 12533710 | + |
| 20 | chr10 | 12533754 | - |
| 26 | chr10 | 15110983 | - |
| 37 | chr10 | 32657656 | - |
| 38 | chr10 | 32657672 | - |
| 79 | chr10 | 62708202 | + |
| 104 | chr10 | 97033706 | - |
| 123 | chr10 | 98129889 | - |
| 154 | chr10 | 102895057 | - |
| 164 | chr10 | 102984248 | + |
| 196 | chr10 | 102987003 | + |
| 199 | chr10 | 102987007 | + |
| 269 | chr10 | 121075316 | + |
| 281 | chr10 | 123914718 | - |
| 315 | chr10 | 124905781 | + |
| 320 | chr10 | 126494586 | - |
| 327 | chr10 | 126494644 | + |
| 333 | chr10 | 130860205 | - |
| 347 | chr10 | 134598357 | + |
| 349 | chr10 | 134598359 | + |
| 364 | chr11 | 627157 | - |
| 382 | chr11 | 1328455 | - |
| 385 | chr11 | 1328485 | - |
| 411 | chr11 | 15025433 | - |
| 431 | chr11 | 19778814 | + |
| 473 | chr11 | 26355627 | + |
| 479 | chr11 | 26626371 | + |
| 483 | chr11 | 26626471 | - |
| 554 | chr11 | 31831858 | + |
| 568 | chr11 | 31831899 | + |
| 572 | chr11 | 31831908 | + |
| 576 | chr11 | 31831919 | + |
| 585 | chr11 | 40136809 | + |
| 677 | chr11 | 69061863 | - |
| 696 | chr11 | 71188761 | - |
| 697 | chr11 | 71188762 | + |
| 709 | chr11 | 82444757 | - |
| 712 | chr11 | 82444771 | - |
| 742 | chr11 | 86085932 | + |
| 760 | chr11 | 113629710 | - |
| 767 | chr11 | 113629767 | - |
| 768 | chr11 | 114052115 | + |
| 793 | chr11 | 122678513 | + |
| 819 | chr11 | 134246113 | + |
| 822 | chr12 | 1943225 | + |
| 823 | chr12 | 1943226 | + |
| 824 | chr12 | 1943232 | + |
| 827 | chr12 | 2526357 | + |
| 831 | chr12 | 2526402 | + |
| 849 | chr12 | 2751699 | + |
| 867 | chr12 | 4381792 | - |
| 873 | chr12 | 4381812 | - |
| 880 | chr12 | 4381851 | + |
| 958 | chr12 | 33592641 | + |
| 963 | chr12 | 33592661 | + |
| 966 | chr12 | 33592673 | + |
| 970 | chr12 | 33592682 | + |
| 976 | chr12 | 34358517 | + |
| 1005 | chr12 | 34503613 | - |
| 1041 | chr12 | 54423543 | + |
| 1052 | chr12 | 54423567 | + |
| 1171 | chr12 | 97140890 | - |
| 1179 | chr12 | 108051521 | + |
| 1196 | chr12 | 112427829 | - |
| 1222 | chr12 | 123203607 | + |
| 1223 | chr12 | 123203612 | + |
| 1227 | chr12 | 123203644 | + |
| 1235 | chr12 | 125571833 | - |
| 1239 | chr12 | 126142895 | - |
| 1244 | chr12 | 129347679 | - |
| 1265 | chr12 | 129886069 | - |
| 1272 | chr12 | 129886183 | + |
| 1287 | chr13 | 36828832 | + |
| 1305 | chr13 | 48806483 | - |
| 1317 | chr13 | 58207814 | - |
| 1319 | chr13 | 58207831 | - |
| 1335 | chr13 | 93325573 | - |
| 1336 | chr13 | 93325602 | - |
| 1348 | chr13 | 100621150 | + |
| 1354 | chr13 | 100621175 | + |
| 1358 | chr13 | 100621185 | - |
| 1362 | chr13 | 100621194 | - |
| 1394 | chr13 | 100624346 | + |
| 1415 | chr14 | 23511181 | + |
| 1441 | chr14 | 35030414 | - |
| 1442 | chr14 | 35030415 | - |
| 1443 | chr14 | 35231195 | + |
| 1461 | chr14 | 37128597 | + |
| 1472 | chr14 | 55907282 | + |
| 1478 | chr14 | 55907299 | + |
| 1487 | chr14 | 57274719 | + |
| 1512 | chr14 | 57275127 | + |
| 1550 | chr14 | 57275995 | + |
| 1610 | chr14 | 57278179 | - |
| 1612 | chr14 | 57278187 | + |
| 1619 | chr14 | 57278220 | - |
| 1637 | chr14 | 58064926 | - |
| 1701 | chr14 | 104486258 | - |
| 1702 | chr14 | 104486260 | + |
| 1710 | chr15 | 26964987 | - |
| 1714 | chr15 | 28262702 | - |
| 1732 | chr15 | 32405064 | + |
| 1733 | chr15 | 32405065 | + |
| 1738 | chr15 | 41925179 | - |
| 1740 | chr15 | 41925187 | + |
| 1741 | chr15 | 41925188 | - |
| 1767 | chr15 | 45409283 | + |
| 1768 | chr15 | 45409293 | + |
| 1780 | chr15 | 56006548 | + |
| 1784 | chr15 | 63349191 | + |
| 1786 | chr15 | 63349194 | + |
| 1787 | chr15 | 63349195 | + |
| 1807 | chr15 | 69087782 | - |
| 1820 | chr15 | 72520614 | + |
| 1831 | chr15 | 83316217 | - |
| 1840 | chr15 | 83316257 | - |
| 1841 | chr15 | 83316258 | + |
| 1846 | chr15 | 83316269 | + |
| 1847 | chr15 | 83316270 | + |
| 1873 | chr15 | 89920814 | + |
| 1879 | chr15 | 89920855 | + |
| 1893 | chr15 | 89922297 | + |
| 1908 | chr15 | 89922344 | - |
| 1910 | chr15 | 89922358 | - |
| 1915 | chr15 | 89922387 | - |
| 1919 | chr15 | 98477887 | + |
| 1934 | chr16 | 526638 | + |
| 1956 | chr16 | 1202458 | - |
| 1973 | chr16 | 2880458 | + |
| 1974 | chr16 | 2880459 | + |
| 1991 | chr16 | 15665653 | + |
| 2014 | chr16 | 24822719 | - |
| 2056 | chr16 | 34255535 | - |
| 2067 | chr16 | 56224754 | + |
| 2071 | chr16 | 56224777 | + |
| 2080 | chr16 | 56224809 | - |
| 2106 | chr16 | 66613071 | + |
| 2133 | chr16 | 66613250 | + |
| 2157 | chr16 | 71528178 | + |
| 2171 | chr16 | 75528661 | + |
| 2187 | chr16 | 86600283 | + |
| 2199 | chr16 | 86600325 | + |
| 2205 | chr16 | 86600340 | + |
| 2214 | chr16 | 88014067 | + |
| 2216 | chr16 | 88014072 | + |
| 2218 | chr16 | 88014083 | - |
| 2222 | chr16 | 89714003 | + |
| 2225 | chr16 | 89714008 | + |
| 2226 | chr16 | 89714009 | - |
| 2237 | chr16 | 89714063 | + |
| 2249 | chr17 | 416795 | - |
| 2274 | chr17 | 750241 | - |
| 2285 | chr17 | 19809748 | - |
| 2286 | chr17 | 19809749 | - |
| 2291 | chr17 | 29174301 | + |
| 2299 | chr17 | 29174410 | + |
| 2317 | chr17 | 33314935 | - |
| 2322 | chr17 | 33314978 | - |
| 2327 | chr17 | 33314988 | + |
| 2333 | chr17 | 33365076 | - |
| 2356 | chr17 | 35299620 | + |
| 2371 | chr17 | 42960474 | + |
| 2373 | chr17 | 42960488 | + |
| 2389 | chr17 | 46799639 | + |
| 2391 | chr17 | 46799644 | + |
| 2393 | chr17 | 46799647 | - |
| 2394 | chr17 | 46799648 | + |
| 2406 | chr17 | 48048981 | + |
| 2411 | chr17 | 48049008 | + |
| 2435 | chr17 | 59532275 | + |
| 2443 | chr17 | 59532314 | + |
| 2453 | chr17 | 64330651 | + |
| 2459 | chr17 | 66292378 | - |
| 2463 | chr17 | 67536298 | - |
| 2465 | chr17 | 72619555 | - |
| 2481 | chr17 | 75142814 | + |
| 2506 | chr17 | 80794324 | - |
| 2530 | chr18 | 3971140 | + |
| 2541 | chr18 | 18658118 | - |
| 2545 | chr18 | 20714345 | + |
| 2550 | chr18 | 21596915 | + |
| 2559 | chr18 | 24131108 | - |
| 2574 | chr18 | 24131391 | + |
| 2600 | chr18 | 61143901 | + |
| 2624 | chr18 | 61144121 | - |
| 2665 | chr19 | 5141394 | + |
| 2688 | chr19 | 10572317 | - |
| 2711 | chr19 | 11891002 | - |
| 2739 | chr19 | 19625286 | - |
| 2756 | chr19 | 29991306 | - |
| 2767 | chr19 | 33102573 | + |
| 2769 | chr19 | 42600236 | + |
| 2789 | chr19 | 44629761 | + |
| 2791 | chr19 | 45782682 | + |
| 2803 | chr19 | 46823435 | - |
| 2815 | chr19 | 49016516 | - |
| 2820 | chr19 | 49016533 | - |
| 2824 | chr19 | 49503049 | + |
| 2839 | chr19 | 49909923 | - |
| 2862 | chr1 | 2198846 | + |
| 2863 | chr1 | 2198847 | + |
| 2867 | chr1 | 2198863 | + |
| 2958 | chr1 | 8787209 | + |
| 2962 | chr1 | 8787261 | - |
| 2973 | chr1 | 15426297 | + |
| 2984 | chr1 | 15670515 | - |
| 2992 | chr1 | 17568000 | - |
| 3005 | chr1 | 17568145 | + |
| 3017 | chr1 | 19764666 | + |
| 3019 | chr1 | 19764669 | + |
| 3025 | chr1 | 19764722 | - |
| 3031 | chr1 | 23284390 | - |
| 3033 | chr1 | 23284423 | - |
| 3045 | chr1 | 27234577 | - |
| 3053 | chr1 | 27234623 | - |
| 3056 | chr1 | 27234626 | - |
| 3066 | chr1 | 34642399 | + |
| 3110 | chr1 | 50883372 | + |
| 3121 | chr1 | 50886745 | + |
| 3127 | chr1 | 50886772 | + |
| 3164 | chr1 | 50886995 | + |
| 3174 | chr1 | 61668834 | + |
| 3176 | chr1 | 63489100 | - |
| 3225 | chr1 | 108975412 | - |
| 3227 | chr1 | 108975445 | + |
| 3284 | chr1 | 115677210 | + |
| 3315 | chr1 | 155162705 | - |
| 3348 | chr1 | 160783053 | - |
| 3390 | chr1 | 161008828 | + |
| 3392 | chr1 | 161008848 | + |
| 3402 | chr1 | 161306175 | - |
| 3454 | chr1 | 180202553 | - |
| 3488 | chr1 | 217310587 | + |
| 3501 | chr1 | 220101724 | + |
| 3509 | chr1 | 220101774 | - |
| 3519 | chr1 | 220101934 | + |
| 3538 | chr1 | 223948910 | + |
| 3554 | chr1 | 236849473 | + |
| 3579 | chr1 | 236849941 | - |
| 3586 | chr1 | 236849958 | + |
| 3616 | chr1 | 240656582 | + |
| 3630 | chr20 | 584741 | + |
| 3631 | chr20 | 584744 | + |
| 3636 | chr20 | 2508981 | - |
| 3641 | chr20 | 5282947 | - |
| 3651 | chr20 | 9706361 | + |
| 3652 | chr20 | 9706362 | + |
| 3655 | chr20 | 19910263 | + |
| 3724 | chr20 | 46323527 | + |
| 3748 | chr20 | 47444816 | + |
| 3762 | chr20 | 47444849 | + |
| 3764 | chr20 | 47444851 | + |
| 3795 | chr20 | 47444971 | + |
| 3809 | chr20 | 47445025 | - |
| 3923 | chr21 | 38077257 | + |
| 3970 | chr22 | 29956486 | + |
| 3977 | chr22 | 30292389 | - |
| 3990 | chr22 | 35697550 | + |
| 3992 | chr22 | 35697558 | + |
| 3999 | chr22 | 40810375 | + |
| 4005 | chr22 | 45129980 | + |
| 4018 | chr22 | 45992027 | + |
| 4021 | chr22 | 45992040 | - |
| 4070 | chr2 | 3642648 | + |
| 4078 | chr2 | 9987439 | - |
| 4142 | chr2 | 32313668 | + |
| 4158 | chr2 | 45171790 | + |
| 4183 | chr2 | 45232432 | + |
| 4236 | chr2 | 50574700 | - |
| 4257 | chr2 | 63276180 | - |
| 4258 | chr2 | 63276181 | - |
| 4265 | chr2 | 63276215 | + |
| 4299 | chr2 | 63281187 | + |
| 4391 | chr2 | 63284132 | + |
| 4399 | chr2 | 63284165 | - |
| 4406 | chr2 | 73021347 | - |
| 4414 | chr2 | 100209375 | - |
| 4434 | chr2 | 113534594 | + |
| 4446 | chr2 | 120418056 | + |
| 4472 | chr2 | 152992678 | - |
| 4479 | chr2 | 155089854 | - |
| 4550 | chr2 | 162283795 | + |
| 4574 | chr2 | 175199659 | + |
| 4597 | chr2 | 175199740 | + |
| 4642 | chr2 | 176964121 | + |
| 4683 | chr2 | 176964710 | + |
| 4761 | chr2 | 176988910 | + |
| 4768 | chr2 | 176988939 | + |
| 4797 | chr2 | 177014908 | + |
| 4800 | chr2 | 177014949 | + |
| 4820 | chr2 | 177027453 | - |
| 4845 | chr2 | 179897288 | + |
| 4864 | chr2 | 200326684 | + |
| 4873 | chr2 | 200326734 | + |
| 4998 | chr2 | 225642025 | + |
| 5005 | chr2 | 236444281 | + |
| 5023 | chr2 | 240882054 | + |
| 5030 | chr2 | 240882093 | + |
| 5040 | chr2 | 241314636 | + |
| 5054 | chr2 | 242273197 | + |
| 5057 | chr2 | 242273208 | - |
| 5058 | chr2 | 242273209 | - |
| 5059 | chr2 | 242273212 | + |
| 5072 | chr3 | 14960021 | + |
| 5093 | chr3 | 37544000 | - |
| 5097 | chr3 | 38567610 | + |
| 5104 | chr3 | 46904943 | + |
| 5108 | chr3 | 48837718 | + |
| 5120 | chr3 | 68947458 | + |
| 5122 | chr3 | 69280664 | + |
| 5125 | chr3 | 69280734 | - |
| 5146 | chr3 | 87032003 | + |
| 5147 | chr3 | 87032004 | + |
| 5153 | chr3 | 114074297 | + |
| 5156 | chr3 | 122710786 | + |
| 5157 | chr3 | 122710787 | + |
| 5160 | chr3 | 122710815 | + |
| 5162 | chr3 | 122710835 | + |
| 5163 | chr3 | 122710836 | + |
| 5164 | chr3 | 122710859 | + |
| 5165 | chr3 | 122710860 | + |
| 5167 | chr3 | 122841632 | - |
| 5282 | chr3 | 147108882 | + |
| 5303 | chr3 | 147113870 | + |
| 5380 | chr3 | 150948274 | - |
| 5388 | chr3 | 160167967 | + |
| 5391 | chr3 | 160167976 | + |
| 5436 | chr3 | 178907655 | + |
| 5483 | chr3 | 184742720 | + |
| 5510 | chr3 | 196440526 | - |
| 5522 | chr4 | 738427 | - |
| 5531 | chr4 | 738469 | - |
| 5532 | chr4 | 738470 | - |
| 5536 | chr4 | 1666071 | + |
| 5550 | chr4 | 8017839 | - |
| 5556 | chr4 | 8288935 | + |
| 5561 | chr4 | 13525657 | + |
| 5567 | chr4 | 13525666 | - |
| 5577 | chr4 | 13525721 | + |
| 5596 | chr4 | 21886957 | + |
| 5602 | chr4 | 26398259 | + |
| 5613 | chr4 | 57522468 | + |
| 5623 | chr4 | 57522505 | + |
| 5624 | chr4 | 57522506 | + |
| 5632 | chr4 | 57522618 | + |
| 5634 | chr4 | 57522620 | + |
| 5640 | chr4 | 57522642 | + |
| 5652 | chr4 | 57522762 | + |
| 5664 | chr4 | 71520485 | + |
| 5670 | chr4 | 77306910 | + |
| 5691 | chr4 | 82520132 | - |
| 5692 | chr4 | 82520133 | + |
| 5698 | chr4 | 94616253 | + |
| 5773 | chr4 | 113432594 | + |
| 5794 | chr4 | 151504732 | - |
| 5816 | chr4 | 176636539 | - |
| 5833 | chr4 | 183696160 | - |
| 5834 | chr4 | 183696161 | + |
| 5836 | chr4 | 183696165 | - |
| 5837 | chr4 | 186659550 | - |
| 5844 | chr5 | 912688 | + |
| 5861 | chr5 | 912783 | + |
| 5863 | chr5 | 912786 | + |
| 5865 | chr5 | 912803 | + |
| 5869 | chr5 | 912820 | + |
| 5872 | chr5 | 912834 | + |
| 5875 | chr5 | 912839 | + |
| 5947 | chr5 | 5146384 | - |
| 5949 | chr5 | 5568539 | + |
| 5955 | chr5 | 5568625 | - |
| 5956 | chr5 | 9782141 | - |
| 5975 | chr5 | 15824429 | - |
| 5992 | chr5 | 16179153 | + |
| 6040 | chr5 | 39187223 | + |
| 6065 | chr5 | 75935385 | + |
| 6081 | chr5 | 125881737 | + |
| 6083 | chr5 | 125881794 | - |
| 6115 | chr5 | 140475801 | + |
| 6128 | chr5 | 140810902 | - |
| 6130 | chr5 | 140810918 | + |
| 6156 | chr5 | 140811674 | - |
| 6163 | chr5 | 146345982 | - |
| 6164 | chr5 | 146345983 | - |
| 6167 | chr5 | 146346033 | - |
| 6169 | chr5 | 155481326 | + |
| 6174 | chr5 | 155481363 | + |
| 6186 | chr5 | 157169968 | - |
| 6191 | chr5 | 160171802 | + |
| 6194 | chr5 | 160171823 | + |
| 6206 | chr5 | 169064444 | - |
| 6233 | chr5 | 176167243 | - |
| 6237 | chr5 | 176167283 | + |
| 6253 | chr5 | 177966092 | - |
| 6255 | chr5 | 179180016 | + |
| 6273 | chr6 | 1656954 | - |
| 6274 | chr6 | 1656975 | + |
| 6281 | chr6 | 1657068 | + |
| 6283 | chr6 | 5132887 | + |
| 6302 | chr6 | 20832186 | - |
| 6309 | chr6 | 20832253 | - |
| 6363 | chr6 | 28227164 | - |
| 6365 | chr6 | 29528773 | - |
| 6367 | chr6 | 30130880 | + |
| 6368 | chr6 | 30130881 | + |
| 6390 | chr6 | 34984930 | - |
| 6394 | chr6 | 36253053 | + |
| 6502 | chr6 | 54074944 | + |
| 6511 | chr6 | 63991019 | - |
| 6512 | chr6 | 63991020 | + |
| 6531 | chr6 | 90709953 | - |
| 6537 | chr6 | 100051149 | + |
| 6565 | chr6 | 100054714 | + |
| 6616 | chr6 | 100061098 | + |
| 6625 | chr6 | 100905468 | + |
| 6666 | chr6 | 108675552 | - |
| 6674 | chr6 | 111744817 | - |
| 6693 | chr6 | 138866887 | + |
| 6698 | chr6 | 139205501 | + |
| 6723 | chr6 | 158056087 | - |
| 6760 | chr6 | 169050364 | + |
| 6778 | chr7 | 187685 | + |
| 6782 | chr7 | 187715 | + |
| 6784 | chr7 | 653308 | - |
| 6805 | chr7 | 1491812 | + |
| 6813 | chr7 | 1491843 | + |
| 6815 | chr7 | 1491858 | + |
| 6821 | chr7 | 1491902 | - |
| 6825 | chr7 | 1491921 | - |
| 6829 | chr7 | 1491966 | - |
| 6890 | chr7 | 4228760 | - |
| 6895 | chr7 | 4228818 | - |
| 6896 | chr7 | 4786867 | + |
| 6903 | chr7 | 4786899 | - |
| 6906 | chr7 | 4786942 | + |
| 6949 | chr7 | 6269027 | - |
| 6958 | chr7 | 19156576 | + |
| 6969 | chr7 | 19156620 | - |
| 6999 | chr7 | 19157240 | - |
| 7093 | chr7 | 40100597 | - |
| 7114 | chr7 | 44200085 | - |
| 7131 | chr7 | 45197448 | - |
| 7157 | chr7 | 65617361 | + |
| 7160 | chr7 | 65617365 | + |
| 7165 | chr7 | 69971769 | + |
| 7185 | chr7 | 73981010 | + |
| 7242 | chr7 | 96622713 | + |
| 7252 | chr7 | 102574104 | + |
| 7253 | chr7 | 102574105 | + |
| 7261 | chr7 | 102574475 | + |
| 7265 | chr7 | 102574499 | - |
| 7269 | chr7 | 102574518 | - |
| 7277 | chr7 | 111825813 | + |
| 7307 | chr7 | 134452411 | - |
| 7312 | chr7 | 134452451 | - |
| 7313 | chr7 | 134452452 | + |
| 7324 | chr7 | 140335252 | + |
| 7326 | chr7 | 140335261 | + |
| 7333 | chr7 | 141397873 | - |
| 7335 | chr7 | 146925681 | - |
| 7365 | chr7 | 151300170 | - |
| 7368 | chr7 | 151300199 | + |
| 7400 | chr7 | 154087938 | + |
| 7410 | chr7 | 157691297 | - |
| 7417 | chr7 | 157691343 | - |
| 7420 | chr7 | 157941187 | + |
| 7424 | chr7 | 157941301 | - |
| 7430 | chr7 | 157980193 | - |
| 7433 | chr7 | 157980216 | + |
| 7439 | chr7 | 158314202 | - |
| 7443 | chr7 | 158314235 | + |
| 7463 | chr8 | 6735007 | + |
| 7466 | chr8 | 10452854 | + |
| 7473 | chr8 | 10452918 | - |
| 7478 | chr8 | 11614472 | - |
| 7482 | chr8 | 11614502 | + |
| 7483 | chr8 | 11614519 | - |
| 7484 | chr8 | 11614520 | + |
| 7502 | chr8 | 14422449 | - |
| 7521 | chr8 | 26286645 | + |
| 7539 | chr8 | 31676500 | + |
| 7544 | chr8 | 31676524 | + |
| 7553 | chr8 | 31676686 | + |
| 7555 | chr8 | 31676697 | - |
| 7565 | chr8 | 41754146 | - |
| 7608 | chr8 | 49382365 | + |
| 7622 | chr8 | 49494676 | - |
| 7626 | chr8 | 49494699 | + |
| 7630 | chr8 | 49494723 | + |
| 7634 | chr8 | 49494773 | - |
| 7636 | chr8 | 49496331 | - |
| 7671 | chr8 | 49823388 | - |
| 7673 | chr8 | 49823395 | + |
| 7696 | chr8 | 62323244 | + |
| 7727 | chr8 | 72184078 | + |
| 7734 | chr8 | 85101900 | + |
| 7740 | chr8 | 110703242 | + |
| 7742 | chr8 | 110703245 | + |
| 7759 | chr8 | 123875007 | + |
| 7762 | chr8 | 123875033 | - |
| 7763 | chr8 | 123875034 | + |
| 7766 | chr8 | 123875051 | + |
| 7773 | chr8 | 123875079 | - |
| 7782 | chr8 | 123875253 | - |
| 7786 | chr8 | 126258311 | - |
| 7787 | chr8 | 126258312 | + |
| 7793 | chr8 | 129339255 | - |
| 7802 | chr8 | 141127120 | + |
| 7821 | chr9 | 8813092 | + |
| 7846 | chr9 | 37002714 | + |
| 7859 | chr9 | 74342704 | + |
| 7863 | chr9 | 74342751 | - |
| 7868 | chr9 | 79060545 | + |
| 7876 | chr9 | 90258167 | + |
| 7879 | chr9 | 90258174 | + |
| 7893 | chr9 | 97061769 | + |
| 7894 | chr9 | 97061770 | + |
| 7900 | chr9 | 115478948 | - |
| 7904 | chr9 | 115478954 | - |
| 7925 | chr9 | 126166763 | + |
| 7926 | chr9 | 126166764 | - |
| 7927 | chr9 | 133792936 | - |
| 7932 | chr9 | 133792984 | - |

| **b) Gruppe** | **Richtigkeit** | **Anzahl an Proben** | |
|---|---|---|---|
| Frühes Stadium | 0 | 4 | |
| Spätes Stadium | 0,8 | 5 | |
| Mittelwert | 0,4 | 9 | |

| **Weitere Parameter** | | | |
|---|---|---|---|
| Typ | C_SVC | | |
| Kernel-Typ | Linear | | |
| Ausgabe | 0.0001 | | |
| Nu | 0,5 | | |
| Epsilon SVR | 0,1 | | |
| Kriterien für das Terminieren | Das Epsilon-Terminieren oder max. Iterationen | | |
| Das Epsilon-Terminieren | 0.001 | | |
| Max. Iterationen | 1000 | | |
| Rangordnung Filtern nach Bedingung | Vergleich zweier Gruppen Gruppe ungleich, != | | |
| Normalisierung Fehlender Wert | Mittelwert = 0, Varianz = 1 Mittelwert | | |

**Tab. 5: Beispielhafte, in dem erfindungsgemäßen Verfahren einsetzbare Oligonukleotide (Capture Targets) für Marker auf Chromosom 1.**

| **Start** | **Stop** | **Länge [bp]** |
|---|---|---|
| 2198804 | 2198961 chr1:2198830-2198930 | 157 |
| 3289010 | 3289139 chr1:3289034-3289134 | 129 |
| 3607047 | 3607181 chr1:3607067-3607167 | 134 |
| 6130197 | 6130338 chr1:6130273-6130274 | 141 |
| 6165201 | 6165361 chr1:6165229-6165329 | 160 |
| 6515521 | 6515702 chr1:6515548-6515648;chr1:6515574-6515674 | 181 |
| 6520115 | 6520257 chr1:6520145-6520245 | 142 |
| 8787128 | 8787253 chr1:8787221-8787321,upstream | 125 |
| 15426262 | 15426418 chr1:15426289-15426389 | 156 |
| 15670403 | 15670539 chr1:15670433-15670533 | 136 |
| | chr1:17567922-17568022;chr1:17568066- | |
| 17567892 | 17568189 17568166 | 297 |
| 18063027 | 18063184 chr1:18063106-18063107 | 157 |
| 19177630 | 19177804 chr1:19177728-19177729 | 174 |
| 19764609 | 19764757 chr1:19764637-19764737 | 148 |
| 23284417 | 23284507 chr1:23284374-23284474 | 90 |
| 24277975 | 24278154 chr1:24278024-24278124 | 179 |
| 26699371 | 26699517 chr1:26699448-26699449 | 146 |
| 27234664 | 27234812 chr1:27234575-27234675,downstream | 148 |
| 34642324 | 34642455 chr1:34642347-34642447 | 131 |
| 36194564 | 36194662 chr1:36194581-36194582 | 98 |
| 38591827 | 38591977 chr1:38591903-38591904 | 150 |
| 47694840 | 47694995 chr1:47694870-47694970 | 155 |
| 47738990 | 47739142 chr1:47739010-47739110 | 152 |
| 50883315 | 50883461 chr1:50883345-50883445 | 146 |
| 50886707 | 50886857 chr1:50886733-50886833 | 150 |
| 50886870 | 50887021 chr1:50886900-50887000 | 151 |
| 52158087 | 52158220 chr1:52158112-52158212 | 133 |
| 57955028 | 57955174 chr1:57955057-57955157 | 146 |
| 61668739 | 61668922 chr1:61668786-61668886 | 183 |
| 63489039 | 63489179 chr1:63489116-63489117 | 140 |
| 64578151 | 64578293 chr1:64578178-64578278 | 142 |
| 77533495 | 77533671 chr1:77533543-77533643 | 176 |
| 79467955 | 79468081 chr1:79467974-79468074 | 126 |
| 79472375 | 79472516 chr1:79472403-79472503 | 141 |
| 85449266 | 85449364 chr1:85449395-85449495,upstream | 98 |
| 108975333 | 108975476 chr1:108975362-108975462 | 143 |
| 109383819 | 109383912 chr1:109383701-109383801,downstream | 93 |
| 110610821 | 110610964 chr1:110610850-110610950 | 143 |
| 110611386 | 110611542 chr1:110611416-110611516 | 156 |
| 110611971 | 110612108 chr1:110611995-110612095 | 137 |
| 115677141 | 115677297 chr1:115677211-115677212 | 156 |
| 119522559 | 119522707 chr1:119522588-119522688 | 148 |
| 150595130 | 150595282 chr1:150595157-150595257 | 152 |
| 153896523 | 153896648 chr1:153896541-153896641 | 125 |
| 154379671 | 154379808 chr1:154379748-154379749 | 137 |
| 155162673 | 155162808 chr1:155162703-155162803 | 135 |
| 158079244 | 158079395 chr1:158079311-158079312 | 151 |
| 158324396 | 158324540 chr1:158324422-158324522 | 144 |
| 158549201 | 158549351 chr1:158549228-158549328 | 150 |
| 158575697 | 158575854 chr1:158575724-158575824 | 157 |
| 158736216 | 158736378 chr1:158736263-158736363 | 162 |
| 159284004 | 159284160 chr1:159284033-159284133 | 156 |
| 159284209 | 159284363 chr1:159284249-159284349 | 154 |
| 159682419 | 159682564 chr1:159682448-159682548 | 145 |
| 160782978 | 160783141 chr1:160783005-160783105 | 163 |
| | chr1:161008656-161008756;chr1:161008701- | |
| 161008634 | 161008907 161008801;chr1:161008777-161008877 | 273 |
| 161284882 | 161285026 chr1:161284950-161284951 | 144 |
| 161306252 | 161306382 chr1:161306151-161306251,downstream | 130 |
| 166039366 | 166039510 chr1:166039395-166039495 | 144 |
| 169138792 | 169138934 chr1:169138868-169138869 | 142 |
| 170464175 | 170464329 chr1:170464254-170464255 | 154 |
| 171868017 | 171868187 chr1:171868066-171868166 | 170 |
| 175050401 | 175050549 chr1:175050430-175050530 | 148 |
| 180202441 | 180202578 chr1:180202463-180202563 | 137 |
| 182025968 | 182026117 chr1:182025995-182026095 | 149 |
| 193191311 | 193191476 chr1:193191356-193191456 | 165 |
| 196682870 | 196683025 chr1:196682896-196682996 | 155 |
| 214646125 | 214646279 chr1:214646154-214646254 | 154 |
| 217310510 | 217310654 chr1:217310537-217310637 | 144 |
| 220101648 | 220101795 chr1:220101678-220101778 | 147 |
| 220101867 | 220102015 chr1:220101896-220101996 | 148 |
| 223948836 | 223948969 chr1:223948861-223948961 | 133 |
| | chr1:226187853-226187854;chr1:226187877- | |
| 226187776 | 226188068 226187878;chr1:226188006-226188007 | 292 |
| 236557105 | 236557253 chr1:236557182-236557183 | 148 |
| 236849398 | 236849548 chr1:236849424-236849524 | 150 |
| 236849891 | 236850048 chr1:236849917-236850017 | 157 |
| 237765796 | 237765947 chr1:237765826-237765926 | 151 |
| | chr1:240656502-240656602;chr1:240656537- | |
| 240656480 | 240656649 240656637 | 169 |
| 240746545 | 240746706 chr1:240746575-240746675 | 161 |
| 246241918 | 246242056 chr1:246241939-246242039 | 138 |
| 248903024 | 248903175 chr1:248903051-248903151 | 151 |

## Patentansprüche

1. Verfahren zur Diagnose von Lungenkrebs, bei dem man die Methylierung eines Satzes von Methylierungsmarkern in einer Probe eines Patienten bestimmt, wobei man bevorzugt zfDNA aus einer Flüssigbiopsie untersucht.

2. Verfahren nach Anspruch 1, wobei der Satz von Methylierungsmarkern mindestens 60 Regionen umfasst, ausgewählt aus der Gruppe bestehend aus:
| Chromosom | Start | Ende |
|---|---|---|
| chr1 | 6165201 | 6165361 |
| chr1 | 17567892 | 17568189 |
| chr1 | 15426262 | 15426418 |
| chr1 | 15670403 | 15670539 |
| chr2 | 1126410 | 1126557 |
| chr2 | 225642009 | 225642217 |
| chr2 | 236745514 | 236745688 |
| chr2 | 240881986 | 240882138 |
| chr2 | 2179742 | 2179886 |
| chr2 | 30747398 | 30747539 |
| chr2 | 175998270 | 175998415 |
| chr2 | 219647407 | 219647560 |
| chr3 | 56445240 | 56445378 |
| chr3 | 85143433 | 85143600 |
| chr3 | 146123966 | 146124095 |
| chr3 | 68947379 | 68947542 |
| chr3 | 197767819 | 197767978 |
| chr4 | 143487129 | 143487273 |
| chr4 | 26398190 | 26398329 |
| chr4 | 77647893 | 77648027 |
| chr4 | 102497551 | 102497732 |
| chr5 | 39187156 | 39187287 |
| chr5 | 56145736 | 56145896 |
| chr5 | 160171748 | 160171896 |
| chr5 | 16793080 | 16793219 |
| chr5 | 76869108 | 76869253 |
| chr6 | 169050287 | 169050447 |
| chr6 | 76773251 | 76773422 |
| chr6 | 123869831 | 123869971 |
| chr7 | 6268960 | 6269087 |
| chr7 | 38508407 | 38508486 |
| chr7 | 153743779 | 153743947 |
| chr7 | 137230794 | 137230963 |
| chr7 | 151300131 | 151300282 |
| chr8 | 3672236 | 3672387 |
| chr8 | 99510084 | 99510252 |
| chr8 | 101170822 | 101170975 |
| chr8 | 141127042 | 141127183 |
| chr9 | 2050654 | 2050804 |
| chr9 | 9227683 | 9227824 |
| chr9 | 79060522 | 79060633 |
| chr9 | 124334690 | 124334848 |
| chr9 | 126166694 | 126166828 |
| chr10 | 96279972 | 96280055 |
| chr10 | 97033594 | 97033733 |
| chr11 | 134245966 | 134246129 |
| chr12 | 8004422 | 8004573 |
| chr12 | 97140774 | 97140905 |
| chr12 | 111566555 | 111566698 |
| chr12 | 117750775 | 117750937 |
| chr13 | 36828740 | 36828902 |
| chr14 | 93214072 | 93214242 |
| chr15 | 56006471 | 56006552 |
| chr15 | 101547384 | 101547527 |
| chr16 | 4141795 | 4141956 |
| chr18 | 21857621 | 21857750 |
| chr18 | 29528340 | 29528468 |
| chr18 | 46845901 | 46846043 |
| chr19 | 874766 | 874934 |
| chr19 | 6799968 | 6800095 |
| chr20 | 20243607 | 20243747 |
| chr20 | 55079800 | 55079945 |
| chr21 | 30502729 | 30502871 |
| chr21 | 46587906 | 46588052 |
wobei man das bevorzugt Vorhandensein eines Tumors analysiert, wobei der Satz von Methylierungsmarkern optional alle Regionen der Gruppe umfasst.

3. Verfahren nach Anspruch 2, wobei der Satz von Methylierungsmarkern mindestens 340 Regionen umfasst, ausgewählt aus der Gruppe bestehend aus den in Tabelle 1a aufgelisteten Regionen, wobei der Satz von Methylierungsmarkern bevorzugt alle in Tabelle 1a aufgelisteten Regionen umfasst.

4. Verfahren nach einem der vorhergehenden Ansprüche, wobei der Satz von Methylierungsmarkern mindestens 134 Regionen umfasst, ausgewählt aus der Gruppe bestehend aus:
| | | |
|---|---|---|
| chr1 | 3289010 | 3289139 |
| chr1 | 17567892 | 17568189 |
| chr1 | 23284417 | 23284507 |
| chr1 | 24277975 | 24278154 |
| chr1 | 47738990 | 47739142 |
| chr1 | 79467955 | 79468081 |
| chr1 | 108975333 | 108975476 |
| chr1 | 196682870 | 196683025 |
| chr1 | 217310510 | 217310654 |
| chr1 | 240656480 | 240656649 |
| chr1 | 240746545 | 240746706 |
| chr1 | 246241918 | 246242056 |
| chr2 | 1129413 | 1129596 |
| chr2 | 1334513 | 1334640 |
| chr2 | 23917010 | 23917136 |
| chr2 | 25124037 | 25124165 |
| chr2 | 46779214 | 46779381 |
| chr2 | 113534514 | 113534653 |
| chr2 | 120417931 | 120418073 |
| chr2 | 131798797 | 131798977 |
| chr2 | 198073787 | 198073950 |
| chr2 | 205889570 | 205889704 |
| chr2 | 207319476 | 207319691 |
| chr3 | 3755582 | 3755730 |
| chr3 | 14959981 | 14960128 |
| chr3 | 25581721 | 25581859 |
| chr3 | 75834579 | 75834736 |
| chr3 | 87031909 | 87032079 |
| chr3 | 122710736 | 122710872 |
| chr3 | 139727561 | 139727706 |
| chr3 | 145864433 | 145864574 |
| chr4 | 1665996 | 1666155 |
| chr4 | 22518120 | 22518271 |
| chr4 | 77306769 | 77306948 |
| chr4 | 82520036 | 82520212 |
| chr4 | 155413871 | 155414011 |
| chr4 | 156601279 | 156601436 |
| chr4 | 162457724 | 162457860 |
| chr4 | 176636441 | 176636580 |
| chr4 | 177654193 | 177654363 |
| chr5 | 14450118 | 14450272 |
| chr5 | 75935318 | 75935450 |
| chr5 | 140475728 | 140475872 |
| chr5 | 146345906 | 146346062 |
| chr5 | 156458027 | 156458167 |
| chr5 | 157169890 | 157170038 |
| chr6 | 20832000 | 20832349 |
| chr6 | 24420281 | 24420413 |
| chr6 | 36331071 | 36331215 |
| chr6 | 54074847 | 54075021 |
| chr6 | 71122323 | 71122483 |
| chr6 | 83604672 | 83604779 |
| hr6 | 90709859 | 90710016 |
| chr6 | 111744738 | 111744881 |
| chr6 | 148806765 | 148806922 |
| chr6 | 155574119 | 155574263 |
| chr6 | 158460178 | 158460323 |
| chr7 | 5549605 | 5549675 |
| chr7 | 40669616 | 40669796 |
| chr7 | 73799798 | 73799908 |
| chr7 | 78030021 | 78030155 |
| chr7 | 81399230 | 81399365 |
| chr7 | 134452355 | 134452524 |
| chr7 | 140335200 | 140335344 |
| chr7 | 146925646 | 146925824 |
| chr7 | 153976496 | 153976643 |
| chr7 | 157941162 | 157941344 |
| chr7 | 157980130 | 157980264 |
| chr7 | 157980485 | 157980624 |
| chr7 | 158314155 | 158314301 |
| chr8 | 6392188 | 6392336 |
| chr8 | 11724061 | 11724159 |
| chr8 | 17237496 | 17237639 |
| chr8 | 21803649 | 21803801 |
| chr8 | 52696850 | 52697008 |
| chr8 | 72183950 | 72184120 |
| chr8 | 81042553 | 81042694 |
| chr8 | 85101824 | 85101952 |
| chr8 | 110703169 | 110703320 |
| chr8 | 121727803 | 121727944 |
| chr8 | 133476418 | 133476558 |
| chr9 | 8813022 | 8813150 |
| chr9 | 90258110 | 90258253 |
| chr9 | 97061691 | 97061835 |
| chr10 | 12533631 | 12533768 |
| chr10 | 32647546 | 32647656 |
| chr10 | 32657588 | 32657719 |
| chr10 | 37511104 | 37511239 |
| chr10 | 62708104 | 62708269 |
| chr10 | 73207931 | 73208064 |
| chr10 | 108812804 | 108812940 |
| chr10 | 115658133 | 115658275 |
| chr10 | 123914649 | 123914808 |
| chr11 | 15025357 | 15025499 |
| chr11 | 19778770 | 19778909 |
| chr11 | 26355535 | 26355711 |
| chr11 | 26600784 | 26600925 |
| chr11 | 26626367 | 26626558 |
| chr11 | 41275397 | 41275536 |
| chr11 | 62158845 | 62158985 |
| chr11 | 70503001 | 70503139 |
| chr11 | 106592142 | 106592304 |
| chr11 | 120644150 | 120644282 |
| chr11 | 122678508 | 122678636 |
| chr11 | 128851150 | 128851286 |
| chr12 | 125571801 | 125571933 |
| chr13 | 48806444 | 48806588 |
| chr13 | 113527733 | 113527876 |
| chr14 | 35030336 | 35030470 |
| chr14 | 104486171 | 104486314 |
| chr15 | 22839905 | 22840043 |
| chr15 | 26964926 | 26965065 |
| chr15 | 29246303 | 29246447 |
| chr15 | 30180680 | 30180842 |
| chr15 | 32404970 | 32405130 |
| chr15 | 64244033 | 64244215 |
| chr15 | 68530927 | 68531091 |
| chr15 | 83579367 | 83579513 |
| chr15 | 88559865 | 88560003 |
| chr16 | 6257325 | 6257474 |
| chr16 | 15665564 | 15665721 |
| chr16 | 24321180 | 24321320 |
| chr16 | 75528556 | 75528698 |
| chr16 | 88013993 | 88014135 |
| chr16 | 89713952 | 89714124 |
| chr17 | 416719 | 416865 |
| chr17 | 19809670 | 19809830 |
| chr17 | 21086965 | 21087112 |
| chr17 | 33364961 | 33365040 |
| chr17 | 64330485 | 64330837 |
| chr17 | 75142732 | 75142885 |
| chr19 | 11890923 | 11891074 |
| chr19 | 49016450 | 49016584 |
| chr19 | 57922060 | 57922195 |
| chr20 | 9706282 | 9706429 |
| chr20 | 33713618 | 33713757 |
| chr21 | 33340955 | 33341038 |
| chr22 | 21206849 | 21206995 |
| chr22 | 30292326 | 30292475 |
| chr22 | 35697444 | 35697606 |
wobei man die bevorzugt Entität eines Tumors identifiziert, wobei der Satz von Methylierungsmarkern optional alle Regionen der Gruppe umfasst.

5. Verfahren nach Anspruch 4, wobei der Satz von Methylierungsmarkern mindestens 240 Regionen umfasst, wobei die Gruppe aus den in Tabelle 1b aufgelisteten Regionen besteht, wobei der Satz von Methylierungsmarkern optional alle in Tabelle 1b aufgelisteten Regionen der Gruppe umfasst.

6. Verfahren nach einem der vorhergehenden Ansprüche, wobei der Satz von Methylierungsmarkern mindestens 620 Regionen umfasst, wobei die Gruppe aus den in Ansprüchen 3 und 5 definierten Methylierungsmarkern und ferner den in Tabelle 1c aufgelisteten Regionen besteht, wobei man bevorzugt ferner die Prognose bestimmt, wobei der Satz von Methylierungsmarkern optional alle Regionen der Gruppe umfasst.

7. Verfahren nach einem der vorhergehenden Ansprüche, wobei der Satz von Methylierungsmarkern die 10 folgenden Positionen umfasst:
| ID | Chromosom | Position |
|---|---|---|
| 596 | chr11 | 57006229 |
| 1717 | chr15 | 28262724 |
| 2636 | chr18 | 61144199 |
| 2805 | chr19 | 46823441 |
| 4674 | chr2 | 176964685 |
| 4999 | chr2 | 225642035 |
| 5071 | chr3 | 14960020 |
| 5576 | chr4 | 13525705 |
| 6105 | chr5 | 140475760 |
| 6434 | chr6 | 46386723 |
und man optional den kNN-Algorithmus zur Analyse verwendet, wobei man das Vorhandensein eines Tumors analysiert.

8. Verfahren nach einem der vorhergehenden Ansprüche, wobei der Satz von Methylierungsmarkern die 10 folgenden Positionen umfasst:
| ID | Chromosom | Position |
|---|---|---|
| 650 | chr11 | 64993331 |
| 2995 | chr1 | 17568007 |
| 4233 | chr2 | 50574690 |
| 4241 | chr2 | 50574708 |
| 4428 | chr2 | 111874494 |
| 4447 | chr2 | 121276804 |
| 5537 | chr4 | 1666074 |
| 5538 | chr4 | 1666075 |
| 6524 | chr6 | 83604790 |
| 7164 | chr7 | 69971740 |
und man optional den RT-Algorithmus zur Analyse verwendet, wobei man die Entität eines Tumors identifiziert.

9. Verfahren nach einem der vorhergehenden Ansprüche, wobei der Satz von Methylierungsmarkern alle in Tabelle 4 aufgelisteten Positionen umfasst und man optional den SVM-Algorithmus zur Analyse verwendet, wobei man das Stadium eines Tumors identifiziert.

10. Verfahren nach einem der vorhergehenden Ansprüche, **dadurch gekennzeichnet, dass** der Lungenkrebs NSCLC, ausgewählt aus der Gruppe umfassend Adenokarzinom und Plattenepithelkarzinom, oder SCLC ist, bevorzugt NSCLC, ausgewählt aus der Gruppe umfassend Adenokarzinom und Plattenepithelkarzinom.

11. Verfahren nach einem der vorhergehenden Ansprüche, **dadurch gekennzeichnet, dass** es eine Aussage über das Vorhandenseins eines Tumors, über die Entität eines Tumors, über das Tumorstadium und/oder über die Prognose erlaubt, bevorzugt über Vorhandensein und Entität des Tumors, optional über alles gleichzeitig.

12. Verfahren nach einem der vorhergehenden Ansprüche, **dadurch gekennzeichnet, dass** die Flüssigbiopsie-Probe ausgewählt ist aus der Gruppe umfassend Blut, Plasma, Serum, Sputum, Bronchialflüssigkeit und Pleuraerguss, bevorzugt Plasma.

13. Mittel, geeignet zur Diagnose von Lungenkrebs mit einem Verfahren nach einem der Ansprüche 2-12 durch Untersuchung der Methylierung eines Satzes von Methylierungsmarkern in zfDNA aus einer Flüssigbiopsie-Probe eines Patienten,
wobei das Mittel Oligonukleotide umfasst, welche mit DNA hybridisieren können, welche die genannten Methylierungsmarker umfasst, ausgewählt aus der Gruppe umfassend Oligonukleotide, die an einen festen Träger gekoppelt werden können, Oligonukleotide, die an einen festen Träger gekoppelt sind und/oder ein Kit umfassend PCR-Primer zur Amplifikation von Regionen, welche die Methylierungsmarker umfassen.

14. Verfahren nach einem der Ansprüche 1-12, wobei man Sequenzierungsdaten von bisulfitkonvertierter zfDNA gegen ein Referenzgenom mit dem Segemehl Algorithmus aligned, wobei man bevorzugt:
a. zfDNA aus der Flüssigbiopsie-Probe oder genomische DNA aus einer soliden Gewebeprobe extrahiert,
b. eine Bisulfitkonvertierung durchführt,
c. eine Whole Genome Bisulfite Sequencing Library hergestellt,
d. DNA-Regionen umfassend die definierten Methlierungsmarker anreichert, wobei man sie bevorzugt mit dem Mittel nach Anspruch 13 in Kontakt bringt,
e. die angereicherten DNA-Regionen sequenziert,
f. die Sequenzierungsdaten gegen ein Referenzgenom mit dem Segemehl Algorithmus aligned,
g. die Methylierungsraten berechnet.

15. Verwendung eines Verfahrens nach einem der Ansprüche 1-12 oder 14 oder eines Mittels nach Anspruch 13 zur Diagnose von Lungenkrebs, wobei die Diagnose eine Aussage über das Vorhandenseins eines Tumors, über die Entität eines Tumors, über das Tumorstadium und/oder über die Prognose erlaubt, bevorzugt über Vorhandensein und Entität des Tumors, optional über alles gleichzeitig.
